# EUROPEAN PATENT APPLICATION

(11) **EP 4 488 283 A1**
(43) Date of publication of application: **08.01.2025**
(21) Application number: 23784800.7
(22) Date of filing: 06.04.2023
(51) Int. Cl.: C07K 1/06

(54) **PRODUCTION METHOD FOR PEPTIDE COMPOUND**

(30) Priority: 08.04.2022 JP 2022064409
(71) Applicant: CHUGAI SEIYAKU KABUSHIKI KAISHA, Tokyo 115-8543 (JP)
(72) Inventor: SHINOHARA Shojiro, Kamakura-shi, Kanagawa 247-8530 (JP); MORITA Yuya, Kamakura-shi, Kanagawa 247-8530 (JP); NAKANO Kazuhiko, Kamakura-shi, Kanagawa 247-8530 (JP); EMURA Takashi, Gotemba-shi, Shizuoka 412-8513 (JP)
(74) Representative: Vossius & Partner Patentanwälte Rechtsanwälte mbB
(86) International application number: PCT/JP2023/014205
(87) International publication number: WO 2023/195516

(57) **Abstract**

A method for producing a peptide compound by a solid phase method, the method comprising:
a preparation step of preparing a first amino acid having an amino group or a first peptide having an amino group supported on a solid phase; and
a condensation step of condensing the first amino acid or first peptide, and a second amino acid having a protected amino group and/or protected hydroxy group and a carboxy group, or a second peptide having a protected amino group and/or
protected hydroxy group and a carboxy group in the presence of a predetermined carbodiimide-based condensing agent and an additive.

## Description

### Technical Field

The present invention relates to a method for producing a peptide compound.

### Background Art

Peptides are molecules in which a large number of amino acids are linked. Peptides play an indispensable role in the life activities of the organisms. With the development of biology and chemistry, the understanding of peptides is deepened, and the utilization of natural peptides oriented to the creation of new drugs and the research and development of functional peptides by the artificial design of peptides are actively performed (Non Patent Literature 1). In particular, it has been reported that the cyclization of peptides and N-alkylation of constituent amino acids, especially N-methylation, contribute to the improvement of membrane permeability and metabolic stability (Non Patent Literature 2, Non Patent Literature 3) and the drug-like cyclic peptide structures that becomes a key for intracellular migration and development of oral agents have been found and discussed, and the importance of these structures in peptide drug discovery has been increasingly recognized (Patent Literature 1).

Synthesis of a peptide is achieved by forming an amide bond to extend into a desired sequence. More specific synthesis method includes a liquid phase method and a solid phase method (Non Patent Literature 4). Among them, the solid phase method is a method of synthesizing a peptide of interest by sequentially linking amino acids using a polymer resin (resin for solid phase synthesis) as a stationary phase (Non Patent Literature 5). Recently, the peptide array technology using a membrane such as cellulose as a stationary phase has been developed, and it is attracting attention as a technology for rapidly and automatically synthesizing peptides of multiple samples of small amounts (Non Patent Literature 6).

### Citation List

### Patent Literature

Patent Literature 1: International Publication No. WO 2013/100132
Patent Literature 2: International Publication No. WO 2018/225851

### Non Patent Literature

Non Patent Literature 1: Future Med. Chem., 2009, 1, 1289-1310.
Non Patent Literature 2: Acc.Chem. Res. 2008, 41, 1331-1342.
Non Patent Literature 3: Angew. Chem. Int. Ed., 2013, 52, 254-269.
Non Patent Literature 4: Amino Acids, Peptides and Proteins in Organic Chemistry: Building Blocks, Catalysis and Coupling Chemistry, Volume 3, 2011.
Non Patent Literature 5: Solid phase peptide synthesis (published by Bachem Holding AG, [search on January 18, 2022], Internet <URL:https://www.bachem.com/wp-admin/admin-ajax.php?juwpfisadmin=false&action=wpfd&task=file.download&wpfd_category_id=180&wpf d_file_id=213490&token=&preview=1>
Non Patent Literature 6: Biotechnology and Bioprocess Engineering, 2016, 21, 119-127.
Non Patent Literature 7: Methods Mol. Biol., 2009, 570, 157-174.
Non Patent Literature 8: Org. Lett. 2021, 23, 6900-6904.

### Summary of Invention

### Technical Problem

In the solid-phase synthesis of peptides, the peptide array technology using a membrane such as cellulose as a stationary phase has been developed, and it is attracting attention as a technology for rapidly and automatically synthesizing peptides of multiple samples of small amounts (Non Patent Literature 6). The present inventors have thus attempted the synthesis of drug-like peptides in the peptide array technology using a membrane as a stationary phase. However, since the drug-like peptide contains a large number of N-alkylamino acids, the amidation reaction in the synthesis of the peptide is highly difficult. When the synthesis using a peptide array has been performed with existing amidation conditions, all conditions have resulted in low yield, revealing that there are currently no satisfactory amidation conditions. For example, in an amidation reaction of N-methylphenylalanine linked to a solid phase of membrane and the following Fmoc-norleucine with the reaction conditions described in Non Patent Literature 7, the reaction conversion rate has resulted in 3-4%. Also, when the amidation reaction has been performed with reference to the conditions using diisopropylcarbodiimide (DIC) and 1-hydroxy-7-azabenzotriazole (HOAt) described in the synthesis method of peptides containing N-substituted amino acids (Patent Literature 2), the reaction conversion rate has resulted in 50-64%.

We have investigated the details of the reaction and found the inactivation of the active esters in the reaction system. When we have further investigated by doubling the concentration of the reagent to improve the reaction conversion rate, crystals of N,N'-diisopropylurea have been precipitated. In the peptide array technology, the automation of liquid delivery is employed to speed up and improve efficiency. However, existing conditions have difficulty in employing the high reagent concentration because crystal precipitation causes clogging and/or increase of impurities due to poor washing.

In one aspect, an object of the present invention is to improve the reaction conversion rate in peptide synthesis methods, especially in the condensation reaction step. In another aspect, an object of the present invention is to provide a method for efficiently producing high-purity peptide compounds by employing amidation conditions that enable the application to automated synthesis by avoiding crystal precipitation.

### Solution to Problem

In view of the above circumstances, the present inventors have intensively studied and finally completed the present invention. The method of the present invention is a method that is applicable not only to the peptide array technology using a membrane as the stationary phase, but also to the solid-phase synthesis using a polymer resin (resin for solid phase synthesis) as the stationary phase.

For example, the present invention provides the following [A1] to [A64].
[A1] A method for producing a peptide compound by a solid phase method, the method comprising:
   a preparation step of preparing a first amino acid having an amino group or a first peptide having an amino group supported on a solid phase; and
   a condensation step of condensing the first amino acid or first peptide, and a second amino acid having a protected amino group and/or protected hydroxy group and a carboxy group, or a second peptide having a protected amino group and/or protected hydroxy group and a carboxy group in the presence of at least one carbodiimide-based condensing agent represented by the following formula (A):

      R^{A}-N=C=N-R^{B} (A)

      wherein R^{A} is C₄-C₁₀ secondary or tertiary alkyl, and R^{B} is C₂-C₁₀ alkyl, C₆-C₁₀ aryl, or C₇-C₁₄ arylalkyl, and each group in R^{A} and R^{B} is optionally substituted with one or more groups independently selected from halogen, C₁-C₆ alkoxy, di-C₁-C₆ alkylamino, or 4- to 8-membered cyclic amino,
   and an additive.
[A2] The method according to [A1], wherein R^{A} is C₄-C₁₀ secondary alkyl.
   [A2-1] The method according to [A1], wherein R^{A} is C₄-C₈ secondary alkyl.
   [A2-2] The method according to [A1], wherein R^{A} is C₄-C₈ secondary or tertiary alkyl having asymmetric carbon.
   [A2-3] The method according to [A1], wherein R^{A} is C₄-C₈ secondary alkyl having asymmetric carbon.
   [A2-4] The method according to [A1], wherein R^{A} is C₄-C₆ secondary alkyl having asymmetric carbon.
   [A2-5] The method according to [A1], wherein R^{A} is 1-methylheptyl, 1-methylbutyl, 1-ethylpropyl, or sec-butyl.
   [A2-6] The method according to [A1], wherein R^{A} is sec-butyl.
[A3] The method according to [A1] or [A2], wherein R^{B} is C₃-C₁₀ secondary or tertiary alkyl, C₆-C₁₀ aryl, or C₇-C₁₄ arylalkyl, and each group in R^{B} is optionally substituted with one or more groups independently selected from halogen, C₁-C₆ alkoxy, di-C₁-C₆ alkylamino, or 4- to 8-membered cyclic amino.
   [A3-1] The method according to [A1] or [A2], wherein R^{B} is C₃-C₁₀ secondary or tertiary alkyl.
   [A3-2] The method according to [A1] or [A2], wherein R^{B} is C₃-C₁₀ secondary or tertiary alkyl, or C₇-C₁₄ arylalkyl.
   [A3-3] The method according to [A1] or [A2], wherein R^{B} is C₃-C₁₀ secondary alkyl or C₇-C₁₀ arylalkyl.
   [A3-4] The method according to [A1] or [A2], wherein R^{B} is C₃-C₆ secondary alkyl.
   [A3-5] The method according to [A1] or [A2], wherein R^{B} is 1-methylbenzyl, 1-methylheptyl, 1-methylbutyl, 1-ethylpropyl, or sec-butyl.
   [A3-6] The method according to [A1] or [A2], wherein R^{B} is sec-butyl.
[A4] The method according to [A1], wherein R^{A} is C₄-C₈ secondary or tertiary alkyl, and R^{B} is C₄-C₁₀ secondary or tertiary alkyl, or C₇-C₁₄ arylalkyl.
   [A4-1] The method according to [A1], wherein R^{A} is C₄-C₈ secondary alkyl, and R^{B} is C₄-C₁₀ secondary alkyl or C₇-C₁₄ arylalkyl.
   [A4-2] The method according to [A1], wherein R^{A} is C₄-C₆ secondary alkyl, and R^{B} is C₄-C₈ secondary alkyl, or C₇-C₁₀ arylalkyl.
[A5] The method according to [A1], wherein R^{A} is C₄-C₈ secondary or tertiary alkyl having asymmetric carbon, and R^{B} is C₃-C₁₀ secondary or tertiary alkyl.
   [A5-1] The method according to [A1], wherein R^{A} is C₄-C₈ secondary alkyl having asymmetric carbon, and R^{B} is C₄-C₁₀ secondary alkyl or C₇-C₁₄ arylalkyl.
   [A5-2] The method according to [A1], wherein R^{A} is C₄-C₈ secondary alkyl having asymmetric carbon, and R^{B} is C₄-C₁₀ secondary alkyl, or C₇-C₁₀ arylalkyl.
   [A5-3] The method according to [A1], wherein R^{A} is C₄-C₆ secondary alkyl having asymmetric carbon, and R^{B} is C₄-C₈ secondary alkyl or C₇-C₁₀ arylalkyl.
[A6] The method according to any of [A1] to [A5], wherein the carbodiimide-based condensing agent includes at least one selected from the group consisting of N,N'-di-sec-butylcarbodiimide (DsBC), N'-(1-phenylethyl)-N-sec-butyl-methanediimine (SS26), N'-(1-methylheptyl)-N-sec-butyl-methanediimine (SS27), N,N'-bis(1-methylbutyl)methanediimine (SS28), N,N'-bis(1-ethylpropyl)methanediimine (SS29), and N'-(1-ethylpropyl)-N-(1-methylbutyl)methanediimine (SS30).
   [A6-1] The method according to any of [A1] to [A5], wherein the carbodiimide-based condensing agent includes at least one selected from the group consisting of N,N'-di-sec-butylcarbodiimide (DsBC), N'-(1-phenylethyl)-N-sec-butyl-methanediimine (SS26), N'-(1-methylheptyl)-N-sec-butyl-methanediimine (SS27), N,N'-bis(1-methylbutyl)methanediimine (SS28), or N'-(1-ethylpropyl)-N-(1-methylbutyl)methanediimine (SS30).
   [A6-2] The method according to any of [A1] to [A5], wherein the carbodiimide-based condensing agent is N,N'-di-sec-butylcarbodiimide (DsBC), N'-(1-phenylethyl)-N-sec-butyl-methanediimine (SS26), N'-(1-methylheptyl)-N-sec-butyl-methanediimine (SS27), N,N'-bis(1-methylbutyl)methanediimine (SS28), or N'-(1-ethylpropyl)-N-(1-methylbutyl)methanediimine (SS30).
   [A6-3] The method according to any of [A1] to [A5], wherein the carbodiimide-based condensing agent is N,N'-di-sec-butylcarbodiimide (DsBC).
   [A6-4] The method according to any of [A1] to [A5], wherein the carbodiimide-based condensing agent is N'-(1-phenylethyl)-N-sec-butyl-methanediimine (SS26).
   [A6-5] The method according to any of [A1] to [A5], wherein the carbodiimide-based condensing agent is N'-(1-methylheptyl)-N-sec-butyl-methanediimine (SS27).
   [A6-6] The method according to any of [A1] to [A5], wherein the carbodiimide-based condensing agent is N,N'-bis(1-methylbutyl)methanediimine (SS28).
   [A6-7] The method according to any of [A1] to [A5], wherein the carbodiimide-based condensing agent is N,N'-bis(1-ethylpropyl)methanediimine (SS29).
   [A6-8] The method according to any of [A1] to [A5], wherein the carbodiimide-based condensing agent is N'-(1-ethylpropyl)-N-(1-methylbutyl)methanediimine (SS30).
[A7] The method according to any of [A1] to [A6], wherein the additive is at least one selected from the group consisting of 1-hydroxy-7-azabenzotriazole (HOAt), 1-hydroxybenzotriazole (HOBt), 3,4-dihydro-3-hydroxy-4-oxo-1,2,3-benzotriazine (HOOBt), cyano(hydroxyimino)ethyl acetate (Oxyma), and 5-(hydroxyimino)-1,3-dimethylpyrimidine-2,4,6(1H,3H,5H)-trione (Oxyma B).
[A8] The method according to any of [A1] to [A6], wherein the additive is at least one selected from the group consisting of HOAt, HOOBt, and Oxyma.
[A9] The method according to any of [A1] to [A6], wherein the additive is HOAt, HOOBt, or Oxyma.
[A10] The method according to any of [A1] to [A9], wherein the condensation step is performed in a solvent, and a concentration of the carbodiimide-based condensing agent in the solvent is 0.4 mol/L or more, 0.5 mol/L or more, 0.6 mol/L or more, or 0.7 mol/L or more.
[A11] The method according to any of [A1] to [A10], wherein the condensation step is performed in a solvent, and a concentration of the carbodiimide-based condensing agent in the solvent is 4.0 mol/L or less, 3.0 mol/L or less, 2.0 mol/L or less, or 1.0 mol/L or less.
[A12] The method according to any of [A1] to [A1 1], wherein the condensation step is performed in a solvent, and a concentration of the carbodiimide-based condensing agent in the solvent is 0.4 to 4.0 mol/L, 0.5 to 3.0 mol/L, 0.6 to 2.0 mol/L, or 0.7 to 1.0 mol/L.
[A13] The method according to any of [A1] to [A11], wherein the condensation step is performed in a solvent, and a concentration of the carbodiimide-based condensing agent in the solvent is 0.7 to 1.0 mol/L.
[A14] The method according to any of [A1] to [A13], wherein the solid phase is a membrane or a resin for solid phase synthesis.
[A15] The method according to any of [A1] to [A13], wherein the solid phase is a membrane.
[A16] The method according to [A15], wherein the membrane is a cellulose membrane, a polypropylene membrane, or a polyaminoethylmethacrylamide membrane.
[A17] The method according to [A15], wherein the membrane is a cellulose membrane.
[A18] The method according to any of [A1] to [A13], wherein the solid phase is a resin for solid phase synthesis.
[A19] The method according to [A18], wherein the resin for solid phase synthesis is a chlorotrityl (CTC) resin, a trityl (Trt) resin, a SASRIN resin, a Rink amide resin, a Merrifield resin, or a Wang resin.
[A20] The method according to [A18], wherein the resin for solid phase synthesis is a chlorotrityl (CTC) resin.
[A21] The method according to any of [A1] to [A20], wherein the solid phase and the first amino acid or first peptide are linked via a photo-cleavable site, a disulfide bond, or an acid-labile site.
   [A21-1] The method according to any of [A1] to [A20], wherein the solid phase and the first amino acid or first peptide are linked via a photo-cleavable site.
   [A21-2] The method according to any of [A1] to [A20], wherein the solid phase and the first amino acid or first peptide are linked via a disulfide bond.
   [A21-3] The method according to any of [A1] to [A20], wherein the solid phase and the first amino acid or first peptide are linked via an acid-labile site.
[A22] The method according to [A21-1], wherein the photo-cleavable site has a nitroveratryloxycarbonyl residue or a coumarin residue.
   [A22-1] The method according to [A21-1], wherein the photo-cleavable site is a nitroveratryloxycarbonyl residue.
[A23] The method according to [A21-3], wherein, the acid-labile site has a trityl ester structure, a chlorotrityl ester structure, an alkoxybenzyl ether structure, a trialkoxybenzylaminocarbonyl structure, a dialkoxyphenyl-alkoxyphenylmethylaminocarbonyl structure, or an alkoxyxanthen-9-ylaminocarbonyl structure.
   [A23-1] The method according to [A21-3], wherein the acid-labile site has a chlorotrityl ester structure.
[A24] The method according to any of [A1] to [A23], wherein the carbodiimide-based condensing agent is used at 1.0 equivalent or more, 1.1 equivalents or more, or 1.2 equivalents or more with respect to the second amino acid or second peptide in the condensation step.
[A25] The method according to any of [A1] to [A24], wherein the carbodiimide-based condensing agent is used at 5.0 equivalents or less, 4.0 equivalents or less, 3.0 equivalents or less, 2.0 equivalents or less, or 1.5 equivalents or less with respect to the second amino acid or second peptide in the condensation step.
[A26] The method according to any of [A1] to [A25], wherein the carbodiimide-based condensing agent is used at 1.0 to 5.0 equivalents, 1.1 to 4.0 equivalents, 1. 1 to 3.0 equivalents, 1.2 to 2.0 equivalents, or 1.2 to 1.5 equivalents with respect to the second amino acid or second peptide in the condensation step.
[A27] The method according to any of [A1] to [A25], wherein the carbodiimide-based condensing agent is used at 1.2 to 1.5 equivalents with respect to the second amino acid or second peptide in the condensation step.
[A28] The method according to any of [A1] to [A27], wherein the additive is used at 0.01 equivalents, 0.05 equivalents, 0.1 equivalents, 0.2 equivalents or more, or 0.3 equivalents or more with respect to the second amino acid or second peptide in the condensation step.
[A29] The method according to any of [A1] to [A28], wherein the additive is used at 1.0 equivalent or less, 0.9 equivalents or less, 0.8 equivalents or less, or 0.7 equivalents or less with respect to the second amino acid or second peptide in the condensation step.
[A30] The method according to any of [A1] to [A29], wherein the additive is used at 0.05 to 1.0 equivalent, 0.1 to 0.9 equivalents, 0.2 to 0.8 equivalents, or 0.3 to 0.7 equivalents with respect to the second amino acid or second peptide in the condensation step.
[A31] The method according to any of [A1] to [A29], wherein the additive is used at 0.3 to 0.7 equivalents with respect to the second amino acid or second peptide in the condensation step.
[A32] The method according to any of [A1] to [A31], wherein the first amino acid or an amino acid at the N-terminus of the first peptide is a non-natural amino acid.
[A33] The method according to any of [A1] to [A32], wherein the first amino acid or an amino acid at the N-terminus of the first peptide is an α,α-di-substituted amino acid, a β-branched amino acid, or an N-alkylamino acid provided that the alkyl in the N-alkylamino acid is optionally substituted with one or more groups independently selected from C₃-C₆ cycloalkyl, C₂-C₆ alkenyl, C₂-C₆ alkynyl, or C₆-C₁₀ aryl.
[A34] The method according to any of [A1] to [A32], wherein the first amino acid or an amino acid at the N-terminus of the first peptide is an N-alkylamino acid in which the alkyl has 1 to 8 carbon atoms, provided that the alkyl is optionally substituted with one or more groups independently selected from halogen, cyano, C₃-C₆ cycloalkyl, C₂-C₆ alkenyl, C₂-C₆ alkynyl, or C₆-C₁₀ aryl.
[A35] The method according to any of [A1] to [A32], wherein the first amino acid or an amino acid at the N-terminus of the first peptide is an N-alkylamino acid, an N-alkylamino acid in which the alkyl has 1 to 8 carbon atoms, an N-alkylamino acid in which the alkyl has 1 to 6 carbon atoms, an N-alkylamino acid in which the alkyl has 1 to 3 carbon atoms, or an N-methylamino acid.
[A36] The method according to any of [A1] to [A32], wherein the first amino acid or an amino acid at the N-terminus of the first peptide is an N-methylamino acid.
[A37] The method according to any of [A1] to [A36], wherein the second amino acid or an amino acid at the C-terminus of the second peptide is a non-natural amino acid.
[A38] The method according to any of [A1] to [A37], wherein the second amino acid or an amino acid at the C-terminus of the second peptide is an α,α-di-substituted amino acid, a β-branched amino acid, or an N-alkylamino acid.
[A39] The method according to any of [A1] to [A38], wherein a protecting group of the protected amino group is a group represented by the following formula (1): 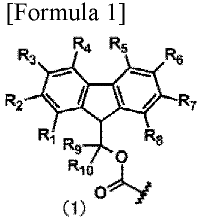 wherein, R₁ to R₈ are each independently selected from the group consisting of hydrogen, C₁-C₈ alkyl, C₁-C₈ fluoroalkyl, halogen, sulfo, and trimethylsilyl; and R₉ to R₁₀ are each independently hydrogen or methyl.
[A40] The method according to any of [A1] to [A38], wherein a protecting group of the protected amino group is a 9-fluorenylmethyloxycarbonyl (Fmoc) group, a Fmoc (2,7tb) group, a Fmoc (1Me) group, a Fmoc (2F) group, a Fmoc (2,7Br) group, a mio-Fmoc group, a dio-Fmoc group, a tdf-Fmoc group, a Fmoc (2TMS) group, a Fmoc (2so3h) group, an sm-Fmoc group, or an rm-Fmoc group.
[A41] The method according to any of [A1] to [A38], wherein a protecting group of the protected amino group is a Fmoc group.
[A42] The method according to any of [A1] to [A41], wherein the condensation step is performed in a solvent.
[A43] The method according to [A42], wherein the solvent includes at least one selected from the group consisting of an amide-based solvent, a urea-based solvent, an ether-based solvent, a halogen-based solvent, a nitrile-based solvent, and a benzene-based solvent.
[A44] The method according to [A43], wherein the solvent is an amide-based solvent.
[A45] The method according to [A44], wherein the amide-based solvent is selected from the group consisting of N,N-dimethylformamide (DMF), N-methyl-2-pyrrolidone (NMP), dimethylacetamide (DMA), N-ethyl-2-pyrrolidone (NEP), N-butyl-2-pyrrolidone (NBP), and formamide.
[A46] The method according to [A43], wherein the solvent is a urea-based solvent.
[A47] The method according to [A46], wherein the urea-based solvent is selected from the group consisting of 1,3-dimethyl-2-imidazolidinone (DMI) and N,N'-dimethylpropyleneurea (DMPU).
[A48] The method according to [A43], wherein the solvent is an ether-based solvent.
[A49] The method according to [A48], wherein the ether-based solvent is selected from the group consisting of tetrahydrofuran, 2-methyltetrahydrofuran, and 4-methyltetrahydropyran.
[A50] The method according to [A43], wherein the solvent is a halogen-based solvent.
[A51] The method according to [A50], wherein the halogen-based solvent is selected from the group consisting of dichloromethane and 1,2-dichloroethane.
[A52] The method according to [A43], wherein the solvent is a nitrile-based solvent.
[A53] The method according to [A52], wherein the nitrile-based solvent is acetonitrile.
[A54] The method according to [A43], wherein the solvent is a benzene-based solvent.
[A55] The method according to [A54], wherein the benzene-based solvent is selected from the group consisting of benzene, toluene, and xylene.
[A56] The method according to any of [A1] to [A55], wherein the condensation step is performed at 0°C to 100°C, 10°C to 80°C, 10°C to 60°C, 10°C to 50°C, 10°C to 40°C, 10°C to 35°C, 15°C to 60°C, 20°C to 60°C, 20°C to 40°C, or 25°C to 40°C.
[A57] The method according to any of [A1] to [A55], wherein the condensation step is performed at 20°C to 60°C.
[A58] The method according to any of [A1] to [A57], wherein the condensation step is repeated 2 or more times.
[A59] The method according to any of [A1] to [A57], wherein the condensation step is repeated 2 times.
[A60] The method according to any of [A1] to [A59], further comprising, after the condensation step, a deprotection step of deprotecting a protecting group of the protected amino group and/or protected hydroxy group in the second amino acid or second peptide supported on a solid phase.
[A61] The method according to [A60], further comprising a washing step of washing the solid phase between the condensation step and the deprotection step.
[A62] The method according to [A60] or [A61], wherein the condensation step and the deprotection step are repeated a plurality of times provided that a plurality of the second amino acids and/or second peptides used in the plurality of condensation steps may be each the same or different.
[A63] Use of a carbodiimide-based condensing agent represented by the above formula (A) in the method according to any of [A1] to [A62].
[A64] Use of DsBC in the method according to any of [A1] to [A62].

Regarding the combination of R^{A} and R^{B} in the above formula (A), [A4] to [A4-2] is preferred from the viewpoint that the active ester can be maintained at a high concentration for a long time in the reaction system, and the reaction conversion rate can be further improved in the condensation reaction step, and [A5] to [A5-3] is preferred from the viewpoint that the precipitation of urea derived from carbodiimide is highly avoided even at the high concentrations, and thus the amidation can be efficiently performed even in automated synthesis.

The methods for producing a peptide compound by the solid phase method have been described in [A1] to [A64], but these production methods can also be applied to a liquid phase method. That is, one aspect of the present invention provides the following [A1']. [A1'] may include features of [A2] to [A64] except for solid phase. [A1'] A method for producing a peptide compound by a liquid phase method, the method comprising:
a preparation step of preparing a first amino acid having an amino group or a first peptide having an amino group; and
a condensation step of condensing the first amino acid or first peptide, and a second amino acid having a protected amino group and/or protected hydroxy group and a carboxy group, or a second peptide having a protected amino group and/or protected hydroxy group and a carboxy group in the presence of at least one carbodiimide-based condensing agent represented by the above formula (A) and an additive.
According to such a method, the peptide compound of interest can be obtained with a high reaction conversion rate.

The present invention also provides, for example, the following [B1] to [B63].
[B1] A method for producing a peptide compound by a solid phase method, the method comprising:
   a preparation step of preparing a first amino acid having an N-alkylamino group or a first peptide having an N-alkylamino group at the N-terminus supported on a solid phase; and
   a condensation step of condensing the first amino acid or first peptide, and a second amino acid having a protected amino group and/or protected hydroxy group and a carboxy group, or a second peptide having a protected amino group and/or protected hydroxy group and a carboxy group in the presence of at least one carbodiimide-based condensing agent represented by the following formula (A'):

      R^{A'}-N=C=N-R^{B'} (A')

      wherein R^{A}' is C₃-C₁₀ alkyl, C₆-C₁₀ aryl, or C₇-C₁₄ arylalkyl, and R^{B}' is C₁-C₁₀ alkyl, C₆-C₁₀ aryl, or C₇-C₁₄ arylalkyl, and each group in R^{A}' and R^{B}' is optionally substituted with one or more groups independently selected from halogen, C₁-C₆ alkoxy, di-C₁-C₆ alkylamino, or 4- to 8-membered cyclic amino, provided that the total number of carbon atoms in R^{A}' is 4 or more,
   and additive, in a solvent, wherein the concentration of the carbodiimide-based condensing agent in the solvent is 0.4 to 4.0 mol/L.
[B2] The method according to [B1], wherein R^{A}' is C₄-C₁₀ alkyl, C₄-C₆ alkyl, or C₄ alkyl.
[B3] The method according to [B1] or [B2], wherein R^{B}' is C₁-C₁₀ alkyl, C₆-C₁₀ aryl, or C₇-C₁₄ arylalkyl.
   [B3-1] The method according to [B1] or [B2], wherein R^{B}' is C₁-C₁₀ alkyl.
   [B3-2] The method according to [B1] or [B2], wherein R^{B}' is C₂-C₆ alkyl.
   [B3-3] The method according to [B1] or [B2], wherein R^{B}' is C₄ alkyl.
[B4] The method according to any of [B1] to [B3], wherein the carbodiimide-based condensing agent is at least one selected from the group consisting of DsBC, 1-tert-butyl-3-ethylcarbodiimide (tBEC), di-tert-butylcarbodiimide (DtBC), 1-(3-dimethylaminopropyl)-3-ethylcarbodiimide (EDCI), N,N'-di-sec-butylcarbodiimide (DsBC), N'-(1-phenylethyl)-N-sec-butyl-methanediimine (SS26), N'-(1-methylheptyl)-N-sec-butyl-methanediimine (SS27), N,N'-bis(1-methylbutyl)methanediimine (SS28), N,N'-bis(1-ethylpropyl)methanediimine (SS29), and N'-(1-ethylpropyl)-N-(1-methylbutyl)methanediimine (SS30).
   [B4-1] The method according to any of [B1] to [B3], wherein the carbodiimide-based condensing agent is at least one selected from the group consisting of DsBC, 1-tert-butyl-3-ethylcarbodiimide (tBEC), di-tert-butylcarbodiimide (DtBC), and 1-(3-dimethylaminopropyl)-3-ethylcarbodiimide (EDCI).
[B5] The method according to any of [B1] to [B3], wherein the carbodiimide-based condensing agent includes at least one selected from the group consisting of N,N'-di-sec-butylcarbodiimide (DsBC), N'-(1-phenylethyl)-N-sec-butyl-methanediimine (SS26), N'-(1-methylheptyl)-N-sec-butyl-methanediimine (SS27), N,N'-bis(1-methylbutyl)methanediimine (SS28), N,N'-bis(1-ethylpropyl)methanediimine (SS29), and N'-(1-ethylpropyl)-N-(1-methylbutyl)methanediimine (SS30).
   [B5-1] The method according to any of [B1] to [B3], wherein the carbodiimide-based condensing agent includes at least one selected from the group consisting of N,N'-di-sec-butylcarbodiimide (DsBC), N'-(1-phenylethyl)-N-sec-butyl-methanediimine (SS26), N'-(1-methylheptyl)-N-sec-butyl-methanediimine (SS27), N,N'-bis(1-methylbutyl)methanediimine (SS28), or N'-(1-ethylpropyl)-N-(1-methylbutyl)methanediimine (SS30).
   [B5-2] The method according to any of [B1] to [B3], wherein the carbodiimide-based condensing agent is N,N'-di-sec-butylcarbodiimide (DsBC), N'-(1-phenylethyl)-N-sec-butyl-methanediimine (SS26), N'-(1-methylheptyl)-N-sec-butyl-methanediimine (SS27), N,N'-bis(1-methylbutyl)methanediimine (SS28), or N'-(1-ethylpropyl)-N-(1-methylbutyl)methanediimine (SS30).
   [B5-3] The method according to any of [B1] to [B3], wherein the carbodiimide-based condensing agent is DsBC.
   [B5-4] The method according to any of [B1] to [B3], wherein the carbodiimide-based condensing agent is N'-(1-phenylethyl)-N-sec-butyl-methanediimine (SS26).
   [B5-5] The method according to any of [B1] to [B3], wherein the carbodiimide-based condensing agent is N'-(1-methylheptyl)-N-sec-butyl-methanediimine (SS27).
   [B5-6] The method according to any of [B1] to [B3], wherein the carbodiimide-based condensing agent is N,N'-bis(1-methylbutyl)methanediimine (SS28).
   [B5-7] The method according to any of [B1] to [B3], wherein the carbodiimide-based condensing agent is N,N'-bis(1-ethylpropyl)methanediimine (SS29).
   [B5-8] The method according to any of [B1] to [B3], wherein the carbodiimide-based condensing agent is N'-(1-ethylpropyl)-N-(1-methylbutyl)methanediimine (SS30).
[B6] The method according to any of [B1] to [B5], wherein the additive is at least one selected from the group consisting of HOAt, HOBt, HOOBt, and Oxyma.
[B7] The method according to any of [B1] to [B5], wherein the additive is at least one selected from the group consisting of HOAt, HOOBt, and Oxyma.
[B8] The method according to any of [B1] to [B5], wherein the additive is HOAt, HOOBt, or Oxyma.
[B9] The method according to any of [B1] to [B8], wherein a concentration of the carbodiimide-based condensing agent in the solvent is 0.5 mol/L or more, 0.6 mol/L or more, or 0.7 mol/L or more.
[B10] The method according to any of [B1] to [B9], wherein a concentration of the carbodiimide-based condensing agent in the solvent is 3.0 mol/L or less, 2.0 mol/L or less, or 1.0 mol/L or less in the condensation step.
[B11] The method according to any of [B1] to [B 10], wherein a concentration of the carbodiimide-based condensing agent in the solvent is 0.5 to 3.0 mol/L, 0.6 to 2.0 mol/L, or 0.7 to 1.0 mol/L in the condensation step.
[B12] The method according to any of [B1] to [B 10], wherein a concentration of the carbodiimide-based condensing agent in the solvent is 0.7 to 1.0 mol/L in the condensation step.
[B13] The method according to any of [B1] to [B12], wherein the solid phase is a membrane or a resin for solid phase synthesis.
[B14] The method according to any of [B1] to [B12], wherein the solid phase is a membrane.
[B15] The method according to [B14], wherein the membrane is a cellulose membrane, a polypropylene membrane, or a polyaminoethylmethacrylamide membrane.
[B16] The method according to [B14], wherein the membrane is a cellulose membrane.
[B17] The method according to any of [B1] to [B12], wherein the solid phase is a resin for solid phase synthesis.
[B18] The method according to [B 17], wherein the resin for solid phase synthesis is a chlorotrityl (CTC) resin, a trityl (Trt) resin, a SASRIN resin, a Rink amide resin, a Merrifield resin, or a Wang resin.
[B19] The method according to [B 17], wherein the resin for solid phase synthesis is a chlorotrityl (CTC) resin.
[B20] The method according to any of [B1] to [B19], wherein the solid phase and the first amino acid or first peptide are linked via a photo-cleavable site, a disulfide bond, or an acid-labile site.
   [B20-1] The method according to any of [B1] to [B19], wherein the solid phase and the first amino acid or first peptide are supported via a photo-cleavable site.
   [B20-2] The method according to any of [B1] to [B19], wherein the solid phase and the first amino acid or first peptide are linked via a disulfide bond.
   [B20-3] The method according to any of [B1] to [B19], wherein the solid phase and the first amino acid or first peptide are linked via an acid-labile site.
[B21] The method according to any of [B20-1], wherein the photo-cleavable site has a nitroveratryloxycarbonyl residue or a coumarin residue.
   [B21-1] The method according to [B20-1], wherein the photo-cleavable site is a nitroveratryloxycarbonyl residue.
[B22] The method according to [B20-3], wherein the acid-labile site has a trityl ester structure, a chlorotrityl ester structure, an alkoxybenzyl ether structure, a trialkoxybenzylaminocarbonyl structure, a dialkoxyphenyl-alkoxyphenylmethylaminocarbonyl structure, or an alkoxyxanthen-9-ylaminocarbonyl structure.
   [B22-1] The method according to [B20-3], wherein the acid-labile site has a chlorotrityl ester structure.
[B23] The method according to any of [B1] to [B22], wherein the carbodiimide-based condensing agent is used at 1.0 equivalent or more, 1.1 equivalents or more, or 1.2 equivalents or more with respect to the second amino acid or second peptide in the condensation step.
[B24] The method according to any of [B1] to [B23], wherein the carbodiimide-based condensing agent is used at 5.0 equivalents or less, 4.0 equivalents or less, 3.0 equivalents or less, 2.0 equivalents or less, or 1.5 equivalents or less with respect to the second amino acid or second peptide in the condensation step.
[B25] The method according to any of [B1] to [B24], wherein the carbodiimide-based condensing agent is used at 1.0 to 5.0 equivalents, 1.1 to 4.0 equivalents, 1.1 to 3.0 equivalents, 1.2 to 2.0 equivalents, or 1.2 to 1.5 equivalents with respect to the second amino acid or second peptide in the condensation step.
[B26] The method according to [B25], wherein the carbodiimide-based condensing agent is used at 1.2 to 1.5 equivalents with respect to the second amino acid or second peptide in the condensation step.
[B27] The method according to any of [B1] to [B26], wherein the additive is used at 0.01 equivalents or more, 0.05 equivalents or more, 0.1 equivalents or more, 0.2 equivalents or more, or 0.3 equivalents or more with respect to the second amino acid or second peptide in the condensation step.
[B28] The method according to any of [B1] to [B27], wherein the additive is used at 1.0 equivalent or less, 0.9 equivalents or less, 0.8 equivalents or less, or 0.7 equivalents or less with respect to the second amino acid or second peptide in the condensation step.
[B29] The method according to any of [B1] to [B28], wherein the additive is used at 0.05 to 1.0 equivalent, 0.1 to 0.9 equivalents, 0.2 to 0.8 equivalents, or 0.3 to 0.7 equivalents with respect to the second amino acid or second peptide in the condensation step.
[B30] The method according to any of [B1] to [B28], wherein the additive is used at 0.3 to 0.7 equivalents with respect to the second amino acid or second peptide in the condensation step.
[B31] The method according to any of [B1] to [B30], wherein the first amino acid or an amino acid at the N-terminus of the first peptide is a non-natural amino acid.
[B32] The method according to any of [B1] to [B31], wherein the alkyl at the N-alkylamino group of the first amino acid or the alkyl at the N-alkylamino group of the amino acid at the N-terminus of the first peptide is C₁-C₁₀ alkyl, C₁-C₆ alkyl, or C₁-C₃ alkyl, wherein the alkyl is optionally substituted with one or more groups independently selected from halogen, cyano, C₁-C₆ alkoxy, C₃-C₆ cycloalkyl, C₂-C₆ alkenyl, C₂-C₆ alkynyl, or C₆-C₁₀ aryl.
[B33] The method according to any of [B1] to [B32], wherein the first amino acid or an amino acid at the N-terminus of the first peptide is an N-alkylamino acid in which the alkyl has 1 to 8 carbon atoms, provided that the alkyl is optionally substituted with one or more groups independently selected from halogen, cyano, C₁-C₆ alkoxy, C₃-C₆ cycloalkyl, C₂-C₆ alkenyl, C₂-C₆ alkynyl, or C₆-C₁₀ aryl.
[B34] The method according to any of [B1] to [B32], wherein the first amino acid or an amino acid at the N-terminus of the first peptide is an N-alkylamino acid, an N-alkylamino acid in which the alkyl has 1 to 8 carbon atoms, an N-alkylamino acid in which the alkyl has 1 to 6 carbon atoms, an N-alkylamino acid in which the alkyl has 1 to 3 carbon atoms, or an N-methylamino acid.
[B35] The method according to any of [B1] to [B34], wherein the alkyl at the N-alkylamino group of the first amino acid or the alkyl at the N-alkylamino group of the amino acid at the N-terminus of the first peptide is a methyl group.
[B36] The method according to any of [B1] to [B35], wherein the second amino acid or an amino acid at the C-terminus of the second peptide is a non-natural amino acid.
[B37] The method according to any of [B1] to [B36], wherein the second amino acid or an amino acid at the C-terminus of the second peptide is an α,α-di-substituted amino acid, a β-branched amino acid, or an N-alkylamino acid.
[B38] The method according to any of [B1] to [B37], wherein a protecting group of the protected amino group is a group represented by the following formula (1): 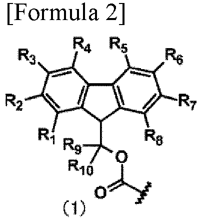 wherein, R₁ to R₈ are each independently selected from the group consisting of hydrogen, C₁-C₈ alkyl, C₁-C₈ fluoroalkyl, halogen, sulfo, and trimethylsilyl; and R₉ to R₁₀ are each independently hydrogen or methyl.
[B39] The method according to any of [B1] to [B37], wherein a protecting group of the protected amino group is a Fmoc group, a Fmoc (2,7tb) group, a Fmoc (1Me) group, a Fmoc (2F) group, a Fmoc (2,7Br) group, a mio-Fmoc group, a dio-Fmoc group, a tdf-Fmoc group, a Fmoc (2TMS) group, a Fmoc (2so3h) group, an sm-Fmoc group, or an rm-Fmoc group.
[B40] The method according to any of [B1] to [B37], wherein a protecting group of the protected amino group is a Fmoc group.
[B41] The method according to any of [B1] to [B40], wherein the condensation step is performed in a solvent.
[B42] The method according to [B41], wherein the solvent includes at least one selected from the group consisting of an amide-based solvent, a urea-based solvent, an ether-based solvent, a halogen-based solvent, a nitrile-based solvent, and a benzene-based solvent.
[B43] The method according to [B42], wherein the solvent is an amide-based solvent.
[B44] The method according to [B43], wherein the amide-based solvent is selected from the group consisting of DMF, NMP, DMA, NEP, NBP and formamide.
[B45] The method according to [B42], wherein the solvent is a urea-based solvent.
[B46] The method according to [B45], wherein the urea-based solvent is selected from the group consisting of DMI and DMPU.
[B47] The method according to [B42], wherein the solvent is an ether-based solvent.
[B48] The method according to[B47], wherein the ether-based solvent is selected from the group consisting of tetrahydrofuran, 2-methyltetrahydrofuran, and 4-methyltetrahydropyran.
[B49] The method according to [B42], wherein the solvent is a halogen-based solvent.
[B50] The method according to [B49], wherein the halogen-based solvent is selected from the group consisting of dichloromethane and 1,2-dichloroethane.
[B51] The method according to [B42], wherein the solvent is a nitrile-based solvent.
[B52] The method according to [B51], wherein the nitrile-based solvent is acetonitrile.
[B53] The method according to [B42], wherein the solvent is a benzene-based solvent.
[B54] The method according to [B53], wherein the benzene-based solvent is selected from the group consisting of benzene, toluene, and xylene.
[B55] The method according to any of [B1] to [B54], wherein the condensation step is performed at 0°C to 100°C, 10°C to 80°C, 10°C to 60°C, 10°C to 50°C, 10°C to 40°C, 10°C to 35°C, 15°C to 60°C, 20°C to 60°C, 20°C to 40°C, or 25°C to 40°C.
[B56] The method according to any of [B1] to [B54], wherein the condensation step is performed at 20°C to 60°C.
[B57] The method according to any of [B1] to [B56], wherein the condensation step is repeated 2 or more times.
[B58] The method according to any of [B1] to [B56], wherein the condensation step is repeated 2 times.
[B59] The method according to any of [B1] to [B58], further comprising, after the condensation step, a deprotection step of deprotecting a protecting group of the protected amino group and/or protected hydroxy group in the second amino acid or second peptide supported on a solid phase.
[B60] The method according to [B59], further comprising a washing step of washing the solid phase between the condensation step and the deprotection step.
[B61] The method according to [B59] or [B60], wherein the condensation step and the deprotection step are repeated a plurality of times provided that a plurality of the second amino acids and/or second peptides used in the plurality of condensation steps may be each the same or different.
[B62] Use of a carbodiimide-based condensing agent represented by formula (A') in the method according to any of [B1] to [B61].
[B63] Use of DsBC in the method according to any of [B1] to [B61].
   [B63-1] Use of N'-(1-phenylethyl)-N-sec-butyl-methanediimine (SS26) in the method according to any of [B1] to [B61].
   [B63-2] Use of N'-(1-methylheptyl)-N-sec-butyl-methanediimine (SS27) in the method according to any of [B1] to [B61].
   [B63-3] Use of N,N'-bis(1-methylbutyl)methanediimine (SS28) in the method according to any of [B1] to [B61].
   [B63-4] Use of N'-(1-ethylpropyl)-N-(1-methylbutyl)methanediimine (SS30) in the method according to any of [B1] to [B61].

The methods for producing a peptide compound by the solid phase method have been described in [B1] to [B63], but these production methods can also be applied to a liquid phase method. That is, one aspect of the present invention provides the following [B1']. [B1'] may include features of [B2] to [B63] except for solid phase. [B1'] A method for producing a peptide compound by a liquid phase method, the method comprising:
a preparation step of preparing a first amino acid having an N-alkylamino group or a first peptide having an N-alkylamino group at the N-terminus; and
a condensation step of condensing the first amino acid or first peptide, and a second amino acid having a protected amino group and/or protected hydroxy group and a carboxy group, or a second peptide having a protected amino group and/or protected hydroxy group and a carboxy group in the presence of at least one carbodiimide-based condensing agent represented by formula (A') described above and an additive, in a solvent, wherein a concentration of the carbodiimide-based condensing agent in the solvent is 0.4 to 4.0 mol/L.

According to such a method, the peptide compound of interest can be obtained with a high reaction conversion rate.

The present invention also provides, for example, the following [C1] to [C16].
[C1] A method for producing a peptide compound supported on a membrane, comprising the method according to any of [A1] to [A64] or [B1] to [B63].
[C2] The method according to [C1], wherein the peptide compound supported on the membrane contains 2 or more constituent units derived from N-alkylamino acids.
[C3] The method according to [C1] or [C2], wherein the peptide compound supported on the membrane contains 3 or more, 4 or more, 5 or more, 6 or more, or 7 or more constituent units derived from N-alkylamino acids.
[C4] The method according to any of [C1] to [C3], wherein the peptide compound supported on the membrane contains 7 or more constituent units derived from N-alkylamino acids.
[C5] The method according to any of [C1] to [C4], wherein the peptide compound supported on the membrane contains a total of 7 or more, 8 or more, 9 or more, or 10 or more constituent units derived from amino acids.
[C6] The method according to [C1] to [C5], wherein the peptide compound contains a total of 10 or more constituent units derived from amino acids.
[C7] The method according to any of [C1] to [C6], wherein the peptide compound contains a total of 30 or less, 25 or less, 20 or less, 18 or less, 17 or less, 16 or less, or 15 or less constituent units derived from amino acids.
[C8] The method according to any of [C1] to [C7], wherein the peptide compound contains a total of 15 or less constituent units derived from amino acids.
[C9] A method for producing a peptide library supported on a membrane, comprising a step of performing the method according to any of [C1] to [C8] to obtain 10 or more kinds of peptide compounds supported on the membrane.
[C10] A method for producing a peptide library supported on a membrane, comprising a step of performing the method according to any of [C1] to [C8] to obtain 20 or more kinds, 40 or more kinds, 80 or more kinds, 100 or more kinds, 200 or more kinds, or 384 or more kinds of peptide compounds supported on the membrane.
[C11] A method for producing a peptide library supported on a membrane, comprising a step of performing the method according to any of [C1] to [C8] to obtain 384 or more kinds of peptide compounds supported on the membrane.
[C12] The method according to any of [C1] to [C11], wherein the method uses an automated synthesizer.
[C13] The method according to any of [C1] to [C12], further comprising a step of isolating a peptide compound from the peptide compound supported on the membrane.
[C14] The method according to [C13], wherein the peptide compound is isolated by light irradiation.
[C15] A method for producing a cyclic peptide or cyclic peptide library, comprising a step of cyclizing the peptide compound isolated by the method according to [C13] or [C14].
[C16] The method according to [C15], wherein the method uses an automated synthesizer.

The present invention also provides, for example, the following [D1] to [D6].
[D1] A peptide compound linked to a membrane via a linker, the peptide compound containing 2 or more constituent units derived from N-alkylamino acids.
[D2] The peptide compound according to [D1], wherein the peptide compound contains 3 or more, 4 or more, 5 or more, 6 or more, or 7 or more constituent units derived from N-alkylamino acids.
[D3] The peptide compound according to [D1] or [D2], wherein the peptide compound contains a total of 7 or more, 8 or more, 9 or more, 10 or more, or 11 or more constituent units derived from amino acids.
[D4] The peptide compound according to any of [D1] to [D3], wherein the peptide compound contains a total of 30 or less, 25 or less, 20 or less, 18 or less, 17 or less, 16 or less, or 15 or less constituent units derived from amino acids.
[D5] A peptide library comprising 10 or more kinds of the peptide compounds linked to a membrane via a linker according to any of [D1] to [D4].
[D6] The peptide library according to [D5], wherein the peptide library comprises 20 or more kinds, 40 or more kinds, 80 or more kinds, 100 or more kinds, 200 or more kinds, or 384 or more kinds of the peptide compounds linked to the membrane via the linker.

The present invention also provides, for example, the following [E1] to [E3].
[E1] A method of screening a compound that binds to a target molecule, the method comprising:
   (1) synthesizing a peptide library or cyclic peptide library by the method according to any of [C9] to [C16];
   (2) bringing the peptide library or cyclic peptide library into contact with a target molecule; and
   (3) selecting a peptide compound that binds to the target molecule.
[E2] The method according to [E1], wherein the target molecule is a protein, a nucleic acid, a polypeptide, or a carbohydrate chain.
[E3] The method according to [E1] or [E2], wherein the selecting a compound that binds to the target molecule is performed by surface plasmon resonance or amplified luminescent proximity homogeneous assay.

The present invention also provides, for example, the following [F1] to [F5].
[F1] A precipitation suppressor in peptide synthesis, comprising DsBC.
[F2] A precipitation suppressor in peptide synthesis, comprising N'-(1-phenylethyl)-N-sec-butyl-methanediimine (SS26).
[F3] A precipitation suppressor in peptide synthesis, comprising N'-(1-methylheptyl)-N-sec-butyl-methanediimine (SS27).
[F4] A precipitation suppressor in peptide synthesis, comprising N,N'-bis(1-methylbutyl)methanediimine (SS28).
[F5] A precipitation suppressor in peptide synthesis, comprising N'-(1-ethylpropyl)-N-(1-methylbutyl)methanediimine (SS30).

The present invention also provides, for example, the following [G1] to [G4].
[G1] N'-(1-Phenylethyl)-N-sec-butyl-methanediimine (SS26).
[G2] N'-(1-Methylheptyl)-N-sec-butyl-methanediimine (SS27).
[G3] N,N'-Bis(1-methylbutyl)methanediimine (SS28).
[G4] N'-(1-Ethylpropyl)-N-(1-methylbutyl)methanediimine (SS30).

The present invention also provides, for example, the following [H1] to [H7].
[H1] A method for producing a compound, comprising a step of forming an amide bond using N,N'-di-sec-butylcarbodiimide (DsBC), N'-(1-phenylethyl)-N-sec-butyl-methanediimine (SS26), N'-(1-methylheptyl)-N-sec-butyl-methanediimine (SS27), N,N'-bis(1-methylbutyl)methanediimine (SS28), N,N'-bis(1-ethylpropyl)methanediimine (SS29), or N'-(1-ethylpropyl)-N-(1-methylbutyl)methanediimine (SS30) as a condensing agent.
[H2] A method for producing a compound, comprising a step of forming an amide bond using N,N'-di-sec-butylcarbodiimide (DsBC) as a condensing agent.
[H3] A method for producing a compound, comprising a step of forming an amide bond using N'-(1-phenylethyl)-N-sec-butyl-methanediimine (SS26) as a condensing agent.
[H4] A method for producing a compound, comprising a step of forming an amide bond using N'-(1-methylheptyl)-N-sec-butyl-methanediimine (SS27) as a condensing agent.
[H5] A method for producing a compound, comprising a step of forming an amide bond using N,N'-bis(1-methylbutyl)methanediimine (SS28) as a condensing agent.
[H6] A method for producing a compound, comprising a step of forming an amide bond using N,N'-bis(1-ethylpropyl)methanediimine (SS29) or N'-(1-ethylpropyl)-N-(1-methylbutyl)methanediimine (SS30) as a condensing agent.
[H7] A method for producing a compound, comprising a step of forming an amide bond using N'-(1-ethylpropyl)-N-(1-methylbutyl)methanediimine (SS30) as a condensing agent.
[H8] The method according to any of [H1] to [H7], wherein the step of forming an amide bond is performed in a solid phase or a liquid phase.

In the above numberings, the number cited in the dependent item includes the branch number of the number, unless otherwise mentioned. For example, the [A2] cited in the dependent item shows that not only [A2] but also its branch numbers [A2-1] to [A2-6] are included. The same applies to other numberings.

### Advantageous Effects of Invention

According to one aspect of the present invention, the reaction conversion rate in the peptide solid-phase synthesis method, especially in the condensation reaction step, can be improved. According to another aspect of the present invention, a method for efficiently producing high-purity peptide compounds by employing amidation conditions that enable the application to automated synthesis by avoiding crystal precipitation in the peptide solid-phase synthesis method, especially in the condensation reaction step, can be provided.

### Description of Embodiments

Hereinafter, the embodiments for carrying out the present invention are described in detail. However, the present invention is not limited by the embodiments given below.

### (Terminology and the like)

The term "one or more" as used herein means a number of 1 or 2 or larger. When the term "one or more" is used in a context related to a substituent for a certain group, this term means a number from 1 to the maximum number of substituents accepted by the group. Specific examples of the term "one or more" include 1, 2, 3, 4, 5, 6, 7, 8, 9, 10, and/or larger numbers.

As used herein, the term "to" that indicates a numerical range includes values of both ends thereof. For example, "A to B" means the numerical range of A or more and B or less.

As used herein, the term "lower limit" includes both meanings of "or more" and "more than", and the term "upper limit" includes both meanings of "or less" and "less than".

As used herein, the term "about", when used in combination with a numeric value, means the value range of +10% and -10% of the numeric value.

The meaning of the term "and/or" as used herein includes any combination in which "and" and "or" are appropriately combined. Specifically, for example, the term "A, B, and/or C" includes the following 7 variations:
(i) A, (ii) B, (iii) C, (iv) A and B, (v) A and C, (vi) B and C, and (vii) A, B, and C.

The term "room temperature" as used herein means a temperature of about 20°C to about 25°C.

The term "halogen" as used herein refers to, for example, F, Cl, Br, or I.

The "alkyl" as used herein is a monovalent group derived from an aliphatic hydrocarbon by removing any one hydrogen atom, and has a subset of hydrocarbyl or hydrocarbon group structures that do not contain a heteroatom (which refers to an atom other than carbon and hydrogen atoms) or an unsaturated carbon-carbon bond, and contain hydrogen and carbon atoms in the backbone. The alkyl includes not only a linear form but also a branched form. Specific examples of the alkyl include alkyl having 1 to 20 carbon atoms (C₁ to C₂₀; hereinafter, "Cₚ to C_{q}" means that the number of carbon atoms is p to q), preferably C₁ to C₁₀ alkyl, more preferably C₁ to C₆ alkyl. Examples of the alkyl specifically include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1,2,2-tetramethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, and 1-ethyl-1-methylbutyl.

Specific examples of "C₃-C₁₀ secondary alkyl" include i-propyl, s-butyl, s-pentyl, 3-pentyl, 1,2-dimethylpropyl, 1,2,2-trimethylpropyl, 1,2-dimethylbutyl, 1,3-dimethylbutyl, and 1-ethylbutyl. Specific examples of "C₄-C₁₀ secondary alkyl having asymmetric carbon" include s-butyl, s-pentyl, 1,2-dimethylpropyl, 1,2-dimethylbutyl, 1,3-dimethylbutyl, and 1-ethylbutyl.

Specific examples of "C₄-C₁₀ tertiary alkyl" include t-butyl, t-pentyl, 1,1,2-trimethylpropyl, 1,1,2,2-tetramethylpropyl, 1,1-dimethylbutyl, and 1-ethyl-1-methylbutyl. Specific examples of " C₄-C₁₀ tertiary alkyl having asymmetric carbon" include 1-ethyl-1-methylbutyl.

The term "alkenyl" as used herein is a monovalent group having at least one double bond (two adjacent SP2 carbon atoms). Depending on the conformation of the double bond and a substituent (if present), the geometric morphology of the double bond can assume entgegen (E) or zusammen (Z) and cis or trans conformations. The alkenyl includes not only a linear form but also a branched form. Preferred examples of the alkenyl include C₂-C₁₀ alkenyl, and more preferred examples thereof include C₂-C₆ alkenyl. Specific examples thereof include vinyl, allyl, 1-propenyl, 2-propenyl, 1-butenyl, 2-butenyl (including cis and trans), 3-butenyl, pentenyl, 3-methyl-2-butenyl, and hexenyl.

The term "alkynyl" as used herein is a monovalent group having at least one triple bond (two adjacent SP carbon atoms). The alkynyl includes not only a linear form but also a branched form. Preferred examples of the alkynyl include C₂-C₁₀ alkynyl, and more preferred examples thereof include C₂-C₆ alkynyl. Specific examples thereof include ethynyl, 1-propynyl, propargyl, 3-butynyl, pentynyl, hexynyl, 3-phenyl-2-propynyl, 3-(2'-fluorophenyl)-2-propynyl, 2-hydroxy-2-propynyl, 3-(3-fluorophenyl)-2-propynyl, and 3-methyl-(5-phenyl)-4-pentynyl.

The term "cycloalkyl" as used herein means a saturated or partially saturated cyclic monovalent aliphatic hydrocarbon group and includes a monocyclic ring, a bicyclo ring, and a spiro ring. Preferred examples of the cycloalkyl include C₃-C₈ cycloalkyl. Specific examples thereof include cyclopropyl, cyclobutyl, cyclopentyl, cyclohexyl, cycloheptyl, cyclooctyl, bicyclo[2.2.1]heptyl, and spiro[3.3]heptyl.

The term "aryl" as used herein means a monovalent aromatic hydrocarbon ring and an aromatic hydrocarbon ring group. Preferred examples of the aryl include C₆-C₁₀ aryl. Specific examples of the aryl include phenyl and naphthyl (e.g., 1-naphthyl and 2-naphthyl).

The term "heteroaryl" as used herein means an aromatic cyclic monovalent group containing 1 to 5 heteroatoms in addition to a carbon atom, and an aromatic heterocyclic group. The ring may be a monocyclic ring or a condensed ring with another ring, and may be partially saturated. The number of atoms constituting the ring of heteroaryl is preferably 5 to 10 (5- to 10-membered heteroaryl), and more preferably 5 to 7 (5- to 7-membered heteroaryl). Specific examples of the heteroaryl include furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyrimidyl, pyridazinyl, pyrazinyl, triazinyl, benzofuranyl, benzothienyl, benzothiadiazolyl, benzothiazolyl, benzoxazolyl, benzoxadiazolyl, benzimidazolyl, benzotriazolyl, indolyl, isoindolyl, indazolyl, azaindolyl, quinolyl, isoquinolyl, cinnolinyl, quinazolinyl, quinoxalinyl, benzodioxolyl, indolizinyl, imidazopyridyl, pyrazolopyridyl, imidazopyridyl, triazolopyridyl, pyrrolopyrazinyl, and flopyridyl.

The term "arylalkyl (aralkyl)" as used herein means a group in which at least one hydrogen atom of the "alkyl" defined above is replaced with the "aryl" as defined above. As the arylalkyl, C₇-C₁₄ arylalkyl is preferred, and C₇-C₁₀ arylalkyl is more preferred. Specific examples of the arylalkyl include benzyl, phenethyl, and 3-phenylpropyl.

The term "alkoxy" as used herein means an oxy group to which the "alkyl" as defined above is bonded. Preferred examples of the alkoxy include C₁-C₆ alkoxy. Specific examples of the alkoxy include methoxy, ethoxy, 1-propoxy, 2-propoxy, n-butoxy, i-butoxy, s-butoxy, t-butoxy, pentyloxy, and 3-methylbutoxy.

The term "amino" as used herein means -NH₂ in the narrow sense and means -NRR' in the broad sense. In this context, R and R' are each independently selected from hydrogen, alkyl, alkenyl, alkynyl, cycloalkyl, heterocyclyl, aryl, or heteroaryl, or R and R' form a ring together with the nitrogen atom bonded thereto. Preferred examples of the amino include -NH₂, mono-C₁ to C₆ alkylamino, di-C₁ to C₆ alkylamino, and 4- to 8-membered cyclic amino.

The term "monoalkylamino" as used herein means a group of the "amino" as defined above in which R is hydrogen and R' is the "alkyl" as defined above. Preferred examples of the monoalkylamino include mono-C₁ to C₆ alkylamino. Specific examples of the monoalkylamino include methylamino, ethylamino, n-propylamino, i-propylamino, n-butylamino, s-butylamino, and t-butylamino.

The term "dialkylamino" as used herein means a group of the "amino" as defined above in which R and R' are each independently the "alkyl" as defined above. Preferred examples of the dialkylamino include di-C₁ to C₆ alkylamino. Specific examples of the dialkylamino include dimethylamino and diethylamino.

The term "cyclic amino" as used herein means a group of the "amino" defined above in which R and R' form a ring together with the nitrogen atom bonded thereto. Preferred examples of the cyclic amino include 4- to 8-membered cyclic amino. Specific examples of the cyclic amino include 1-azetidyl, 1-pyrrolidyl, 1-piperidyl, 1-piperazyl, 4-morpholinyl, 3-oxazolidyl, 1,1-dioxidothiomorpholinyl-4-yl, and 3-oxa-8-azabicyclo[3.2.1]octan-8-yl.

If, in a production method described herein, a defined group undergoes undesired chemical conversion under conditions of the production method, the compounds can be produced, for example, by means such as protection or deprotection of the functional group. Here, for operations of selection and desorption of a protecting group, for example, methods described in "Greene's, "Protective Groups in Organic Synthesis" (5th edition, John Wiley & Sons 2014)" can be mentioned and may be appropriately used according to reaction conditions. It is also possible to change the order of reaction steps, such as steps of introducing a substituent, if necessary.

Examples of the "protecting group for an amino group" as used herein include a carbamate-type protecting group, an amide-type protecting group, an aryl sulfonamide-type protecting group, an alkyl amine-type protecting group, and an imide-type protecting group. Specific examples thereof include Fmoc, Boc, Alloc, Cbz, Teoc, trifluoroacetyl, pentafluoropropionyl, phthaloyl, benzenesulfonyl, tosyl, nosyl, dinitronosyl, t-butyl, trityl, cumyl, benzylidene, 4-methoxybenzylidene, and diphenylmethylidene.

The term "protected amino group" as used herein means an amino group protected with any protecting group. Specific examples of the protected amino group include an amino group protected with the protecting group for the amino group described above.

Examples of the "protecting group for a hydroxy group" as used herein include an alkyl ether type protecting group, an aralkyl ether type protecting group, a silyl ether type protecting group, and a carbonate ester type protecting group. Specific examples of the protecting group for hydroxy include methoxymethyl, benzyloxymethyl, tetrahydropyranyl, t-butyl, allyl, 2,2,2-trichloroethyl, benzyl, 4-methoxybenzyl, trimethylsilyl, triethylsilyl, triisopropylsilyl, t-butyldimethylsilyl, t-butyldiphenylsilyl, methoxycarbonyl, 9-fluorenylmethoxycarbonyl, and 2,2,2-trichloroethoxycarbonyl.

The term "protected hydroxy group" as used herein means a hydroxy group protected with any protecting group. Specific examples of the protected hydroxy group include a hydroxy group protected with the protecting group for the hydroxy group described above.

Examples of the "protecting group for a carboxy group" as used herein include an alkyl ester-type protecting group, a benzyl ester-type protecting group, and a substituted alkyl ester-type protecting group. Specific examples of the protecting group for a carboxy group include methyl, ethyl, t-butyl, benzyl, trityl, cumyl, methoxytrityl, 2-(trimethylsilyl)ethyl, 2,2,2-trichloroethyl, and allyl.

The term "protected carboxy group" as used herein means a carboxy group protected with any protecting group. Specific examples of the protected carboxy group include a carboxy group protected with the protecting group for the carboxy group described above.

The "peptide" as used herein is not particularly limited as long as it is a peptide formed from natural amino acids and/or non-natural amino acids linked via an amide bond or an ester bond. The number of residues contained in the peptide is preferably 5 to 30 residues, more preferably 7 to 15 residues, and further preferably 9 to 13 residues. The peptide may be a linear peptide or a cyclic peptide.

The term "peptide compound" as used herein is not particularly limited as long as it is a peptide compound having natural amino acids and/or non-natural amino acids linked via an amide bond and partially an ester bond, and the peptide compound is preferably one having 5 to 30 residues, more preferably one having 8 to 15 residues, still more preferably one having 9 to 13 residues. The peptide compound synthesized in the present embodiments contains preferably at least three N-substituted amino acids, more preferably at least five or more N-substituted amino acids in one peptide. These N-substituted amino acids may be present continuously or discontinuously in the peptide compound. The peptide compound in the present embodiments may be linear or cyclic, and a cyclic peptide compound is preferred. The number of residues contained in the peptide compound is preferably 5 to 30 residues, more preferably 8 to 15 residues, and further preferably 9 to 13 residues. The peptide compound preferably includes at least three N-substituted amino acids, more preferably at least five or more N-substituted amino acids in one peptide compound. These N-substituted amino acids may be present continuously or discontinuously in the peptide compound. The peptide compound in the present embodiments may be linear or cyclic, and a cyclic peptide compound is preferred.

The "cyclic peptide compound" as used herein is a cyclic peptide compound that can be obtained by bonding any groups of a linear peptide compound to each other to cyclize. As aspects of cyclization of the cyclic peptide compound, the cyclization may be in any form, for example, cyclization with a carbon-nitrogen bond such as an amide bond, cyclization with a carbon-oxygen bond such as an ester bond or ether bond, cyclization with a carbon-sulfur bond such as a thioether bond, cyclization with a carbon-carbon bond, or cyclization by heterocyclic construction. Among these, cyclization via a covalent bond such as an amide bond, a carbon-sulfur bond, or a carbon-carbon bond is preferred. Cyclization with an amide bond is particularly preferred, and the carboxy group or amino group used for cyclization may be positioned on the main chain or on a side chain. Cyclization via an amide bond between a carboxy group on a side chain and an amino group at the N-terminus of the main chain is more preferred.

The term "cyclization" of a peptide compound means a formation of a cyclic portion containing 4 or more amino acid residues. The number of amino acids contained in the cyclic portion of the cyclic peptide compound is not particularly limited herein, but examples thereof include 4-20 residues, 5-15 residues, and 6-13 residues. The method for converting a linear peptide compound to a cyclic peptide compound can be carried out by performing an intramolecular bond formation reaction by the method described in Comprehensive Organic Transformations, A Guide to Functional Group Preparations, 3rd Edition (authored by R. C. Larock), March's Advanced Organic Chemistry: Reactions, Mechanisms, and Structure, 7th Edition (authored by M. B. Smith and J. March), or the like. It is also possible to further perform a functional group conversion reaction after the bond formation reaction. Examples of the bond formation reaction include a C(O)-N bond formed from a carboxylic acid and an amine; a C-O-C bond, a C(O)-O bond, and a C(S)-O bond utilizing an oxygen atom; a C(O)-S bond, a C(S)-S bond, a C-S-S-C bond, a C-S-C bond, a C-S(O)-C bond, a C-S(O₂)-C bond utilizing a sulfur atom; and a C-N-C bond, a C=N-C bond, an N-C(O)-N bond, an N-C(S)N bond, and a C(S)-N bond utilizing a nitrogen atom. Further examples thereof include a C-C bond formation reaction catalyzed by a transition metal, such as the Suzuki reaction, the Heck reaction, and the Sonogashira reaction. Examples of the functional group conversion reaction that is further performed after the bond formation reaction include an oxidation reaction or a reduction reaction. Specific examples thereof include a reaction in which a sulfur atom is oxidized and converted to a sulfoxide group or a sulfone group. Other examples thereof include a reduction reaction in which a triple bond or a double bond among carbon-carbon bonds is reduced and converted to a double bond or a single bond. Two amino acids may be bonded at the main chain of the amino acid to form a closed ring structure by a peptide bond, or a covalent bond may be formed between two amino acids via, for example, a bond between side chains or between a side chain and the main chain of the two amino acids.

The "membrane" herein is not particularly limited as long as it can be used for the synthesis of a peptide compound by a solid phase method. Specific examples of such a membrane include a cellulose membrane, a polypropylene membrane, or a polyaminoethylmethacrylamide membrane, and preferably a cellulose membrane.

The "amino group-modified membrane" as used herein refers to those that have been chemically modified to have an amino group on the surface of, for example, a cellulose membrane or polypropylene membrane, and are not particularly limited as long as they can be used in the synthesis of peptide compounds by a solid phase method. Specifically, such amino group-modified membranes can be prepared by ester bonding a carboxy group of β-alanine to the hydroxyl group of a cellulose membrane by known methods (Preparation of a Cellulose Membrane for Spot Synthesis), for example, those described in, Methods Mol. Biol., 2009, 570, 157-174, or the like. Specific examples of the membrane having already modified with amino groups include Amino-PEG500-UC540 Sheets and CelluSpots 384 frame with acid stable discs as a membrane processed in a disc form and mounted on the frame, which are commercially available from CEM Corporation (formerly Intavis Bioanalytical Instruments AG) or the like. Note that "membrane disc" may be described as "membrane" herein.

The amount and rate supported on the solid phase including a membrane herein is not particularly limited as long as it can be used for the synthesis of a peptide compound by a solid phase method. In some aspects, the amount and rate supported can be reduced when elongating amino acids. The methods of appropriately reducing the amount and rate supported are not particularly limited, and any method can be employed. For example, the methods of mixing Fmoc-Photo-Linker and 4-phenoxybutyric acid in any proportion to elongate can be employed.

The "resin for solid phase synthesis" as used herein is not particularly limited as long as it can be used for the synthesis of a peptide compound by a solid phase method. Specific examples of such a resin for solid phase synthesis include those removable under acidic conditions, such as CTC resin, NovaSyn TGT resin (TGT resin), Wang resin, SASRIN resin, tritylchloride resin (Trt resin), 4-methyltritylchloride resin (Mtt resin) and 4-methoxytritylchloride resin (Mmt resin). The resin can be appropriately selected in line with a functional group of an amino acid to be used. For example, when a carboxy group (main-chain carboxy group or side-chain carboxy group such as Asp or Glu) or a hydroxy group on the aromatic ring (phenol group such as Tyr) is used as the functional group of the amino acid, tritylchloride resin (Trt resin) or 2-chlorotritylchloride resin (CTC resin) is preferably used as the resin. When an aliphatic hydroxy group (aliphatic alcohol group such as Ser or Thr) is used as the functional group on the amino acid, tritylchloride resin (Trt resin), 2-chlorotritylchloride resin (CTC resin) or 4-methyltritylchloride resin (Mtt resin) is preferably used as the resin. The resin herein is sometimes referred to as resin.

The type of the polymer constituting the resin is also not particularly limited. In the case of the resin constituted by polystyrene, either resin of 100-200 mesh or 200-400 mesh may be used. The crosslinking ratio is not particularly limited, and resins crosslinked with DVB (divinylbenzene) at 1% are preferable. Examples of the type of polymer constituting the resin include TentaGel (registered trademark) and ChemMatrix (registered trademark).

The compound described herein can be a salt thereof or a solvate thereof. Examples of the salt of the compound include hydrochloride; hydrobromide; hydroiodide; phosphate; phosphonate; sulfate; sulfonate such as methanesulfonate and p-toluenesulfonate; carboxylate such as acetate, citrate, malate, tartrate, succinate, and salicylate; alkali metal salts such as sodium salt and potassium salt; alkaline earth metal salts such as magnesium salt and calcium salt; and ammonium salts such as ammonium salt, alkylammonium salt, dialkylammonium salt, trialkylammonium salt, and tetraalkylammonium salt. These salts are produced by, for example, bringing the compound into contact with an acid or a base. The solvate of the compound is one in which the compound and a solvent together form a molecular aggregate, and is not particularly limited as long as it is a solvate formed by a solvent. Examples of the solvate include a hydrate, an alcohol solvate (such as ethanol solvate, methanol solvate, 1-propanol solvate, or 2-propanol solvate), and not only solvates formed with a single solvent such as dimethyl sulfoxide, but also solvates formed with a plurality of solvents per one molecule of the compound, or solvates formed with a plurality of types of solvents per one molecule of the compound. When the solvent is water, the solvate is called a hydrate. The solvate of the compound of the present invention is preferably a hydrate. Specific examples of such a hydrate include a mono- to deca-hydrate, preferably a mono- to penta-hydrate, further preferably a mono- to tri-hydrate.

The term "amino acid" as used herein includes a natural amino acid and a non-natural amino acid (sometimes also referred to as an amino acid derivative). The term "amino acid" as used herein may mean an amino acid residue. The term "natural amino acid" as used herein refers to glycine (Gly), alanine (Ala), serine (Ser), threonine (Thr), valine (Val), leucine (Leu), isoleucine (Ile), phenylalanine (Phe), tyrosine (Tyr), tryptophan (Trp), histidine (His), glutamic acid (Glu), aspartic acid (Asp), glutamine (Gln), asparagine (Asn), cysteine (Cys), methionine (Met), lysine (Lys), arginine (Arg), and proline (Pro). Non-natural amino acids (amino acid derivatives) are not particularly limited, and examples thereof include a β-amino acid, a D-type amino acid, an N-substituted amino acid, an α,α-di-substituted amino acid, an amino acid having a side chain different from that of natural amino acids, and a hydroxycarboxylic acid. As the amino acids as used herein, amino acids having any conformation are acceptable. The selection of a side chain of the amino acid is not particularly limited, and the side chain is freely selected from, in addition to a hydrogen atom, for example, an alkyl group, an alkenyl group, an alkynyl group, an aryl group, a heteroaryl group, an aralkyl group, a heteroaralkyl group, a cycloalkyl group, and a spiro-bonded cycloalkyl group. A substituent may be added to each of the side chains. Such a substituent is not limited either, and can be one or two or more substituents each independently freely selected from any substituents including, for example, a halogen atom, an O atom, a S atom, a N atom, a B atom, a Si atom, or a P atom. That is, examples of the side chain include an alkyl group, an alkoxy group, an alkoxyalkyl group, an alkenyl group, an alkynyl group, an aryl group, a heteroaryl group, an aralkyl group, or a cycloalkyl group that is optionally substituted, or oxo, aminocarbonyl, and a halogen atom. In one non-limiting aspect, the amino acid in the present specification may be a compound having a carboxy group and an amino group in the same molecule (even in this case, the amino acid also includes imino acids such as proline and hydroxyproline).

The amino group of the main chain of the amino acid may be unsubstituted (-NH₂) or substituted (i.e., -NHR, wherein R represents alkyl, alkenyl, alkynyl, aryl, heteroaryl, aralkyl or cycloalkyl optionally having a substituent, or a carbon chain bonded to the N atom and a carbon atom at position α may form a ring as in proline). As used herein, such an amino acid having the substituted main-chain amino group may be referred to as "N-substituted amino acid". Preferred examples of the "N-substituted amino acid" herein include, but are not limited to, N-alkylamino acid, N-C₁-C₆ alkylamino acids, N-C₁-C₄ alkylamino acids, N-methylamino acids, N-C₂-C₆ alkenylamino acids, N-allylamino acids, N-C₇-C₁₄ aralkylamino acid, N-benzylamino acid, and N-phenethylamino acid.

Specific examples of the "α,α-di-substituted amino acid" herein include Aib, (Me)Abu, (Me)Leu, (Me)Algly, (Me)Phe, (Me)Phe(3-I), 1-ACPrC, cVal, cLeu, cHex, and Athpc.

Specific examples of the "β-branched amino acid" herein include MeVal, D-MeVal, Val, Ile, MeIle, MeChg, Chg, MeGly(cPent), Gly(cPent), MeGly(cBu), Gly(cBu), MeGly(cPr), Gly(cPr), MeThr(tBu), and Thr(tBu).

Relationships between the abbreviations of α,α-di-substituted amino acids and β-branched amino acids illustrated herein and the structures thereof are shown below. The amino acids listed in the table below have the amino group protected with a Fmoc group, and relationships between the abbreviations of the amino acids having a free amino group resulting from the removal of the Fmoc group, or residues thereof and the structures thereof can also be understood from the table below. Specifically, for example, it is obvious to those skilled in the art that MeVal-OH is an amino acid having the following structure in which the Fmoc group is removed from Fmoc-MeVal-OH, and the structure of MeVal that is an amino acid residue thereof is also obvious to those skilled in the art.

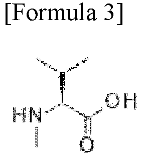

A

The "amino acid" as used herein includes all isotopes corresponding to each. The isotope of the "amino acid" is a form in which at least one atom is replaced with an atom having the same atomic number (proton number) and a different mass number (total number of protons and neutrons) at an abundance ratio different from the natural abundance ratio. Examples of the isotope contained in the "amino acid" as used herein include a hydrogen atom, a carbon atom, a nitrogen atom, an oxygen atom, a phosphorus atom, a sulfur atom, a fluorine atom, and a chlorine atom, and they include ²H , ³H; ¹³C , ¹⁴C; ¹⁵N; ¹⁷O, ¹⁸O; ³²P; ³⁵S; ¹⁸F; ³⁶Cl; and the like, respectively. For the compounds as used herein, all the compounds containing any proportions of radioactive or non-radioactive isotopic element are encompassed within the scope of the present invention.

### (Production method)

The method for producing a peptide compound by the solid phase method of the present embodiment comprises a preparation step of preparing a first amino acid or first peptide supported on a solid phase; and a condensation step of condensing the first amino acid or first peptide and a second amino acid or second peptide in the presence of a condensing agent and an additive.

In an aspect, the peptide compound obtained by the production method of the present embodiment may be an intermediate or final product of an elongation step of an amino acid or peptide by a solid phase method. The elongation step may be performed a plurality of times depending on the length of the amino acid sequence of the desired peptide compound, and the condensation step of the present embodiment may be included at least once or a plurality of times during such an elongation step. During the elongation step, methods known in the art can be used for condensation steps other than the condensation step of the present embodiment.

In an aspect, when the condensation step of the present embodiment is used in the final step of the elongation step, the condensate obtained by the condensation step of the present embodiment may be a peptide compound having the desired sequence. When the condensation step of the present embodiment is furtherelongated by known methods to obtain a peptide compound having the desired sequence, the condensate obtained by the condensation step of the present embodiment is contained in the compound as a partial structure of the peptide compound.

In the preparation step of the present embodiment, a first amino acid or first peptide supported on a solid phase is prepared. Specific examples of the solid phase include a membrane or a resin for solid phase synthesis.

In the step of isolating a peptide from a solid phase including a membrane herein, the cleavage site may be cleaved in any form, such as the cleavage of a photo-cleavable site by UV irradiation, the cleavage of a disulfide bond by the reducing conditions, the cleavage of an acid labile site by the weak acid conditions, or the like.

The solid phase and the first amino acid or first peptide may be linked via a photo-cleavable site. The photo-cleavable site is a site that is cleaved by absorbing light. The absorption wavelength at which the photo-cleavable site undergoes cleavage may be 300 nm or more, 320 nm or more, 340 nm or more, or 350 nm or more, and may be 500 nm or less, 450 nm or less, 400 nm or less, 380 nm or less, or 370 nm or less. The absorption wavelength at which the photo-cleavable site undergoes cleavage may be 300 nm or more and 500 nm or less, or 350 nm or more and 370 nm or less. The wavelength of the irradiated light may be a wavelength in the UV-A region from the viewpoint of better handling. A wavelength in the UV-A region refers to light with a wavelength of 320 to 400 nm.

The photo-cleavable site may have a nitroveratryloxycarbonyl residue or a coumarin residue.

The solid phase and the first amino acid or first peptide may be linked via a disulfide bond. The disulfide bond can be cut by the reducing conditions such as a water/DMF solution containing tris(2-carboxyethyl)phosphine to isolate the peptide of interest from the solid phase.

The solid phase and the first amino acid or first peptide may be linked via an acid labile site. The acid labile site can be degraded by acidic conditions, such as in TFA/DCM solution or TFE/DCM solution containing DIPEA, to isolate the peptide of interest from the solid phase.

The acid labile site may have a trityl ester structure, a chlorotrityl ester structure, an alkoxybenzyl ether structure, a trialkoxybenzylaminocarbonyl structure, a dialkoxyphenyl-alkoxyphenylmethylaminocarbonyl structure, or an alkoxyxanthen-9-ylaminocarbonyl structure.

The solid phase and the first amino acid or first peptide may be linked by a group capable of isolating a peptide with an acid. Specific examples of the solid phase having a group capable of isolating a peptide with an acid include resins for solid phase synthesis, such as CTC resin, NovaSyn TGT resin (TGT resin), Wang resin, SASRIN resin, tritylchloride resin (Trt resin), 4-methyltritylchloride resin (Mtt resin) and 4-methoxytritylchloride resin (Mmt resin).

When the solid phase is a membrane, an amount of 5 nmol/cm² or more and 500 nmol/cm² or less based on the Fmoc quantification method can be used as the amount of the first amino acid or first peptide supported on the membrane, and the reaction can be efficiently performed at a high amount supported of 20 nmol/cm² or more, 50 nmol/cm² or more, 100 nmol/cm² or more, and even 200 nmol/cm² or more. When the solid phase is a resin for solid phase synthesis, an amount of 0.1 mmol/g or more and 0.8 mmol/g or less based on the Fmoc quantification method can be used as the amount of the first amino acid or first peptide supported on the resin for solid phase synthesis, and the reaction can be efficiently performed at a high amount supported of 0.2 mmol/g or more, 0.3 mmol/g or more, and even 0.4 mmol/g or more.

In the condensation step (condensation reaction) of the present embodiment, the second amino acid or second peptide is used in an equal or excess amount with respect to the first amino acid or first peptide. Specifically, the molar ratio of the second amino acid or second peptide to the first amino acid or first peptide can be in a range that can be specified by a combination of a lower limit selected from the group consisting of 1, 2, 3, 5, 7, and 10, and an upper limit selected from the group consisting of 3, 5, 7, 10, 15, 25, 50, 100, 300, 500, and 700. The molar ratio of the second amino acid or second peptide to the first amino acid or first peptide is preferably 1.5 or more, more preferably 2 or more. Also, the molar ratio of the second amino acid or second peptide to the first amino acid or first peptide is preferably 50 or less, more preferably 15 or less. The molar ratio of the second amino acid or second peptide to the first amino acid or first peptide is most preferably 7 to 15.

Examples of the additive used in the condensation step of the present embodiment include Oxyma, HOBt, HOOBt, or HOAt.

In the condensation step of the present embodiment, the additive is used at a molar number that is less than the molar number of the second amino acid or second peptide. In other words, the molar ratio of the additive to the second amino acid or second peptide is less than 1. The molar ratio is preferably 0.8 or less, for example, 0.1 to 0.8. In this case, the molar ratio of the additive to the second amino acid or second peptide can be in a range that can be specified by a combination of a lower limit selected from the group consisting of 0.01, 0.05, 0.1, 0.2, 0.3, 0.4, 0.5, 0.6, and 0.7, and an upper limit selected from the group consisting of 0.2, 0.3, 0.4, 0.5, 0.6, 0.7, and 0.8. The molar ratio of the additive to the second amino acid or second peptide is more preferably 0.3 to 0.7.

The condensing agent used in the condensation step of the present embodiment includes at least one carbodiimide-based condensing agent represented by the following formula (A).

R^{A}-N=C=N-R^{B} (A)

wherein R^{A} is C₄-C₁₀ secondary or tertiary alkyl, and R^{B} is C₂-C₁₀ alkyl, C₆-C₁₀ aryl, or C₇-C₁₄ arylalkyl, and each group in R^{A} and R^{B} is optionally substituted with one or more groups independently selected from halogen, C₁-C₆ alkoxy, di-C₁-C₆ alkylamino, or 4- to 8-membered cyclic amino. Specific examples of the carbodiimide-based condensing agent represented by formula (A) include DsBC, tBEC, and DtBC.

The condensing agent used in the condensation step of the present embodiment may include at least one carbodiimide-based condensing agent represented by the following formula (A').

R^{A'}-N=C=N-R^{B'} (A')

wherein R^{A}' is C₃-C₁₀ alkyl, C₆-C₁₀ aryl, or C₇-C₁₄ arylalkyl, and R^{B'} is C₁-C₁₀ alkyl, C₆-C₁₀ aryl, or C₇-C₁₄ arylalkyl, and each group in R^{A}' and R^{B}' is optionally substituted with one or more groups independently selected from halogen, C₁-C₆ alkoxy, di-C₁-C₆ alkylamino, or 4- to 8-membered cyclic amino, provided that the total number of carbon atoms in R^{A}' is 4 or more. Specific examples of the carbodiimide-based condensing agent represented by formula (A') include DsBC, tBEC, DtBC, and EDCI.

In the condensation step of the present embodiment, the condensing agent is used at a molar number equal to or greater than the molar number of the second amino acid or second peptide. Specifically, in the condensation step of the present embodiment, the molar ratio of the condensing agent to the second amino acid or second peptide can be in a range of 1 or more, a range of 2 or more, a range of 3 or more, or a range of 4 or more. More specifically, the molar ratio of the condensing agent to the second amino acid or second peptide can be in a range that can be specified by a combination of a lower limit selected from the group consisting of 1.0, 1.1, 1.2, 1.5, 2.0, 3.0, and 4.0, and an upper limit selected from the group consisting of 1.5, 2.0, 3.0, 4.0, and 5.0. Preferred examples of the range of the molar ratio of the condensing agent to the second amino acid or second peptide include 1.0 to 5.0, 1.1 to 4.0, 1.1 to 3.0, 1.2 to 2.0, and 1.2 to 1.5.

In the condensation step of the present embodiment, regarding the molar ratios of the condensing agent and the additive to the second amino acid or second peptide,
the molar ratio of the condensing agent to the second amino acid or second peptide can be in a range that can be specified by a combination of a lower limit selected from the group consisting of 1.0, 1.1, 1.2, 1.5, 2.0, 3.0, and 4.0, and an upper limit selected from the group consisting of 1.5, 2.0, 3.0, 4.0, and 5.0, and
the molar ratio of the additive to the second amino acid or second peptide can be in a range that can be specified by a combination of a lower limit selected from a value consisting of 0.01, 0.05, 0.1, 0.2, 0.3, 0.4, 0.5, 0.6, and 0.7, and an upper limit selected from a value consisting of 0.2, 0.3, 0.4, 0.5, 0.6, 0.7, 0.8, 0.9, and 1.0.

The molar ratio of the condensing agent and the additive to the second amino acid or second peptide is preferably the second amino acid or second peptide : condensing agent : additive = about 2 : about 2.4 to 3.2 : about 0.4 to 1.4.

The condensation step of the present embodiment can be performed at a reaction temperature of 0 to 100°C, preferably 10 to 60°C, more preferably 20 to 50°C.

The condensation step of the present embodiment can be performed in a reaction time of 10 minutes to 2 days, preferably 10 minutes to 6 hours, more preferably 30 minutes to 60 minutes. The condensation step can also be repeated 2 or more times.

In the condensation step of the present embodiment, it is preferable that the additive is HOAt, HOOBt, or Oxyma, and the condensing agent is DsBC.

The condensation reaction of the present embodiment can be performed in an appropriate solvent. As the solvent, non-protonic solvents can be used, and examples thereof include an amide-based solvent, an ester-based solvent, an ether-based solvent, an alkylnitrile-based solvent, and a urea-based solvent. Examples of the amide-based solvent include DMF, DMA, and NMP. Examples of the ester-based solvent include ethyl acetate and dimethyl carbonate. Examples of the ether-based solvent include tetrahydrofuran and 2-methyltetrahydrofuran. Examples of the alkylnitrile-based solvent include acetonitrile. Examples of the urea-based solvent include DMI and TMU.

The condensation step of the present embodiment can be performed by bringing a second amino acid or second peptide, a condensing agent, and an additive into contact with a first amino acid or first peptide supported on a solid phase. The second amino acid or second peptide, the condensing agent, and the additive can be brought into contact with the first amino acid or first peptide in any order, and the second amino acid or second peptide, the condensing agent, and the additive may be brought into contact with the first amino acid or first peptide simultaneously or sequentially. All or some of the second amino acid or peptide, the condensing agent, and the additive may be pre-mixed, and then brought into contact with the first amino acid or first peptide. The second amino acid or peptide, the condensing agent, and the additive may be mixed with an appropriate solvent, and then the mixture may be brought into contact with the first amino acid or first peptide.

The method of the present embodiment may be performed using a solid-phase synthesizer. In an aspect, the method of the present embodiment can be performed by mixing a first amino acid or first peptide supported on a solid phase with a second amino acid or second peptide, a condensing agent, and an additive in an appropriate solvent. When the solid phase is a resin for solid phase synthesis, the mixing of the resin for solid phase synthesis with these reagents may be performed after a pre-treatment of swelling the resin for solid phase synthesis by bringing it into contact with an appropriate solvent to proceed the condensation reaction of interest efficiently. The amount of the solvent used in this pre-treatment can be any amount as long as the swollen resin is immersed in the solvent. For example, when DMF is used as the solvent, the amount can be 3 v/w to 15 v/w, preferably 4 v/w to 10 v/w, more preferably 4 v/w to 8 v/w. The description of the amount of solvent of 4 v/w represents that the amount of the solvent is 4 mL per 1 g of resin weight.

After completion of the reaction, the reaction solution may be discharged from the membrane or solid-phase synthesizer and excess reagents and by-products may be discharged by washing the residual membrane or resin for solid phase synthesis with an appropriate solvent to obtain the peptide compound of interest bound to the membrane or resin for solid phase synthesis. Examples of the suitable solvent for washing and/or swelling the membrane or resin for solid phase synthesis include an amide-based solvent and an alcohol-based solvent, and DMF or ethanol or 2-propanol is preferably used. These solvents may be used a plurality of times or alternately. The swollen resin for solid phase synthesis may be shrunk if needed, and for which, the resin is washed with an alcohol-based solvent or an ether-based solvent. As the alcohol-based solvent, methanol is preferred, and as the ether-based solvent, methyl t-butyl ether (MTBE) is preferred.

In an aspect, a mixture of a second amino acid or second peptide, a condensing agent, and an additive may be prepared by mixing them in a solvent before performing a condensation reaction, and then used in the condensation reaction. The mixing time is not particularly limited, but is preferably 0 minutes to 2 hours, more preferably 0 minutes to 1 hour, and even more preferably about 15 minutes.

Stirring and shaking of the resin using an automated synthesizer can be important in allowing the resin to sufficiently penetrate the reaction solution and proceeding the reaction as desired. The stirring speed, shaking speed, and frequency of them are not particularly limited, but since excessive stirring can cause physical damage to the resin, the stirring may be performed, for example, at 60 rpm for about 2 minutes per hour. If the penetration is sufficient, it is not necessary to perform the stirring and shaking.

In an aspect, the method of the present embodiment may further include a step of removing the membrane and resin for solid phase synthesis, and for this step, methods known in the art may be employed. The peptide compounds extended to the desired sequence can be separated from the membrane and resin, and isolated.

In an aspect, the method of the present embodiment may further include a step of removing the protecting group, and for this step, methods known in the art may be employed. For example, the method described in "Greene's, "Protective Groups in Organic Synthesis" (5th edition, John Wiley & Sons 2014)" may be employed for the removal of the protecting group, and these methods may be appropriately used depending on the reaction conditions. Specifically, when the amino group of the second amino acid or the amino group of the amino acid at the N-terminus of the second peptide is protected by a protecting group, the protecting group may be removed to prepare for the next condensation reaction. The protecting group may be removed simultaneously during the condensation reaction or may be removed separately from the condensation reaction.

In an aspect, the present embodiment also relates to a method for producing a cyclic peptide compound by obtaining a linear peptide compound using the method of the present embodiment, and then cyclizing the N-terminal side group and the C-terminal side group of the linear peptide compound by the method described in Comprehensive Organic Transformations, A Guide to Functional Group Preparations, 3rd Edition (authored by R. C. Larock), March's Advanced Organic Chemistry: Reactions, Mechanisms, and Structure, 7th Edition (authored by M. B. Smith and J. March) or the like, to produce the cyclic peptide compound.

It should be noted that all of the prior art cited herein are incorporated herein by reference.

### Examples

The present invention will be further illustrated with reference to Examples given below, but is not limited by Examples below. In Examples, the following abbreviations were used.
Ac: acetyl
DBU: 1,8-diazabicyclo[5.4.0]-7-undecene
DCE: 1,2-dichloroethane
DCM: dichloromethane
DGDE: diethylene glycol diethyl ether
DIC: N,N'-diisopropylcarbodiimide
DIPEA: N,N-diisopropylethylamine
DMF: N,N-dimethylformamide
DMSO: dimethyl sulfoxide
DsBC: N,N'-di-sec-butylcarbodiimide
tBEC: 1-tert-butyl-3-ethylcarbodiimide
EDCI·HCI: 1-ethyl-3-(3-dimethylaminopropyl)carbodiimide hydrochloride
FA: formic acid
Fmoc: 9-fluorenylmethyloxycarbonyl
HFIP: 1,1,1,3,3,3-hexafluoroisopropyl alcohol
HOAt: 1-hydroxy-7-azabenzotriazole
HOBt: 1-hydroxybenzotriazole
HOOBt: 3,4-dihydro-3-hydroxy-4-oxo-1,2,3-benzotriazine
MeCN: acetonitrile
NMP: N-methyl-2-pyrrolidone
Oxyma: ethyl cyano(hydroxyimino)acetate
Oxyma B: 5-(hydroxyimino)-1,3-dimethylpyrimidine-2,4,6(1H,3H,5H)-trione
PyOxim: [ethylcyano(hydroxyimino)acetat-O2]tri-1-pyrrolidinylphosphonium hexafluorophosphate
TBME: t-butyl methyl ether
TFA: trifluoroacetic acid
TFE: 2,2,2-trifluoroethanol
THF: tetrahydrofuran
THP: tetrahydropyranyl
TIPS: triisopropylsilane

The analysis conditions of LCMS and the like are as shown in Tables 1 and 2.

**[Table 1]**

| Preparative conditions | Device | Column (I.D. × Length (mm) | Mobile phase | Gradient (A/B) | Flow rate (ml/min) | Column temperature (°C) | Wavelength |
|---|---|---|---|---|---|---|---|
| SQD FA05_1 | Acquity UPLC/SQD2 | Aldrich Ascentis Express C18 (2.1 × 50) | A) 0.1% FA, H₂O | 95/5 => 0/100 (1.0 min)=> 0/100(0.4 min) | 0.9 | 35 | 210-400nm PDA total |
| | | | B) 0.1% FA CH₃CN | | | | |
| SQD FA05_2 | Acquity UPLC/SQD2 | Aldrich Ascentis Express C18 (2.1 × 50) | A) 0.1% FA, H₂O | 95/5 => 0/100 (1.0 min)=> 0/100(0.4 min) | 1.0 | 35 | 210-400nm PDA total |
| | | | B) 0.1% FA CH₃CN | | | | |
| SQD FA05_3 | Acquity UPLC/SQD2 | Aldrich Ascentis Express C18 (2.1 × 50) | A) 0.1% FA, H₂O | 95/5 => 0/100 (1.0 min)=> 0/100(0.4 min) | 0.9 | 55 | 210-400nm PDA total |
| | | | B) 0.1% FA CH₃CN | | | | |
| SQD AA05long | Acquity UPLC/SQD2 | Aldrich Ascentis Express C18 (2.1 × 50) | A) 10mM AcONH₄, H₂O | 95/5 => 0/100 (4.5 min)=> 0/100(0.5 min) | 0.9 | 35 | 210-400nm PDA total |
| | | | B) MeOH | | | | |
| SMD method_1 | Shimadzu LCMS-2020 LC-20ADXR | CORTECS C18 (2.1 × 50) | A) 0.1% FA, H₂O | 95/5 => 0/100 (1.0 min)=> 0/100(0.5 min) | 1.0 | 40 | 190-400nm PDA total |
| | | | B) 0.1% FA CH₃CN | | | | |
| SMD method_2 | Shimadzu LCMS-2020 LC-20AD | Shim-Pack XR ODS-C18 (3.0 × 50) | A) 0.05% TFA, H₂O | 95/5 => 5/95 (1.1 min)=> 5/95(0.6 min) | 1.2 | 40 | 190-400nm PDA total |
| | | | B) 0.05% TFA CH₃CN | | | | |
| SMD method_3 | Shimadzu LCMS-2020 LC-20AD | Shim-Pack XR ODS-C18 (3.0 × 50) | A) 0.05% TFA, H₂O | 70/30 => 5/95 (2.0 min)=> 5/95(0.7 min) | 1.2 | 40 | 190-400nm PDA total |
| | | | B) 0.05% TFA CH₃CN | | | | |

**[Table 2]**

| Analysis conditions | Device | Sample injection | Mode | Polarity | Resolution | Capillary Temp. (°C) | Capillary voltage (V) | Tube Lens (V) |
|---|---|---|---|---|---|---|---|---|
| LTQ method_1 | Thermo LTQ Orbitrap XL | Direct infusion | Fullscan | Positive | 30,000 | 275 | 13 | 100 |

In peptide synthesis described herein, Fmoc-protected amino acids shown Tables 3 to 5 were used.

**[Table 3]**

| Abbreviation | Structure | | Abbreviation | Structure | | Abbreviation | Structure |
|---|---|---|---|---|---|---|---|
| Fmoc-Photo-Linker | 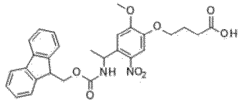 | | Fmoc-PEG6-OH | 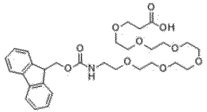 | | Fmoc-nPrGly-OH | 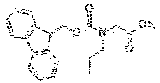 |
| Fmoc-MeGly-OH | 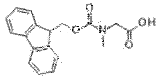 | | Fmoc-Gly-OH | 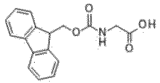 | | Fmoc-MaAla-OH | 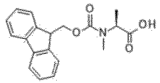 |
| Fmoc-Ala-OH | 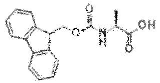 | | Fmoc-D-MeAla-OH | 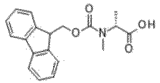 | | Fmoc-D-Ala-OH | 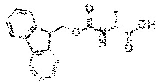 |
| Fmoc-MeVal-OH | 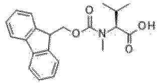 | | Fmoc-Val-OH | 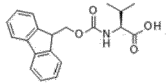 | | Fmoc-MeLeu-OH | 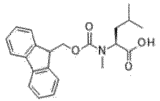 |
| Fmoc-Leu-OH | 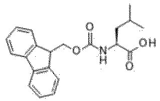 | | Fmoc-Ile-OH | 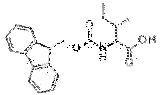 | | Fmoc-Nie-OH | 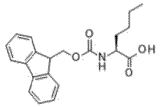 |
| Fmoc-MePhe-OH | 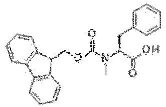 | | Fmoc-MePhe(3-Cl)-OH | 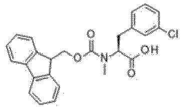 | | Fmoc-Phe(3-Cl)-OH | 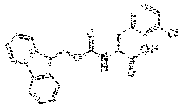 |
| Fmoc-Pic(2)-OH | 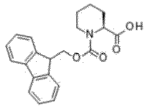 | | Fmoc-Pro-OH | 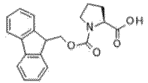 | | **Fmoc-**Aze(2)-OH | 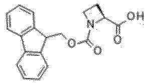 |
| Fmoc-Asp(OPis )-OH | 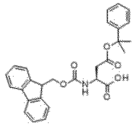 | | Fmoc-R-AMPA-OH | 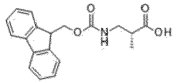 | | | |

**[Table 4]**

| Abbreviation | Structure | | Abbreviation | Structure | | Abbreviation | Structure |
|---|---|---|---|---|---|---|---|
| Fmoc-MeSer(TH P)-OH | 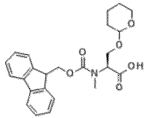 | | Fmoc-MeSer(nP r)-OH | 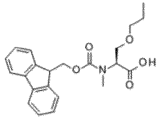 | | Fmoc-MeSer(iP en)-OH | 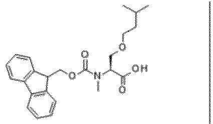 |
| Fmoc-Ser(iPen)-OH | 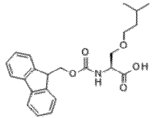 | | Fmoc-Ser(Ph-2-Cl)-OH | 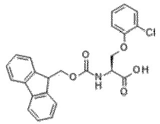 | | Fmoc-Ser(tBuO H)-OH | 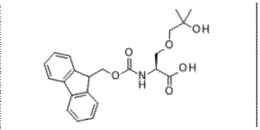 |
| Fmoc-Thr(THP)-OH | 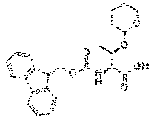 | | | | | | |

**[Table 5]**

| Abbreviation | Structure |
|---|---|
| Fmoc-MeAsp(O Pis)-OH | 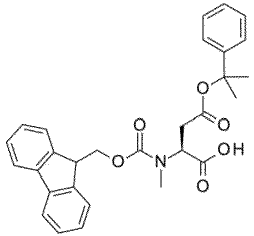 |

The Fmoc-protected amino acids shown in Table 3 were purchased from commercial suppliers.

The Fmoc-protected amino acids shown in Table 4 were synthesized in accordance with methods described in WO 2018/225864.

The Fmoc-protected amino acid shown in Table 5 was synthesized by the method described in Example 1-1.

Fluctuation may be found herein even with the same compounds in the ranges of about ± 0.2 in the value of LCMS (ESI) m/z and about ± 0.07 minutes in retention time.

### Example 1: Preparation of Fmoc-protected amino acids, urea, peptides supported on membrane, and the like used in this Example

For membrane discs, CelluSpots 384 frame with acid stable discs or Refill of two frames, 384 acid stable discs (amino group-modified membranes) purchased from Intavis Bioanalytical Instruments AG (now, CEM Corporation) were used.

### Example 1-1: Synthesis of compound SS01, (2S)-2-[9H-fluoren-9-ylmethoxycarbonyl(methyl)amino]-4-(1-methyl-1-phenyl-ethoxy)-4-oxo-butanoic acid (Fmoc-MeAsp(OPis)-OH)

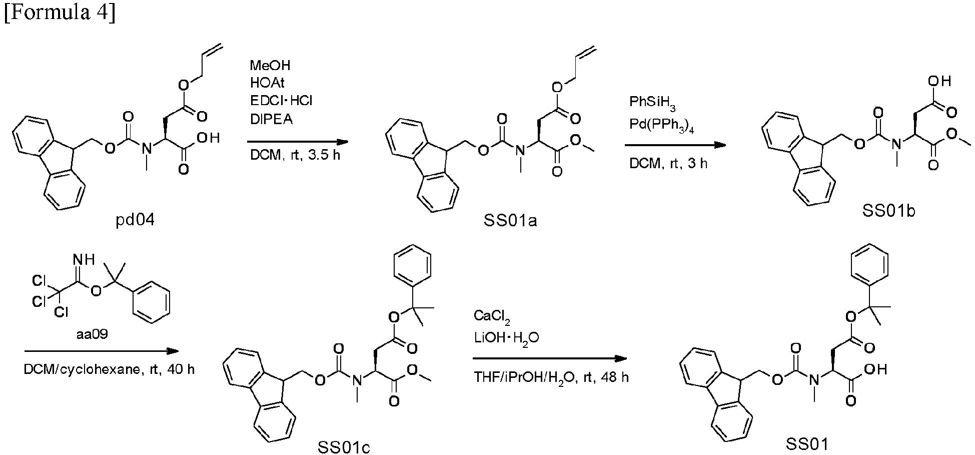

According to the scheme described above, (2S)-2-[9H-fluoren-9-ylmethoxycarbonyl(methyl)amino]-4-(1-methyl-1-phenyl-ethoxy)-4-oxo-butanoic acid (Fmoc-MeAsp(OPis)-OH, SS01) was synthesized.

### Example 1-1-1: Synthesis of O4-allyl O1-methyl (2S)-2-[9H-fluoren-9-ylmethoxycarbonyl(methyl)amino]butanedioate (compound SS01a)

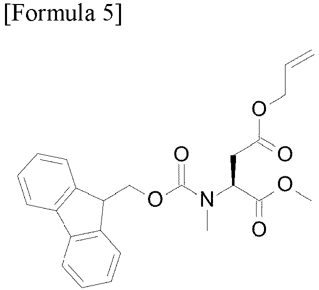

To (S)-2-((((9H-fluoren-9-yl-)methoxy)carbonyl)(methyl)amino)-4-(allyloxy)-4-oxobutanoic acid (compound pd04) (100.0 g, 244.2 mmol) synthesized by the method described in WO 2018/124162, DCM (1.15 L) was added under nitrogen atmosphere, and EDCI·HCl (60.87 g, 317.5 mmol) and HOAt (43.22 g, 317.5 mmol) were added at room temperature, and the mixture was stirred for 30 minutes. To this solution, methanol (8.61 g, 268.7 mmol) and DIPEA (41.04 g, 317.5 mmol) were added at room temperature, and the mixture was stirred for 3 hours. DCM was then added to dilute, and the organic layer was washed with aqueous ammonium chloride solution and saturated saline, and then dried over sodium sulfate. The desiccant was filtered off, and then the filtrate was concentrated under reduced pressure to obtain 93 g (yield 90%) of compound SS01a as a crude product.
LCMS (ESI) m/z = 424.1 [M + H]⁺
Retention time: 1.13 minutes (analysis condition SMD method_1)

### Example 1-1-2: Synthesis of (3S)-3-[9H-fluoren-9-ylmethoxycarbonyl(methyl)amino]-4-oxo-butanoic acid (compound SS01b)

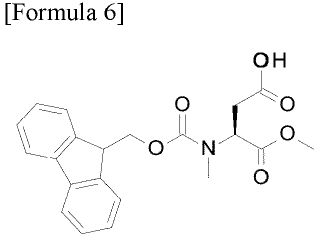

To the crude product of compound SS01a (100.0 g, purity 76%, 179.5 mmol), DCM (350 mL) was added under a nitrogen atmosphere, and tetrachristriphenylphosphine palladium (2.07 g, 1.795 mmol) and phenylsilane (13.60 g, 125.7 mmol) were added at room temperature, and the mixture was stirred for 3 hours. TBME was then added to dilute, and the resulting dilution was extracted with saturated aqueous sodium carbonate solution. The obtained aqueous layer was washed with TBME, then 0.1 M aqueous phosphoric acid solution was added to make it acidic, and the aqueous layer was extracted 2 times with ethyl acetate. The resulting organic layer was washed with saturated saline and then dried over sodium sulfate. The desiccant was filtered off, and then the filtrate was concentrated under reduced pressure to obtain 82 g (yield 110%) of compound SS01b.
LCMS (ESI) m/z = 384.4 [M + H]⁺
Retention time: 1.32 minutes (analysis condition SMD method_2)

### Example 1-1-3: Synthesis of O1-methyl O4-(1-methyl-1-phenyl-ethyl) (2S)-2-[9H-fluoren-9-ylmethoxycarbonyl(methyl)amino]butanedioate (compound SS01c)

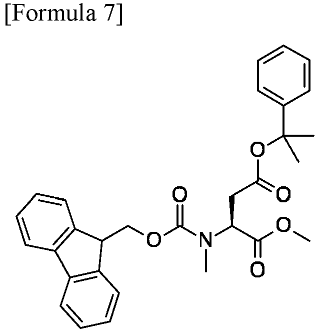

To compound SS01b (50.00 g, 130.4 mmol), DCM (100 mL) was added under a nitrogen atmosphere, and a solution of cyclohexane (400 mL) in 2-phenylpropan-2-yl 2,2,2-trichloroacetimidate (compound aa09) (148.0 g, 527.5 mmol) synthesized by the method described in WO 2018/124162 was added dropwise at room temperature, and the mixture was stirred for 40 hours. The mixture was filtered and the filtrate was concentrated under reduced pressure. The resulting residue was purified by normal phase silica gel column chromatography (ethyl acetate/petroleum ether) to obtain 68 g (yield 101%) of compound SS01c.
LCMS (ESI) m/z = 524.2 [M + Na]⁺
Retention time: 1.22 minutes (analysis condition SMD method_1)

### Example 1-1-4: Synthesis of (25)-2-[9H-fluoren-9-ylmethoxycarbonyl(methyl)amino]-4-(1-methyl-1-phenyl-ethoxy)-4-oxo-butanoic acid (compound SS01, Fmoc-MeAsp(OPis)-OH)

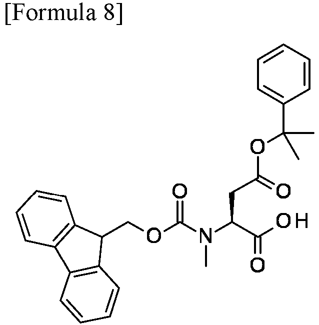

Calcium chloride (248.9 g, 2243 mmol) and lithium hydroxide monohydrate (25.10 g, 588.1 mmol) were dissolved in a mixed solvent of 2-propanol (3 L) and H₂O (750 mL), and the mixture was cooled to 0°C in an ice bath. To the solution, a solution of compound SS01c (75.00 g, 149.5 mmol) in THF (750 mL) was added dropwise, and the mixture was stirred at room temperature for 48 hours. The mixture was then filtered, and the filtrate was concentrated under reduced pressure to remove volatile organic compounds. The resulting solution was extracted with ethyl acetate. The organic layer was washed with aqueous 1M phosphoric acid solution and saturated saline, then dried over sodium sulfate. The desiccant was filtered off, and then the filtrate was concentrated under reduced pressure, and the resulting residue was dissolved in a mixed solvent of TBME and hexane (1 : 1). The solution was extracted with saturated aqueous sodium bicarbonate solution and the aqueous layer was extracted 2 times with ethyl acetate. The resulting organic layer was washed with 0.1 M aqueous phosphoric acid solution and saturated saline and then dried over sodium sulfate. The desiccant was filtered off, and then the filtrate was concentrated under reduced pressure to obtain 60 g (yield 82%) of compound SS01.
LCMS (ESI) m/z = 510.5 [M + Na]⁺
Retention time: 1.95 minutes (analysis condition SMD method_3)

### Example 1-2: Preparation of membrane-supported amino acid and peptide and the like used in this Example

A membrane or resin part may be written herein as ○ when the membrane or resin is bonded to the compound. For the purpose of clarifying the reaction point of the membrane part, the chemical structure of the reaction part may be written with the compound connected to ○. For example, in the following structure (Fmoc-MePhe-Photo-Linker-Membrane (compound SS02)), the amino group of the amino group-modified membrane forms an amide bond with the carboxylic acid of Photo-Linker.

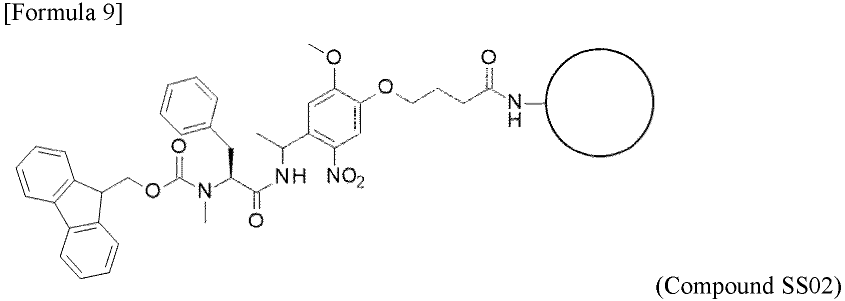

To confirm the product of the amino acid elongation reaction herein, the solution after the peptide isolation reaction from the membrane using the membrane obtained after elongation was measured by LCMS. Specific procedures are shown below. As the UV irradiation equipment, a UV-LED irradiation box MUB-031 outsourcing-manufactured by MLC Co., Ltd or a separate-type UV-LED irradiation device manufactured by Shodensha Vietnam Co., Ltd. was used. The membrane disc dried after elongation in a reaction vessel placed on ice or at room temperature was irradiated with light at a UV wavelength of 365 nm and an illuminance of 380 to 600 mW/cm² for 2 minutes and 30 seconds. Then, 100 µL of DMSO was added to the membrane, and the mixture was allowed to stand for 15 minutes or more to dissolve the peptide. The solution was analyzed by LCMS.

### Example 1-2-1: Synthesis of compound SS02 (Fmoc-MePhe-Photo-Linker-Membrane), compound SS03 (Fmoc-Pro-Photo-Linker-Membrane), compound SS24 (Fmoc-MeAla-Photo-Linker-Membrane), and compound SS25 (Fmoc-MeSer(THP)-Photo-Linker-Membrane)

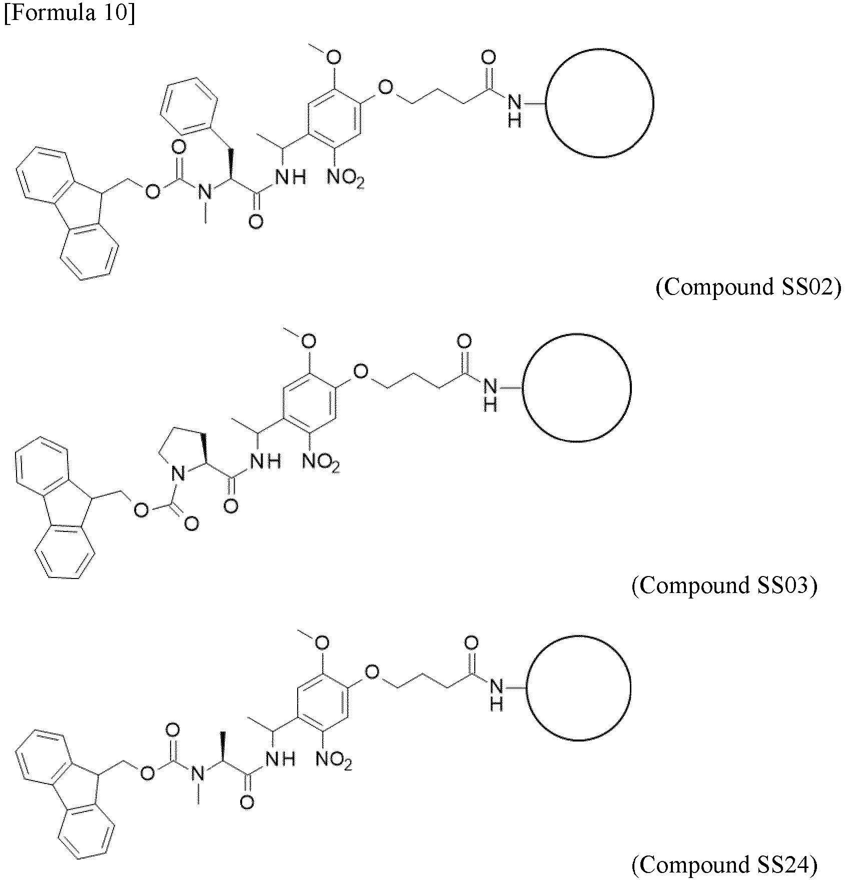

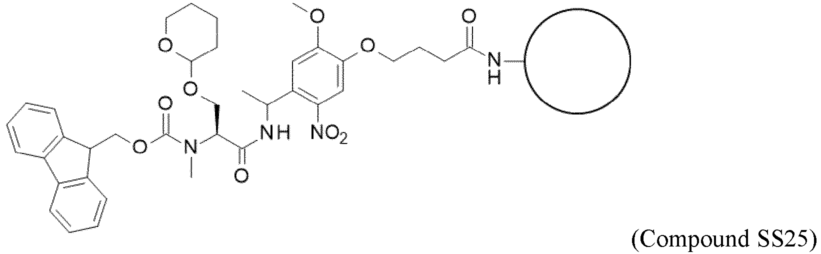

Preparation of compounds SS02, SS03, SS24, and SS25 used in this Example was performed by the Fmoc method using a peptide synthesizer (Multipep RS; manufactured by Intavis Bioanalytical Instruments AG). Detailed procedures of operations were performed in accordance with the manual attached to the synthesizer. The preparation was performed by the methods of operation 1 or Operation 2 below with reference to WO 2018/225851 for reaction conditions. In the preparation of compound SS02, Fmoc-Photo-Linker and Fmoc-MePhe-OH were used in this order; in the preparation of compound SS03, Fmoc-Photo-Linker and Fmoc-Pro-OH were used in this order; in the preparation of compound SS24, Fmoc-Photo-Linker and Fmoc-MeAla-OH were used in this order; in the preparation of compound SS25, Fmoc-Photo-Linker and Fmoc-MeSer(THP)-OH were used in this order as Fmoc-protected amino acids, respectively.

### [Operation 1]

An Fmoc-protected amino acid (0.6 mol/L) constituting a peptide of interest, and HOAt, Oxyma, or HOOBt (0.375 mol/L) as a carboxylic acid activating agent were dissolved in NMP to prepare solution 1. N,N'-diisopropylcarbodiimide (DIC) (0.71 mol/L) was mixed with N,N-dimethylformamide (DMF) to prepare solution 2.

CelluSpots 384 frame with acid stable discs (amino group-modified membranes manufactured by Intavis Bioanalytical Instruments AG; hereinafter also referred to as "membrane discs") were set into a peptide synthesizer. Solutions 1 and 2 were set in the peptide synthesizer, and automated synthesis with the peptide synthesizer was started.

### De-Fmoc step

A solution of DBU in DMF (2%v/v) was added in amounts of 2.0 µL and 4.0 µL per membrane disc to deprotect the Fmoc group at room temperature. Deprotection was not required before the first residue elongation. In the deprotection after the first residue elongation, 2.0 µL of the solution was added and allowed to react for 5 minutes, then the solution was discharged once, and then 4.0 µL of the solution was added and allowed to react for another 10 minutes, and then the solution was discharged. Subsequently, the membrane disc was washed 7 times with DMF (25 µL. per membrane disc), 2 times with ethanol (25 µL per membrane disc), 2 times with ethanol (37.5 µL per membrane disc), and 2 times with ethanol (25 µL per membrane disc), and then dried under reduced pressure for 15 minutes.

### Elongation step

Solution 1 and solution 2 were mixed at a ratio of 5 : 6 in a mixing vial of the synthesizer and allowed to stand for 15 minutes. The mixed solution was then added in an amount of 1.2 µL per membrane disc and allowed to react at room temperature for 40 minutes to perform a condensation reaction between the amino group on the membrane disc and the Fmoc-protected amino acid, then the solution was discharged. This condensation reaction was repeated one more time. Subsequently, a solution of acetic anhydride (Ac₂O) in DMF (4%v/v) was added in an amount of 4.0 µL per membrane disc to perform acetyl capping of unreacted amines at room temperature. After the reaction for 5 minutes, the solution was discharged. The membrane disc was then washed 7 times with DMF (25 µL. per membrane disc) and dried under reduced pressure for 10 minutes.

This condensation reaction of the Fmoc-protected amino acid subsequent to the deprotection reaction of the Fmoc group and the acetyl capping were set to one cycle, and this cycle was repeated to elongate a peptide on the surface of the membrane disc. After the last amino acid elongation, a de-Fmoc step was not performed, and the membrane disc was washed 7 times with DMF (25 µL per membrane disc), 2 times with ethanol (25 µL per membrane disc), 2 times with ethanol (37.5 µL per membrane disc), and 2 or 3 times with ethanol (25 µL per membrane disc), and then dried under reduced pressure for 10 to 15 minutes. The resulting membrane disc was then used for subsequent studies.

### [Operation 2]

An Fmoc-protected amino acid (0.29 mol/L) constituting a peptide of interest, and HOAt, Oxyma, or HOOBt (0.181 mol/L) as a carboxylic acid activating agent were dissolved in NMP/DMF = 6.68/4.34 or 5/6 to prepare solution 1. N,N'-diisopropylcarbodiimide (DIC) was used without dilution as solution 2.

CelluSpots 384 frame with acid stable discs (manufactured by Intavis Bioanalytical Instruments AG) were set into a peptide synthesizer. Solutions 1 and 2 were set in the peptide synthesizer, and automated synthesis with the peptide synthesizer was started.

### De-Fmoc step

This step was performed in the same manner as the de-Fmoc step of [Operation 1].

### Elongation step

Solution 1 and solution 2 were mixed at a ratio of 11.29 : 0.72 in a mixing vial of the synthesizer and allowed to stand for 15 minutes. The mixed solution was then added in an amount of 1.2 µL per membrane disc and allowed to react at room temperature for 40 minutes to perform a condensation reaction between the amino group on the membrane disc and the Fmoc-protected amino acid, then the solution was discharged. This condensation reaction was repeated one more time. Thereafter, this step was performed in the same manner as the elongation step of [Operation 1].

### Example 1-2-2: Isolation and analysis of Fmoc-MePhe or Fmoc-Pro supported on membrane

The peptides were isolated by the method described in Examples 1-2 using compounds SS02, SS03, SS24, and SS25 prepared by the method of Operation 1 or Operation 2, and the production of the peptides of interest (compounds SS02*, SS03*, SS24*, and SS25*) was confirmed. In this Example, the compound of compound No. with * indicates a compound confirmed by isolating a peptide from the membrane disc for checking the reaction. 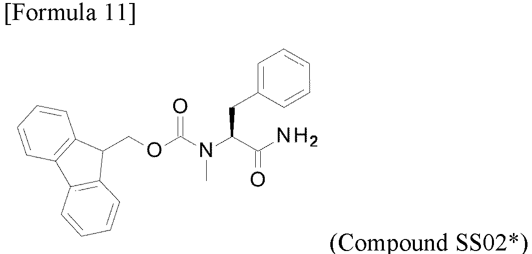
LCMS (ESI) m/z = 401.3 [M + H]⁺
Retention time: 3.33 minutes (analysis condition SQDAA05long) 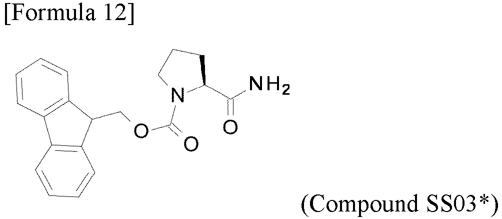
LCMS (ESI) m/z = 337.3 [M + H]⁺
Retention time: 2.74 minutes (analysis condition SQDAA05long) 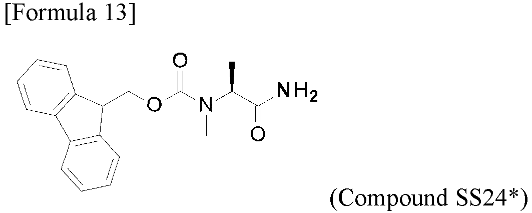
LCMS (ESI) m/z = 325.3 [M + H]⁺
Retention time: 2.76 minutes (analysis condition SQDAA05long) 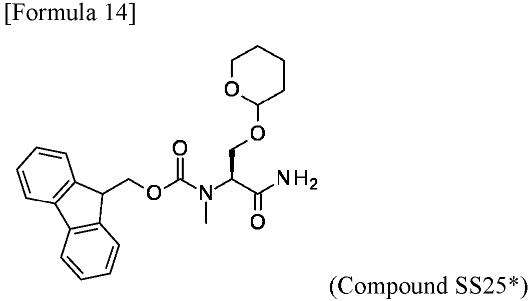
LCMS (ESI) m/z = 425.4 [M + H]⁺
Retention time: 3.17 minutes (analysis condition SQDAA05long)

### Example 1-2-3: Confirmation of amount of Fmoc-amino acids supported on membrane

The amount of the Fmoc amino acid supported on the membrane was confirmed by the following method.

Compound SS02, SS03, SS24, or SS25 (one membrane disc each) prepared by the method of Operation 1 or Operation 2 was placed in a reaction vessel. A solution of DBU in DMF (2%v/v) was added in an amount of 4.0 µL per membrane disc and allowed to react at room temperature for 5 minutes, and then added again in an amount of 4.0 µL and allowed to react at room temperature for 10 minutes to deprotect the Fmoc group. DMF was then added in an amount of 392 µL per membrane disc to elute and the resulting solution was analyzed by LC/MS (injection volume: 5 µL). 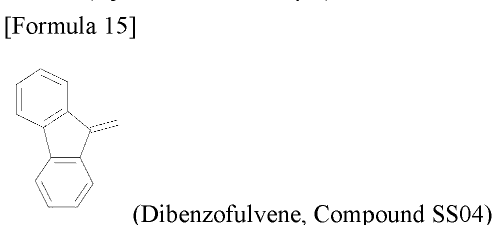
Retention time: 1.02 minutes (analysis condition SQDFA05_1)
UV area value at wavelength 304 nm for compound SS04 from compound SS02: 16359.88
UV area value at wavelength 304 nm for compound SS04 from compound SS03: 15793.11
UV area value at wavelength 304 nm for compound SS04 from compound SS24: 18782.30
UV area value at wavelength 304 nm for compound SS04 from compound SS25: 18300.73

Fmoc-Gly-OH (3.51 mg, 0.012 mmol) was dissolved in a solution of DBU in DMF (1.5 mL, 0.201 mmol) and allowed to react at room temperature for 15 minutes to deprotect the Fmoc group. DMF (148.5 mL) was then added to the reaction mixture to dilute. The resulting dilution solution was analyzed by LC/MS (analysis condition SQDFA05_1, injection volume: 5 µL). The UV area value for dibenzofulvene at a wavelength of 304 nm was 8903.11, and this value was used to calculate the UV area value per nmol (wavelength 304 nm). Using these values, the amounts of compounds SS02 and SS03 supported were calculated from the following equation. Amount supported (nmol) = (UV area value of compound SS04 (wavelength 304 nm))/(UV area value per nmol of dibenzofulvene (wavelength 304 nm))

As a result, the amounts of compounds SS02, SS03, SS24, and SS25 supported were calculated as 57.9 nmol/membrane disc, 55.9 nmol/membrane disc, 66.4 nmol/membrane disc, and 64.7 nmol/membrane disc, respectively.

Another lot with a different amount supported, which had been similarly synthesized, was also used for peptide synthesis, studies and the like.

### Example 1-3: Synthesis of urea derived from carbodiimide used in this Example

### Example 1-3-1: Synthesis of 1,3-diisopropylurea (DIC urea, compound SS05)

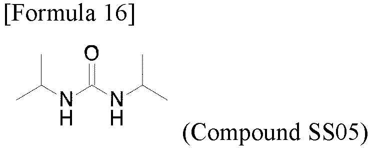

To DIC (1.22 mL, 7.92 mmol), TBME (31.5 mL) was added at room temperature, then acetic acid (1.178 mL, 20.60 mmol) was added dropwise for 5 minutes or more, and the reaction mixture was stirred at room temperature for 1 hour and 30 minutes. The resulting precipitate was then recovered by filtration, and the solids obtained using TBME were washed and dried under reduced pressure to obtain 1,3-diisopropylurea (DIC urea, compound SS05) (909.1 mg, 80%).
¹H-NMR (BRUKER Ascend 400, 400 MHz, DMSO-d₆) δ 5.48 (2H, d, J = 7.2 Hz), 3.69-3.57 (2H, m), 1.00 (12H, d, J = 6.8 Hz)

### Example 1-3-2: Synthesis of 1,3-di-sec-butylurea (DsBC urea, compound SS06)

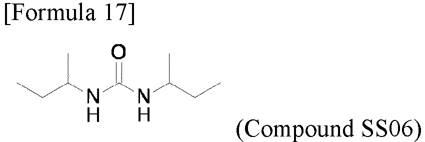

To DsBC (0.356 µL, 1.945 mmol), TBME (7.73 mL) was added at room temperature, then acetic acid (0.289 µL, 5.06 mmol) was added dropwise for 5 minutes or more, and the reaction mixture was stirred at room temperature for 15 minutes. Water (70.1 µL, 3.89 mmol) was then added, and the mixture was stirred at room temperature for 2 hours and 30 minutes. Nitrogen was then sprayed to concentrate, and water was added. The resulting precipitate was then recovered by filtration, and the obtained solids were washed with water and dried under reduced pressure to obtain 1,3-di-sec-butylurea (DsBC urea, compound SS06) (210.7 mg, 63%). ¹H-NMR (BRUKER Ascend 400, 400 MHz, DMSO-d₆) δ 5.46 (2H, d, J = 8.0 Hz), 3.50-3.43 (2H, m), 1.32 (4H, dq, J = 7.2, 7.2 Hz), 0.97 (6H, d, J = 6.8 Hz), 0.81 (6H, t, J = 7.2 Hz)

### Example 1-3-3: Synthesis of 1-(tert-butyl)-3-ethylurea (tBEC urea, compound SS07)

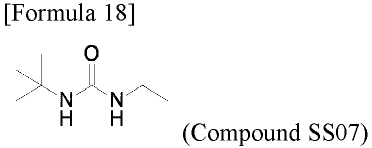

To tBEC (0.614 µL, 3.96 mmol), TBME (15.7 mL) was added at room temperature, then acetic acid (0.589 µL, 10.30 mmol) was added dropwise for 5 minutes or more, and the reaction mixture was stirred at room temperature for 2 hours and 30 minutes. Nitrogen was then sprayed to concentrate, and a 1 : 2 mixture of MeCN and water was added. The resulting precipitate was recovered by filtration, and the obtained solid was washed with a 1 : 2 mixture of MeCN and water. The filtrate was concentrated under reduced pressure, and water was added. The resulting precipitate was recovered by filtration, and the obtained solid was washed with water. The two resulting solids were dissolved in MeCN and then mixed and concentrated under reduced pressure to obtain 1-(tert-butyl)-3-ethylurea (tBEC urea, compound SS07) (169.2 mg, 30%).
¹H-NMR (BRUKER Ascend 400, 400 MHz, DMSO-d₆) δ 5.53 (2H, br), 2.98-2.91 (2H, m), 1.20 (9H, s), 0.95 (3H, t, J = 7.2 Hz)

### Example 1-4: Synthesis of carbodiimide used in this Example

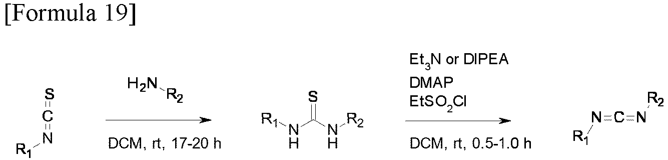

According to the scheme described above, N'-(1-phenylethyl)-N-sec-butyl-methanediimine (SS26), N'-(1-methylheptyl)-N-sec-butyl-methanediimine (SS27), N,N'-bis(1-methylbutyl)methanediimine (SS28), N,N'-bis(1-ethylpropyl)methanediimine (SS29), and N'-(1-ethylpropyl)-N-(1-methylbutyl)methanediimine (SS30) were synthesized.

### Example 1-4-1: Synthesis of 1-(1-phenylethyl)-3-sec-butyl-thiourea (compound SS26a)

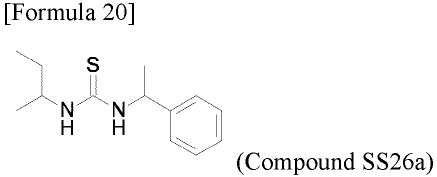

2-Isothiocyanatobutane (500 µL, 4.08 mmol) and 1-phenylethanamine (567 µL, 4.49 mmol) were added to DCM (20.4 mL) at room temperature, and the mixture was stirred at room temperature for 17 hours. The mixture was concentrated under reduced pressure, and the resulting residue was purified 2 times by normal phase silica gel column chromatography (ethyl acetate/n-hexane) to obtain 874.6 mg (yield 91%) of compound SS26a as a diastereomer mixture.
LCMS (ESI) m/z = 237.1 [M + H]⁺
Retention time: 0.77 minutes (analysis condition SQDFA05_1)

### Example 1-4-2: Synthesis of N'-(1-phenylethyl)-N-sec-butyl-methanediimine (SS26)

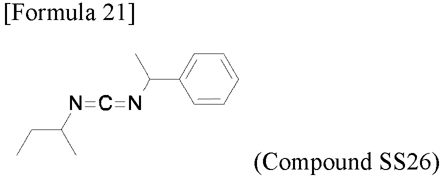

Compound SS26a (149.6 mg, 0.633 mmol) was added to DCM (6.329 mL), and triethylamine (265 µL, 1.899 mmol), 4-dimethylaminopyridine (77 mg, 0.633 mmol) and ethanesulfonyl chloride (120 µL, 1.266 mmol) were added under ice cooling. The mixture was stirred for 5 minutes under ice cooling, then heated to room temperature and stirred at room temperature for 1 hour. To the mixture, ISOLUTE (registered trademark) HM-N (manufactured by Biotage Ltd.) was added and the resulting mixture was concentrated under reduced pressure. The resulting residue was purified a plurality of times by normal phase silica gel column chromatography (ethyl acetate/n-hexane and DCM) to obtain 35.21 mg (yield 28%) of compound SS26 as a diastereomer mixture.
MS (ESI) m/z = 203.2 [M + H]⁺ (analysis condition LTQ method_1)
¹H-NMR (BRUKER Ascend 400, 400 MHz, DMSO-d₆) δ 7.38-7.25 (5H, m), 4.62 (1H, q, J = 6.4 Hz), 3.31-3.22 (1H, m), 1.45 (3H, d, J = 6.4 Hz), 1.42-1.25 (2H, m), 1.06 (3H, d and d, J = 6.4 Hz), 0.81 (3H, t and t, J = 7.6 Hz)

### Example 1-4-3: Synthesis of 1-(1-methylheptyl)-3-sec-butyl-thiourea (compound SS27a)

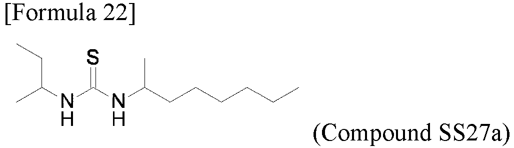

2-Isothiocyanatobutane (300 µL, 2.45 mmol) and 2-octanamine (452 µL, 2.69 mmol) were added to DCM (12.2 mL) at room temperature, and the mixture was stirred at room temperature for 17 hours. The mixture was concentrated under reduced pressure, and the resulting residue was purified 2 times by normal phase silica gel column chromatography (ethyl acetate/n-hexane) to obtain 552.0 mg (yield 92%) of compound SS27a as a diastereomer mixture.
LCMS (ESI) m/z = 245.2 [M + H]⁺
Retention time: 0.94 minutes (analysis condition SQDFA05_1)

### Example 1-4-4: Synthesis of N'-(1-methylheptyl)-N-sec-butyl-methanediimine (SS27)

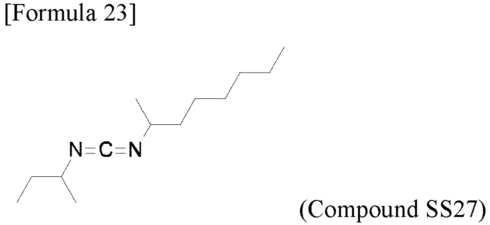

Compound SS27a (150 mg, 0.614 mmol) was added to DCM (6.136 mL), and DIPEA (315 µL, 1.841 mmol), 4-dimethylaminopyridine (75 mg, 0.614 mmol) and ethanesulfonyl chloride (116 µL, 1.227 mmol) were added under ice cooling. The mixture was stirred for 5 minutes under ice cooling, then heated to room temperature and stirred at room temperature for 1 hour. To the mixture, ISOLUTE (registered trademark) HM-N (manufactured by Biotage Ltd.) was added and the resulting mixture was concentrated under reduced pressure. The resulting residue was purified several times by normal phase silica gel column chromatography (ethyl acetate/n-hexane and DCM) to obtain 22.83 mg (yield 18%) of compound SS27 as a diastereomer mixture.
MS (ESI) m/z = 211.2 [M + H]⁺ (analysis condition LTQ method_1)
¹H-NMR (BRUKER Ascend 400, 400 MHz, DMSO-d₆) δ 3.38-3.24 (2H, m), 1.48-1.25 (12H, m), 1.14 (6H, d, J = 6.4 Hz), 0.88 (3H, t, J = 7.6 Hz), 0.86 (3H, t, J = 7.6 Hz)

### Example 1-4-5: Synthesis of 1,3-bis(1-methylbutyl)thiourea (compound SS28a)

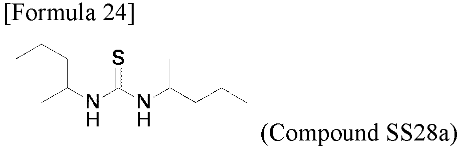

2-Isothiocyanatopentane (300 µL, 2.16 mmol) and 2-pentanamine (280 µL, 2.38 mmol) were added to DCM (10.8 mL) at room temperature, and the mixture was stirred at room temperature for 20 hours. The mixture was concentrated under reduced pressure, and the resulting residue was purified by normal phase silica gel column chromatography (ethyl acetate/n-hexane) to obtain 426.8 mg (yield 91%) of compound SS28a as a diastereomer mixture.
LCMS (ESI) m/z = 217.2 [M + H]⁺
Retention time: 0.82 minutes (analysis condition SQDFA05_1)

### Example 1-4-6: Synthesis of N,N'-bis(1-methylbutyl)methanediimine (SS28)

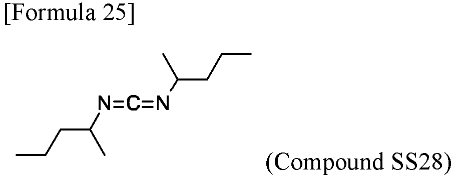

Compound SS28a (199.8 mg, 0.923 mmol) was added to DCM (9.233 mL), and triethylamine (386 µL, 2.77 mmol), 4-dimethylaminopyridine (113 mg, 0.923 mmol) and ethanesulfonyl chloride (175 µL, 1.847 mmol) were added under ice cooling. The mixture was stirred for 5 minutes under ice cooling, then heated to room temperature and stirred at room temperature for 1 hour. To the mixture, ISOLUTE (registered trademark) HM-N (manufactured by Biotage Ltd.) was added and the resulting mixture was concentrated under reduced pressure. The resulting residue was purified several times by normal phase silica gel column chromatography (ethyl acetate/n-hexane and DCM) to obtain 36.30 mg (yield 22%) of compound SS28 as a diastereomer mixture.
MS (ESI) m/z = 183.2 [M + H]⁺ (analysis condition LTQ method_1)
¹H-NMR (BRUKER Ascend 400, 400 MHz, DMSO-d₆) δ 3.39-3.33 (2H, m), 1.42-1.23 (8H, m), 1.14 (6H, d, J = 6.4 Hz), 0.91-0.83 (6H, m)

### Example 1-4-7: Synthesis of 1,3-bis(1-ethylpropyl)thiourea (compound SS29a)

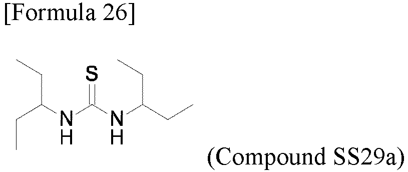

3-Isothiocyanatopentane (300 µL, 2.18 mmol) and 3-pentanamine (279 µL, 2.40 mmol) were added to DCM (10.9 mL) at room temperature, and the mixture was stirred at room temperature for 20 hours. The mixture was concentrated under reduced pressure, and the resulting residue was purified by normal phase silica gel column chromatography (ethyl acetate/n-hexane) to obtain 401.4 mg (yield 85%) of compound SS29a.
LCMS (ESI) m/z = 217.2 [M + H]⁺
Retention time: 0.79 minutes (analysis condition SQDFA05_1)

### Example 1-4-8: Synthesis of N,N'-bis(1-ethylpropyl)methanediimine (SS29)

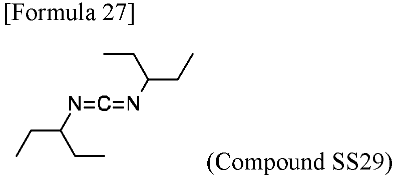

Compound SS29a (200.8 mg, 0.928 mmol) was added to DCM (9.280 mL), and triethylamine (388 µL, 2.78 mmol), 4-dimethylaminopyridine (113 mg, 0.928 mmol) and ethanesulfonyl chloride (175 µL, 1.856 mmol) were added under ice cooling. The mixture was stirred for 5 minutes under ice cooling, then heated to room temperature and stirred at room temperature for 0.5 hours. To the mixture, ISOLUTE (registered trademark) HM-N (manufactured by Biotage Ltd.) was added and the resulting mixture was concentrated under reduced pressure. The resulting residue was purified several times by normal phase silica gel column chromatography (ethyl acetate/n-hexane and DCM) to obtain 37.59 mg (yield 22%) of compound SS29.
MS (ESI) m/z = 183.2 [M + H]⁺ (analysis condition LTQ method_1)
¹H-NMR (BRUKER Ascend 400, 400 MHz, DMSO-d₆) δ 3.08 (2H, tt, J = 4.8 Hz, 8.0 Hz), 1.56-1.30 (8H, m), 0.89 (12H, t, J = 7.2 Hz)

### Example 1-4-9: Synthesis of 1-(1-ethylpropyl)-3-(1-methylbutyl)thiourea (compound SS30a)

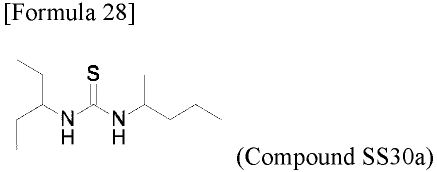

2-Isothiocyanatopentane (300 µL, 2.16 mmol) and 3-pentanamine (276 µL, 2.37 mmol) were added to DCM (10.8 mL) at room temperature, and the mixture was stirred at room temperature for 20 hours. The mixture was concentrated under reduced pressure, and the resulting residue was purified by normal phase silica gel column chromatography (ethyl acetate/n-hexane) to obtain 418.3 mg (yield 90%) of compound SS30a as a diastereomer mixture.
LCMS (ESI) m/z = 217.2 [M + H]⁺
Retention time: 0.80 minutes (analysis condition SQDFA05_1)

### Example 1-4-10: Synthesis of N'-(1-ethylpropyl)-N-(1-methylbutyl)methanediimine (SS30)

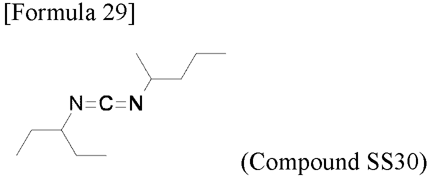

Compound SS30a (201.1 mg, 0.929 mmol) was added to DCM (9.294 mL), and triethylamine (389 µL, 2.79 mmol), 4-dimethylaminopyridine (114 mg, 0.929 mmol) and ethanesulfonyl chloride (176 µL, 1.859 mmol) were added under ice cooling. The mixture was stirred for 5 minutes under ice cooling, then heated to room temperature and stirred at room temperature for 0.5 hours. To the mixture, ISOLUTE (registered trademark) HM-N (manufactured by Biotage Ltd.) was added and the resulting mixture was concentrated under reduced pressure. The resulting residue was purified several times by normal phase silica gel column chromatography (ethyl acetate/n-hexane and DCM) to obtain 46.88 mg (yield 28%) of compound SS30 as a diastereomer mixture.
MS (ESI) m/z = 183.2 [M + H]⁺ (analysis condition LTQ method_1)
¹H-NMR (BRUKER Ascend 400, 400 MHz, DMSO-d₆) δ 3.40-3.33 (1H, m), 3.11-3.05 (1H, m), 1.55-1.27 (8H, m), 1.14 (3H, d and d, J = 6.4 Hz), 0.91-0.85 (9H, m)

### Example 2: Experiment examining types of reagent and reaction conditions for elongation step in peptide synthesis on membrane

In solid-phase synthesis of a peptide, amino acid elongation is performed subsequently to de-Fmoc and membrane washing. In this experiment, the preferred reagents and range of reaction conditions in peptide synthesis on the membrane were specified by using the peptide sequences supported on the membrane (compound SS02), varying the reaction conditions such as reagents, equivalent ratios, and the like of the amino acid elongation step, and comparing the degree of reaction efficiency of the elongation reaction of interest.

To evaluate the elongation efficiency of amino acids, glycine capping was performed to confirm the residual rate of the starting material. That is, after the elongation reaction of the amino acid of interest was carried out, the elongation reaction of Fmoc-Gly-OH was subsequently carried out and the reaction with unreacted amine was performed. Then, peptides were isolated from the membrane by the method described in Examples 1-2, and the UV area values between the peptide in which the amino acid of interest was elongated and the peptide in which Fmoc-Gly-OH was elongated were compared to calculate the elongation efficiency of the amino acid of interest.

### Reference Example 2-1: Peptide synthesis experiments on membrane under the reaction conditions described in Methods Mol. Biol., 2009, 570, 157-174

### Reference Example 2-1-1: Removal of Fmoc group on MePhe supported on membrane

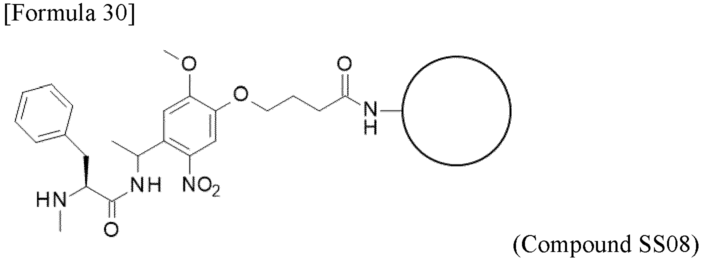

One peptide-supported membrane disc (compound SS02) prepared in Example 1-2-1 was placed in a reaction vessel. A solution of DBU in DMF (2%v/v) was added in an amount of 4.0 µL and allowed to react at room temperature for 5 minutes, and then added again in an amount of 4.0 µL and allowed to react at room temperature for 10 minutes to deprotect the Fmoc group. After the solution was discharged, the membrane was washed 4 times with DMF (100 µL per membrane disc) and 3 times with ethanol (100 µL per membrane disc) and dried in air to obtain compound SS08.

### Reference Example 2-1-2: Elongation reaction of Fmoc-Nle-OH to MePhe supported on membrane

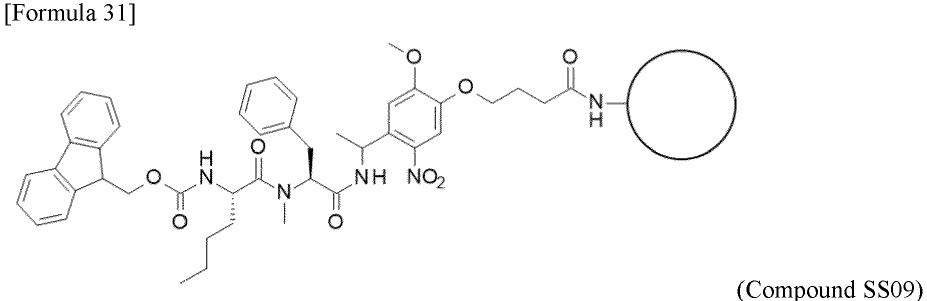

The elongation reaction of Fmoc-Nle-OH to MePhe on a membrane was performed with reference to the report of natural peptide synthesis on membrane using a peptide synthesizer (Multipep RS; manufactured by Intavis Bioanalytical Instruments AG) (Methods Mol. Biol., 2009, 570, 157-174).

A solution of Fmoc-Nle-OH (0.45 mol/L) and HOBt (0.675 mol/L) dissolved in NMP and a solution of DIC (1.485 mol/L) dissolved in NMP were mixed at a ratio of 3 : 1 and allowed to stand for 15 minutes. Then, 1.2 µL of this mixed solution was added to the membrane (compound SS08) obtained in Reference Example 2-1-1, which was placed in a reaction vessel, and allowed to stand at room temperature for a predetermined time. Subsequently, the membrane was washed 3 times with DMF (100 µL per membrane disc) and 3 times with ethanol (100 µL per membrane disc) and dried in air. Subsequently, a solution of Fmoc-Gly-OH (0.6 mol/L) and HOAt (0.375 mol/L) dissolved in NMP and a solution of DIC (0.71 mol/L) dissolved in DMF were mixed at a ratio of 5 : 6 and allowed to stand for 10 to 15 minutes. Then, 3.0 µL of this mixed solution was added to the membrane in the reaction vessel. After sealing, the vessel was allowed to stand at room temperature for 25 to 60 minutes. Then, the solution was discharged from the membrane, and the Fmoc-Gly-OH elongation reaction described above was performed again. After the solution was discharged, the membrane was washed 3 to 4 times with DMF (100 µL per membrane disc) and 3 times with ethanol (100 µL per membrane disc) and dried in air.

### Reference Example 2-1-3: Isolation and analysis of Fmoc-Nle-MePhe supported on membrane

The peptides were isolated from the membrane obtained in Reference Example 2-1-2 by the method described in Example 1-2, and the production of the peptide of interest (compound SS09*) and the peptide (compound SS10*) in which Fmoc-Gly-OH was elongated in place of Fmoc-Nle-OH was confirmed. 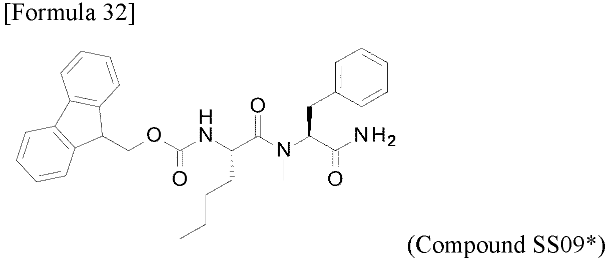 LCMS (ESI) m/z = 514.5 [M + H]⁺
Retention time: 3.64 minutes (analysis condition SQDAA05long) 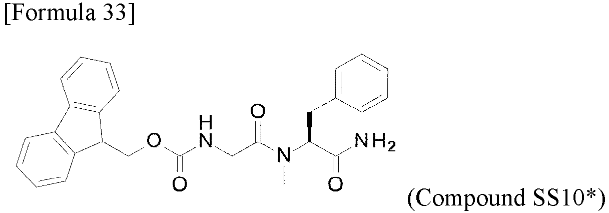 LCMS (ESI) m/z = 458.3 [M + H]⁺
Retention time: 3.20 minutes (analysis condition SQDAA05long)

The elongation efficiency was calculated from the following equation using UV area values (wavelength 299 nm) of peaks of each compound in the LC data. Elongation efficiency (%) = (UV area value of SS09*)/(sum of UV area value of SS09* and UV area value of SS10*) × 100

The conditions and results of Reference Example 2-1 are shown in Table 6 below.

**[Table 6]**

| Reference Example No. | No. | Fmoc-protected amino acid (concentration in reaction solution) | Activating agent (concentration in reaction solution) | Carbodiimide (concentration in reaction solution) | Reaction solvent | Reaction time | Peptide of interest | Elongation efficiency |
|---|---|---|---|---|---|---|---|---|
| 2-1 | 1 | Fmoc-Nle-OH (0.34mol/L) | HOBt (0.51 mol/L) | DIC (0.37mol/L) | NMP | 30 min | SS09* | 3% |
| | 2 | | | | | 60 min | | 4% |

The elongation efficiency under the reaction conditions with reference to Methods Mol. Biol., 2009, 570, 157-174 resulted in extremely low of 4% even after 60 minutes.

### Reference Example 2-2: Peptide synthesis experiment on membrane under the reaction conditions described in WO 2018/225851

### Reference Example 2-2-1: Elongation reaction of Fmoc-Nle-OH to MePhe supported on membrane

An elongation reaction of Fmoc-Nle-OH to MePhe on a membrane was performed by setting the reaction conditions with reference to the synthesis method of peptides containing N-substituted amino acids (WO 2018/225851) and using the membrane obtained in Reference Example 2-1-1 (compound SS08).

A solution of Fmoc-Nle-OH (0.6 mol/L) and HOAt (0.375 mol/L) dissolved in NMP or DMF and a solution of DIC (0.71 mol/L) dissolved in DMF were mixed at a ratio of 5 : 6 and allowed to stand for 15 minutes. Then, 1.2 µL of this mixed solution was added to the membrane (compound SS08) obtained in Reference Example 2-1-1, which was placed in a reaction vessel, and allowed to stand at room temperature for a predetermined time. Thereafter, the reaction was performed in the same manner as in Reference Example 2-1.

### Reference Example 2-2-2: Isolation and analysis of Fmoc-Nle-MePhe supported on membrane

The peptides were isolated from the membrane obtained in Reference Example 2-2-1 by the method described in Example 1-2, and the production of the peptide of interest (compound SS09*) and the peptide (compound SS10*) in which Fmoc-Gly-OH was elongated in place of Fmoc-Nle-OH was confirmed. The elongation efficiency was calculated according to the equation described in Reference Example 2-1.

The conditions and results of Reference Example 2-2 are shown in Table 7 below.

**[Table 7]**

| Reference Example No. | No. | Fmoc-protected amino acid (concentration in reaction solution) | Activating agent (concentration in reaction solution) | Carbodiimide (concentration in reaction solution) | Reaction solvent | Reaction time | Peptide of interest | Extension efficiency |
|---|---|---|---|---|---|---|---|---|
| 2-2 | 1 | Fmoc-Nle-OH (0.27mol/L) | HOAt (0.17mol/L) | DIC (0.38mol/L) | NMP/DMF=5/6 | 30 min | SS09* | 50% |
| | 2 | | | | | 60 min | | 52% |
| | 3 | | | | DMF | 30 min | | 62% |
| | 4 | | | | | 60 min | | 64% |

The elongation efficiency under the reaction conditions described in WO 2018/225851 resulted in low efficiency of 52% (when using a mixed solvent of NMP/DMF = 5/6) and 64% (when using a DMF solvent) even after 60 minutes. The change in elongation efficiency between the reaction times of 30 minutes and 60 minutes was minor.

### Reference Example 2-3: Peptide synthesis experiment on membrane under high reagent concentration conditions

### Reference Example 2-3-1: Elongation reaction of Fmoc-Nle-OH to MePhe supported on membrane

An elongation reaction of Fmoc-Nle-OH to MePhe on a membrane was performed while increasing the reagent concentration to increase the elongation efficiency and using the membrane obtained in Reference Example 2-1-1 (compound SS08).

A solution of Fmoc-Nle-OH (0.62 mol/L) and HOAt (0.193 mol/L or 0.387 mol/L) dissolved in NMP/DMF = 5/6 or 6.68/4.34 or dissolved in DMF was mixed with DIC (neat) at a ratio of 10 : 1.353 and allowed to stand for 15 minutes. Then, 1.2 µL of this mixed solution was added to the membrane (compound SS08) obtained in Reference Example 2-1-1 and allowed to stand at room temperature for 60 minutes. At this time, a portion of the solution that was allowed to stand for 15 minutes was diluted with MeCN, and the resulting solution was analyzed by LCMS (analysis condition SQDFA05_1). In addition, 1 mL of MeCN was added to the membrane after the reaction time had elapsed, the reaction solution impregnated in the membrane was dissolved, and the resulting MeCN solution was analyzed using LCMS (analysis condition SQDFA05_1). 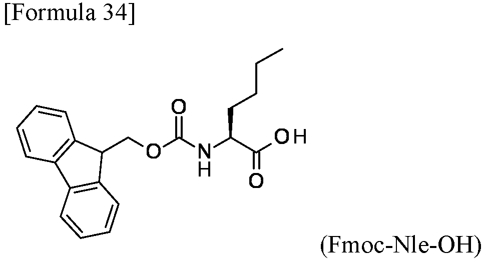 LCMS (ESI) m/z = 354.3 [M + H]⁺
Retention time: 0.89 minutes (analysis condition SQDFA05_1) 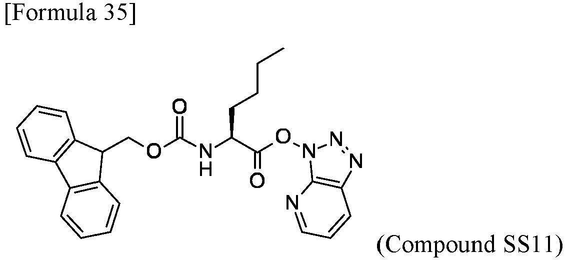 LCMS (ESI) m/z = 472.3 [M + H]⁺
Retention time: 1.02 minutes (analysis condition SQDFA05_1)

The percentage of UV area value (wavelength 299 nm) of the active ester (compound SS11) in each analysis result was calculated from the following equation. Percentage of UV area value of active ester (%) = (UV area value of active ester)/(UV area value of Fmoc-Nle-OH + UV area value of active ester + UV area value of peak 1 + UV area value of peak 2) × 100

It should be noted that peak 1 is a peak with a retention time of 1.07 minutes, estimated to be a peak of a product consisting of Fmoc-Nle-OH and DIC, and peak 2 is a peak with a retention time of 1.22 minutes, estimated to be a peak of a dimer of Fmoc-Nle-OH.

Thereafter, the reaction was performed in the same manner as in Reference Example 2-1.

### Reference Example 2-3-2: Isolation and analysis of Fmoc-Nle-MePhe supported on membrane

The peptides were isolated from the membrane obtained in Reference Example 2-3-1 by the method described in Example 1-2, and the production of the peptide of interest (compound SS09*) and the peptide (compound SS10*) in which Fmoc-Gly-OH was elongated in place of Fmoc-Nle-OH was confirmed. The elongation efficiency was calculated according to the equation described in Reference Example 2-1.

The elongation efficiency under the conditions of high reagent concentration using DIC as the condensing agent resulted in high of 83% to 96% as described in Table 9 below. However, precipitates occurred in any of these conditions, thus these conditions were difficult for applying to automated synthesis.

### Example 2-4: Peptide synthesis experiment on membrane under reaction conditions where DsBC was used instead of DIC

### Example 2-4-1: Elongation reaction of Fmoc-Nle-OH to MePhe supported on membrane

An elongation reaction of Fmoc-Nle-OH to MePhe on a membrane was performed using the membrane obtained in Reference Example 2-1-1 (compound SS08) under reaction conditions where DsBC was used instead of DIC.

A solution of Fmoc-Nle-OH (0.63 mol/L) and HOAt (0.198 mol/L or 0.397 mol/L) dissolved in NMP/DMF = 5/6 or 6.68/4.34 or dissolved in DMF was mixed with DsBC (neat) at a ratio of 10 : 1.642 and allowed to stand for 15 minutes. Then, 1.2 µL of this mixed solution was added to the membrane (compound SS08) obtained in Reference Example 2-1-1 and allowed to stand at room temperature for a predetermined time. At this time, a portion of the solution that was allowed to stand for 15 minutes was diluted with MeCN, and the resulting solution was analyzed by LCMS (analysis condition SQDFA05_1). In addition, 1 mL of MeCN was added to the membrane after the reaction time had elapsed, the reaction solution impregnated in the membrane was dissolved, and the resulting MeCN solution was analyzed using LCMS (analysis condition SQDFA05_1). The percentage of UV area value (wavelength 299 nm) of the active ester (compound SS11) in each analysis result was calculated from the following equation. Percentage of UV area value of active ester (%) = (UV area value of active ester)/(UV area value of Fmoc-Nle-OH + UV area value of active ester + UV area value of peak 2 + UV area value of peak 3) × 100

It should be noted that peak 3 is a peak with a retention time of 1.15 minutes, estimated to be a peak of a product consisting of Fmoc-Nle-OH and DsBC, and peak 2 is a peak with a retention time of 1.22 minutes, estimated to be a peak of a dimer of Fmoc-Nle-OH.

Thereafter, the reaction was performed in the same manner as in Reference Example 2-1.

### Example 2-4-2: Isolation and analysis of Fmoc-Nle-MePhe supported on membrane

The peptides were isolated from the membrane obtained in Example 2-4-1 by the method described in Example 1-2, and the production of the peptide of interest (compound SS09*) and the peptide (compound SS10*) in which Fmoc-Gly-OH was elongated in place of Fmoc-Nle-OH was confirmed. The elongation efficiency was calculated according to the equation described in Reference Example 2-1.

The conditions and results of Reference Examples 2-3 and 2-4 are shown in Tables 8 and 9 below.

**[Table 8]**

| Reference Example or Example No. | No. | Fmoc-protected amino acid (concentration in reaction solution) | Activating agent (concentration in reaction solution) | Carbodiimide (concentration in reaction solution) | Reaction solvent | Reaction time |
|---|---|---|---|---|---|---|
| 2-3 | 1 | Fmoc-Nle-OH (0.55mol/L) | HOAt (0.17mol/L) | DIC (0.77mol/L) | NMP/DMF=5/6 | 60 min |
| | 2 | | | | NMP/DMF=6.68/4.34 | |
| | 3 | | | | DMF | |
| | 4 | | HOAt (0.34mol/L) | | NMP/DMF=5/6 | |
| | 5 | | | | NMP/DMF=6.68/4.34 | |
| | 6 | | | | DMF | |
| 2-4 | 1 | Fmoc-Nle-OH (0.55mol/L) | HOAt (0.17mol/L) | DsBC (0.77mol/L) | NMP/DMF=5/6 | 60 min |
| | 2 | | | | NMP/DMF=6.68/4.34 | |
| | 3 | | | | DMF | |
| | 4 | | HOAt (0.34mol/L) | | NMP/DMF=5/6 | |
| | 5 | | | | NMP/DMF=6.68/4.34 | |
| | 6 | | | | DMF | |

**[Table 9]**

| Reference Example or Example No. | No. | Presence or absence of precipitates after 15 minutes of preactivation | Percentage of UV area of active ester at spot | Percentage of UV area of active ester after 60 minutes | Residual rate of active ester | Peptide of interest | Elongation efficiency |
|---|---|---|---|---|---|---|---|
| 2-3 | 1 | presence | 32% | 8% | 25% | SS09* | 93% |
| | 2 | | 37% | 9% | 24% | | 89% |
| | 3 | | 31% | 14% | 47% | | 96% |
| | 4 | | 56% | 12% | 22% | | 90% |
| | 5 | | 64% | 15% | 23% | | 83% |
| | 6 | | 55% | 17% | 31% | | 93% |
| 2-4 | 7 | absence | 32% | 19% | 59% | SS09* | 97% |
| | 8 | | 32% | 20% | 63% | | 97% |
| | 9 | | 36% | 29% | 82% | | 98% |
| | 10 | | 57% | 17% | 30% | | 91% |
| | 11 | | 55% | 16% | 29% | | 90% |
| | 12 | | 56% | 21% | 38% | | 94% |

The condensation was examined under the conditions of high reagent concentration using DsBC as the condensing agent, and the results showed high elongation efficiency of 90% to 98% as described in Table 9. In this condition, no precipitates occurred, so it is possible to apply these conditions to automated synthesis.

### Example 2-5: Comparative effects of DsBC use and DIC use in peptide synthesis on membrane using other Fmoc-protected amino acids and various activating agents

### Example 2-5-1: Elongation reaction of Fmoc-MeSer(THP)-OH to MePhe supported on membrane under conditions using DsBC

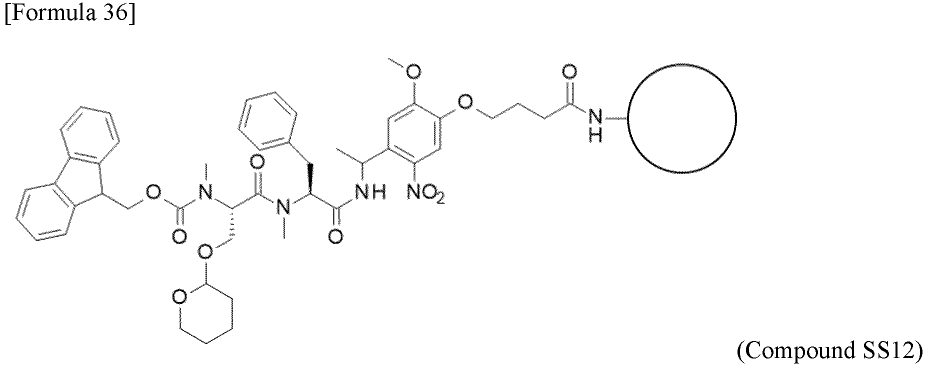

An elongation reaction of Fmoc-MeSer(THP)-OH to MePhe on a membrane was performed using the membrane obtained in Reference Example 2-1-1 (compound SS08) and referring to the reaction conditions of Example 2-4-1. A solution of Fmoc-MeSer(THP)-OH (0.63 mol/L) and HOAt or HOOBt (0.198 mol/L) dissolved in DMF was mixed with DsBC (neat) at a ratio of 10 : 1.642 and allowed to stand for 15 minutes. Then, 1.2 µL of this mixed solution was added to the membrane (compound SS08) obtained in Reference Example 2-1-1 and allowed to stand at room temperature for 30 minutes. Thereafter, the reaction was performed in the same manner as in Reference Example 2-1.

### Reference Example 2-5-2: Elongation reaction of Fmoc-MeSer(THP)-OH to MePhe supported on membrane under conditions using DIC

An elongation reaction of Fmoc-MeSer(THP)-OH to MePhe on a membrane was performed using the membrane obtained in Reference Example 2-1-1 (compound SS08) and referring to the reaction conditions of Reference Example 2-3-1. A solution of Fmoc-MeSer(THP)-OH (0.62 mol/L) and HOAt or HOOBt (0.193 mol/L) dissolved in DMF was mixed with DIC (neat) at a ratio of 10 : 1.353 and allowed to stand for 15 minutes. Then, 1.2 µL of this mixed solution was added to the membrane (compound SS08) obtained in Reference Example 2-1-1 and allowed to stand at room temperature for 30 minutes. Thereafter, the reaction was performed in the same manner as in Reference Example 2-1.

### Example 2-5-3: Elongation reaction of Fmoc-MeAsp(OPis)-OH to MeAla supported on membrane under conditions using DsBC

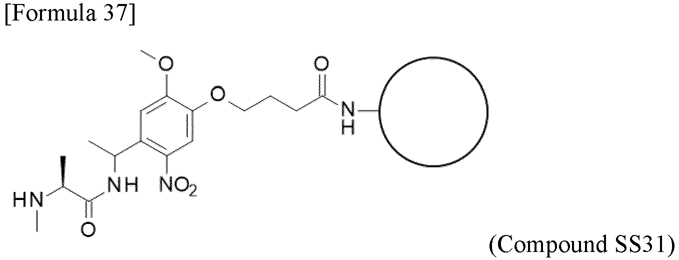

Compound SS31 was obtained using the peptide-supported membrane disc (compound SS24) prepared in Example 1-2-1 and removing the Fmoc group according to the method described in Reference Example 2-1-1.

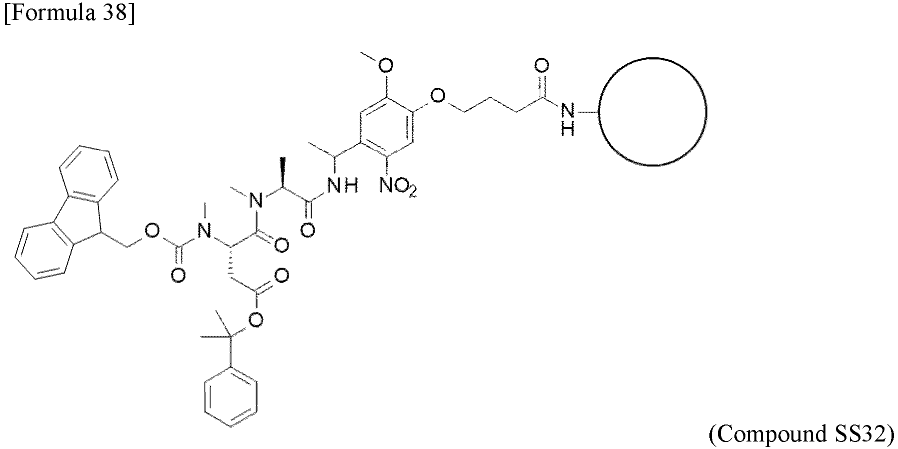

An elongation reaction of Fmoc-MeAsp(OPis)-OH to MeAla on a membrane was performed using the obtained membrane (compound SS31) and referring to the reaction conditions of Example 2-4-1. A solution of Fmoc-MeAsp(OPis)-OH and Oxyma (0.198 mol/L) dissolved in DMF was mixed with DsBC (neat) at a ratio of 10 : 1.642 and allowed to stand for 15 minutes. Then, 1.2 µL of this mixed solution was added to the membrane (compound SS31) and allowed to stand at room temperature for 30 minutes. Thereafter, the reaction was performed in the same manner as in Reference Example 2-1.

### Reference Example 2-5-4: Elongation reaction of Fmoc-MeAsp(OPis)-OH to MeAla supported on membrane under conditions using DIC

An elongation reaction of Fmoc-MeAsp(OPis)-OH to MeAla on a membrane was performed using the membrane (compound SS31) obtained in Example 2-5-3 and referring to the reaction conditions of Reference Example 2-3-1. A solution of Fmoc-MeAsp(OPis)-OH (0.62 mol/L) and Oxyma (0.193 mol/L) dissolved in DMF was mixed with DIC (neat) at a ratio of 10 : 1.353 and allowed to stand for 15 minutes. Then, 1.2 µL of this mixed solution was added to the membrane (compound SS31) and allowed to stand at room temperature for 30 minutes. Thereafter, the reaction was performed in the same manner as in Reference Example 2-1.

### Example 2-5-5: Elongation reaction of Fmoc-MePhe-OH to MeSer (THP) supported on membrane under conditions using DsBC

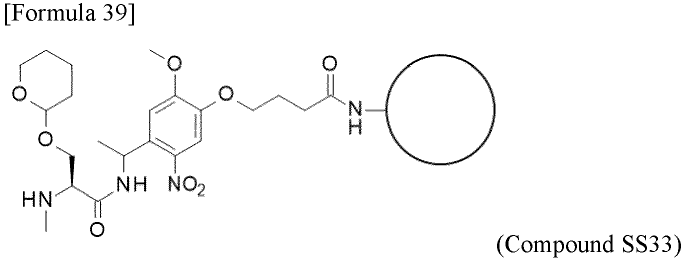

Compound SS33 was obtained using the peptide-supported membrane disc (compound SS25) prepared in Example 1-2-1 and removing the Fmoc group according to the method described in Reference Example 2-1-1.

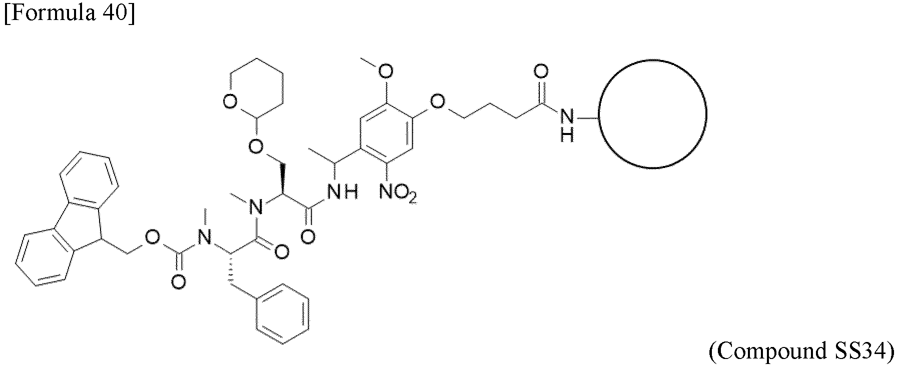

An elongation reaction of Fmoc-MePhe-OH to MeSer(THP) on a membrane was performed using the obtained membrane (compound SS33) and referring to the reaction conditions of Example 2-4-1. A solution of Fmoc-MePhe-OH (0.63 mol/L) and HOAt (0.198 mol/L) dissolved in DMF was mixed with DsBC (neat) at a ratio of 10 : 1.642 and allowed to stand for 15 minutes. Then, 1.2 µL of this mixed solution was added to the membrane (compound SS33) and allowed to stand at room temperature for 30 minutes. Thereafter, the reaction was performed in the same manner as in Reference Example 2-1.

### Reference Example 2-5-6: Elongation reaction of Fmoc-MePhe-OH to MeSer(THP) supported on membrane under conditions using DIC

An elongation reaction of Fmoc-MePhe-OH to MeSer(THP) on a membrane was performed using the membrane obtained in Example 2-5-5 (compound SS33) and referring to the reaction conditions of Reference Example 2-3-1. A solution of Fmoc-MePhe-OH (0.62 mol/L) and HOAt (0.193 mol/L) dissolved in DMF was mixed with DIC (neat) at a ratio of 10 : 1.353 and allowed to stand for 15 minutes. Then, 1.2 µL of this mixed solution was added to the membrane (compound SS33) and allowed to stand at room temperature for 30 minutes. Thereafter, the reaction was performed in the same manner as in Reference Example 2-1.

### Example 2-5-7: Isolation and analysis of various peptides supported on membrane

The peptides were isolated from the membranes obtained in Examples (Reference Examples) 2-5-1 to 2-5-6 by the method described in Example 1-2, and the production of the peptides of interest (compounds SS12*, SS32*, and SS34*) and the peptides (compounds SS10*, SS35*, and SS36*) in which Fmoc-Gly-OH was elongated in place of a desired Fmoc-protected amino acid (the peptides are also referred to as Glycine capping peptides) was confirmed. 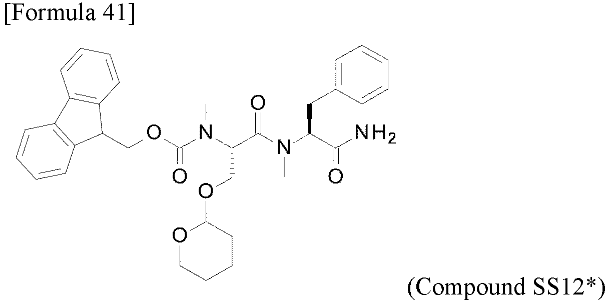 LCMS (ESI) m/z = 586.5 [M + H]⁺
Retention time: 3.61 minutes (analysis condition SQDAA05long) 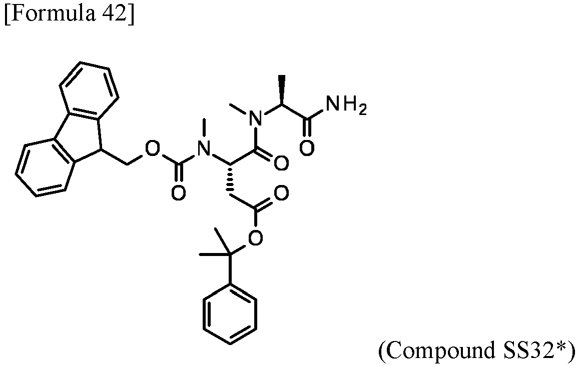 LCMS (ESI) m/z = 572.5 [M + H]⁺
Retention time: 3.52 minutes (analysis condition SQDAA05long) 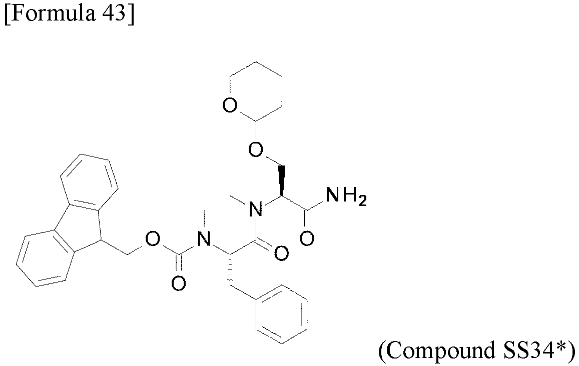 LCMS (ESI) m/z = 586.4 [M + H]⁺
Retention time: 3.56 minutes (analysis condition SQDAA05long) 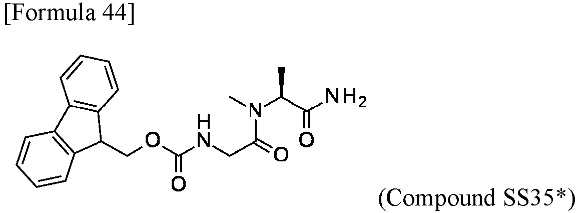 LCMS (ESI) m/z = 382.3 [M + H]⁺
Retention time: 2.58 minutes (analysis condition SQDAA05long) 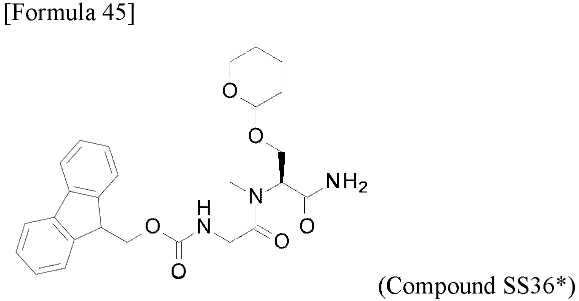 LCMS (ESI) m/z = 482.4 [M + H]⁺
Retention time: 3.03 minutes (analysis condition SQDAA05long)

The elongation efficiency was calculated according to the equation described in Reference Example 2-1, using UV area values (wavelength 299 nm) of peaks of each compound in the LC data. Elongation efficiency (%) = (UV area value of peptide of interest)/(sum of UV area value of peptide of interest and UV area value of Glycine capping peptide) × 100

The conditions and results of Examples (Reference Examples) 2-5-1 to 2-5-6 are shown in Table 10 below.

**[Table 10]**

| Example or Reference Example No. | No. | Compound No. of starting material membrane and amino acid on membrane | Fmoc-protected amino acid (concentration in reaction solution) | Activating agent (concentration in reaction solution) | Carbodiimide (concentration in reaction solution) | Presence or absence of precipitates after 15 minutes of preactivation | Peptide of interest | Glycine capping peptide | Elongation efficiency |
|---|---|---|---|---|---|---|---|---|---|
| Example 2-5-1 | 1 | SS08 MePhe | Fmoc-MeSer(THP)-OH (0.55mol/L) | HOAt (0.17mol/L) | DsBC (0.77mol/L) | absence | SS12* | SS10* | 92% |
| | 2 | | | HOOBt (0.17mol/L) | | absence | SS12* | SS10* | 97% |
| Reference Example 2-5-2 | 3 | | | HOAt (0.17mol/L) | DIC (0.77mol/L) | presence | SS12* | SS10* | 83% |
| | 4 | | | HOOBt (0.17mol/L) | | presence | SS12* | SS10* | 93% |
| Example 2-5-3 | 5 | SS31 MeAla | Fmoc-MeAsp(OPis)-OH (0.55mol/L) | Oxyma (0.17mol/L) | DsBC (0.77mol/L) | absence | SS32* | SS35* | 73% |
| Reference Example 2-5-4 | 6 | | | | DIC (0.77mol/L) | presence | SS32* | SS35* | 68% |
| Example 2-5-5 | 7 | SS33 MeSer(THP) | Fmoc-MePhe-OH (0.55mol/L) | HOAt (0.17mol/L) | DsBC (0.77mol/L) | absence | SS34* | SS36* | 89% |
| Reference Example 2-5-6 | 8 | | | | DIC (0.77mol/L) | presence | SS34* | SS36* | 88% |

Precipitates occurred in any of the conditions in which DIC was used as the condensing agent, thus these conditions were difficult for applying to automated synthesis. On the other hand, in the conditions using DsBC as the condensing agent, no precipitates occurred even in condensation to various peptides with various Fmoc-protected amino acids and activating agents, thus these conditions were possible to apply to automated synthesis, and the elongation efficiency was equal to or higher than that in the conditions using DIC.

### Example 2-6: Verification experiment of solubility of urea

### Example 2-6-1: Experiments to confirm solubility of DIC urea, DsBC urea and tBEC urea in DMF

The concentration of carbodiimide at the time of preactivation performed in the elongation steps of Reference Example 2-3 and Examples 2-4 and 2-5 was 0.77 mol/L. To confirm the risk of precipitation in case that all carbodiimide is converted to urea, the solubility in NMP and DMF was confirmed according to the following procedure, using the DIC-derived urea with which precipitates were confirmed in Reference Examples 2-3 and the DsBC-derived urea with which no precipitates were confirmed in Examples 2-4 and 2-5.

Into 1.5 mL vials with a screw cap, DIC urea (compound SS05) and DsBC urea (compound SS06) synthesized in Example 1-3 were weighed and placed as described in Table 11, and NMP or DMF was added to be each concentration. The mixture was stirred at room temperature for 5 minutes or more, and then allowed to stand to confirm whether the urea was dissolved or not. As a result, the solid of compound SS05 that is a DIC urea was not fully dissolved in NMP at a concentration of 0.34 M or more and in DMF at 0.17 M or more, while compound SS06 that is a DsBC urea was completely dissolved in both NMP and DMF at 0.77 M and no re-precipitation was confirmed even after 24 hours or more.

**[Table 11]**

| Urea | Urea weight | Solvent | Concentration | Dissolved or not |
|---|---|---|---|---|
| | 5.26 mg | NMP | 0.17 M | dissolved |
| | 5.26 mg | | 0.34 M | not fully dissolved |
| | 8.11 mg | | 0.38 M | not fully dissolved |
| | 8.83 mg | | 0.51 M | not fully dissolved |
| DIC Urea (SS05) | 9.74 mg | | 0.77 M | not fully dissolved |
| | 5.39 mg | DMF | 0.17 M | not fully dissolved |
| | 5.83 mg | | 0.34 M | not fully dissolved |
| | 8.24 mg | | 0.38 M | not fully dissolved |
| | 8.91 mg | | 0.51 M | not fully dissolved |
| | 9.79 mg | | 0.77 M | not fully dissolved |
| DsBC Urea (SS06) | 11.00 mg | NMP | 0.77 M | dissolved |
| | 15.06 mg | DMF | 0.77 M | dissolved |

### Example 2-6-2: Experiment to confirm solubility of tBEC urea in DMF

An experiment similar to Example 2-6-1 but using tBEC, which has an alkyl group different from those of DIC and DsBC, was performed. Into 1.5 mL vials with a screw cap, 10.18 mg and 10.19 mg of tBEC urea (compound SS07) synthesized in Example 1-3 were weighed and placed, and NMP or DMF was added to each to be 0.77 mol/L. The mixture was stirred at room temperature for 5 minutes or more, and then allowed to stand to confirm whether the urea was dissolved or not. As a result, similarly to DsBC urea, tBEC urea was completely dissolved and no re-precipitation was confirmed even after 24 hours or more.

### Example 2-7: Peptide synthesis experiment on membrane under high concentration conditions with other carbodiimides

### Example 2-7-1: Elongation reaction of Fmoc-Nle-OH to MePhe supported on membrane with tBEC

An elongation reaction of Fmoc-Nle-OH to MePhe on a membrane was performed with tBEC with which good solubility of the urea in DMF was confirmed in Example 2-6, using the membrane (compound SS08) obtained in Reference Example 2-1-1 and referring to the reaction conditions of Example 2-4-1.

A solution of Fmoc-Nle-OH (0.62 mol/L) and HOAt (0.194 mol/L) dissolved in DMF was mixed with tBEC (neat) at a ratio of 10 : 1.355 and allowed to stand for 15 minutes. Then, 1.2 µL of this mixed solution was added to the membrane (compound SS08) obtained in Reference Example 2-1-1 and allowed to stand at room temperature for 30 minutes. Thereafter, the reaction was performed in the same manner as in Reference Example 2-1.

### Example 2-7-2: Isolation and analysis of Fmoc-Nle-MePhe supported on membrane

The peptides were isolated from the membrane obtained in Example 2-7-1 by the method described in Example 1-2, and the production of the peptide of interest (compound SS09*) and the peptide (compound SS10*) in which Fmoc-Gly-OH was elongated in place of Fmoc-Nle-OH was confirmed. The elongation efficiency was calculated according to the equation described in Reference Example 2-1.

The results of Example 2-7 are shown in Table 12.

**[Table 12]**

| Example No. | Activating agent (concentration in reaction solution) | Carbodiimide (concentration in reaction solution) | Presence or absence of precipitates after 15 minutes of preactivation | Elongation efficiency |
|---|---|---|---|---|
| 2-7 | HOAt (0.17mol/L) | tBEC (0.77mol/L) | absence | 87% |

In the condensation reaction using tBEC instead of DsBC as the condensing agent, no precipitates occurred and the elongation of Fmoc-protected amino acid was possible as described in Table 12.

### Example 2-7-3: Elongation reaction of Fmoc-Nle-OH to MePhe supported on membrane with various condensing agents

An elongation reaction of Fmoc-Nle-OH to MePhe on a membrane was performed with various carbodiimides synthesized in Example 1-4, using the membrane (compound SS08) obtained in Reference Example 2-1-1 and referring to the reaction conditions of Example 2-4-1.

A solution of Fmoc-Nle-OH and HOAt dissolved in DMF was mixed with carbodiimides (neat) according to Table 13 below and allowed to stand for 15 minutes. Then, 1.2 µL of this mixed solution was added to the membrane (compound SS08) obtained in Reference Example 2-1-1 and allowed to stand at room temperature for 30 minutes. Thereafter, the reaction was performed in the same manner as in Reference Example 2-1.

**[Table 13]**

| Example No. | No. | Solution A | | Solution B | Mixing ratio of solution A and solution B |
|---|---|---|---|---|---|
| | | Concentration of Fmoc-Nle-OH in DMF solution | Concentration of HOAt in DMF solution | Carbodiimide (concentration of neat) | |
| 2-7 | 1 | 0.65mol/L | 0.20mol/L | SS26 (4.69mol/L) | 10 : 1.964 |
| | 2 | 0.68mol/L | 0.21mol/L | SS27 (3.97mol/L) | 10 : 2.402 |
| | 3 | 0.66mol/L | 0.21mol/L | SS28 (4.54mol/L) | 10 : 2.041 |
| | 4 | 0.66mol/L | 0.20mol/L | SS29 (4.58mol/L) | 10 : 2.020 |
| | 5 | 0.66mol/L | 0.20mol/L | SS30 (4.59mol/L) | 10 : 2.017 |

### Example 2-7-4: Isolation and analysis of Fmoc-Nle-MePhe supported on membrane

The peptides were isolated from the membrane obtained in Example 2-7-3 by the method described in Example 1-2, and the production of the peptide of interest (compound SS09*) and the peptide (compound SS10*) in which Fmoc-Gly-OH was elongated in place of Fmoc-Nle-OH was confirmed. The elongation efficiency was calculated according to the equation described in Reference Example 2-1.

The results are shown in Table 14.

**[Table 14]**

| Example No. | No. | Fmoc-protected amino acid (concentration in reaction solution) | Activating agent (concentration in reaction solution) | Carbodiimide (concentration in reaction solution) | Presence or absence of precipitates after 15 minutes of preactivation | Elongation efficiency |
|---|---|---|---|---|---|---|
| 2-7 | 1 | Fmoc-Nle-OH (0.55mol/L) | HOAt (0.17mol/L) | SS26 (0.77mol/L) | absence | 99% |
| | 2 | | | SS27 (0.77mol/L) | absence | 99% |
| | 3 | | | SS28 (0.77mol/L) | absence | 97% |
| | 4 | | | SS29 (0.77mol/L) | presence | 99% |
| | 5 | | | SS30 (0.77mol/L) | absence | 98% |

The use of any carbodiimide as the condensing agent resulted in high elongation efficiency of 97% to 99% as described in Table 14. In addition, no precipitates occurred and the elongation of Fmoc-protected amino acids was possible in the condensation reactions using compounds SS26, SS27, SS28, and SS30, thus these conditions were possible to apply to automated synthesis.

### Example 2-8: Peptide synthesis experiments on membrane with various reaction temperatures, reaction solvents and equivalent amounts of reagents

### Example 2-8-1: Elongation reaction of Fmoc-Nle-OH to MePhe supported on membrane with various reaction temperatures, reaction solvents and equivalent ratios of reagents

An elongation reaction of Fmoc-Nle-OH to MePhe on a membrane was performed using the membrane (compound SS08) obtained in Reference Example 2-1-1 and by changing the reaction temperature, the reaction solvent and the equivalent of reagents based on the reaction conditions of Example 2-4-1.

A solution of Fmoc-Nle-OH and HOAt dissolved in various reaction solvents was mixed with DsBC (neat) according to Table 15 below and allowed to stand for 15 minutes. Then, 1.2 µL of this mixed solution was added to the membrane (compound SS08) obtained in Reference Example 2-1-1 and allowed to stand at various temperatures for 30 minutes. Thereafter, the reaction was performed in the same manner as in Reference Example 2-1.

**[Table 15]**

| Example No. | No. | Solution A | | | Solution B | Mixing ratio of solution A and solution B | Reaction temperature |
|---|---|---|---|---|---|---|---|
| | | Concentration of Fmoc-Nle-OH | Concentration of HOAt | Solvent | DsBC (concentration of neat) | | |
| | 1 | 0.63mol/L | 0.20mol/L | DMF | | 10 : 1.642 | 40°C |
| | 2 | 0.63mol/L | 0.20mol/L | DMF | | 10 : 1.642 | 60°C |
| | 3 | 0.63mol/L | 0.20mol/L | DMI | | 10 : 1.642 | room temperature |
| | 4 | 0.63mol/L | 0.20mol/L | toluene/NMP=1/1 | | 10 : 1.642 | room temperature |
| | 5 | 0.63mol/L | 0.20mol/L | DCE/NMP=1/1 | | 10 : 1.642 | room temperature |
| 2-8 | 6 | 0.63mol/L | 0.20mol/L | DGDE/NMP=1/1 | 5.46mol/L | 10 : 1.642 | room temperature |
| | 7 | 0.63mol/L | 0.095mol/L | DMF | | 10 : 1.642 | room temperature |
| | 8 | 0.63mol/L | 0.14mol/L | DMF | | 10 : 1.642 | room temperature |
| | 9 | 0.61mol/L | 0.19mol/L | DMF | | 10 : 1.266 | room temperature |
| | 10 | 0.62mol/L | 0.19mol/L | DMF | | 10 : 1.427 | room temperature |
| | 11 | 0.68mol/L | 0.21mol/L | DMF | | 10 : 2.497 | room temperature |

### Example 2-8-2: Isolation and analysis of Fmoc-Nle-MePhe supported on membrane

The peptides were isolated from the membrane obtained in Example 2-8-1 by the method described in Example 1-2, and the production of the peptide of interest (compound SS09*) and the peptide (compound SS10*) in which Fmoc-Gly-OH was elongated in place of Fmoc-Nle-OH was confirmed. The elongation efficiency was calculated according to the equation described in Reference Example 2-1.

The results are shown in Table 16.

**[Table 16]**

| Example No. | No. | Concentration of Fmoc-Nle-OH in reaction solution | Concentration of HOAt in reaction solution | Concentration of DsBC in reaction solution | Ratio (Fmoc-Nle-OH : HOAt : DsBC) | Reaction solvent | Reaction temperature | Presence or absence of precipitates after 15 minutes of preactivation | Elongation efficiency |
|---|---|---|---|---|---|---|---|---|---|
| | 1 | | 0.17mol/L | 0.77mol/L | 1.00 : 0.31 : 1.41 | DMF | 40°C | absence | 99% |
| | 2 | | 0.17mol/L | 0.77mol/L | 1.00 : 0.31 : 1.41 | DMF | 60°C | absence | 99% |
| | 3 | | 0.17mol/L | 0.77mol/L | 1.00 : 0.31 : 1.41 | DMI | room temperature | absence | 82% |
| | 4 | | 0.17mol/L | 0.77mol/L | 1.00 : 0.31 : 1.41 | toluene/NMP=1/1 | room temperature | absence | 93% |
| | 5 | | 0.17mol/L | 0.77mol/L | 1.00 : 0.31 : 1.41 | DCE/NMP=1/1 | room temperature | absence | 94% |
| 2-8 | 6 | 0.55mol/L | 0.17mol/L | 0.77mol/L | 1.00 : 0.31 : 1.41 | DGDE/NMP=1/1 | room temperature | absence | 88% |
| | 7 | | 0.082mol/L | 0.77mol/L | 1.00 : 0.15 : 1.41 | DMF | room temperature | absence | 97% |
| | 8 | | 0.12mol/L | 0.77mol/L | 1.00 : 0.23 : 1.41 | DMF | room temperature | absence | 97% |
| | 9 | | 0.17mol/L | 0.61mol/L | 1.00 : 0.31 : 1.13 | DMF | room temperature | absence | 96% |
| | 10 | | 0.17mol/L | 0.68mol/L | 1.00 : 0.31 : 1.25 | DMF | room temperature | absence | 97% |
| | 11 | | 0.17mol/L | 1.09mol/L | 1.00 : 0.31 : 2.00 | DMF | room temperature | absence | 99% |

As shown in Table 16, even when the reaction temperature was 40°C or 60°C, no precipitates occurred and the elongation efficiency was high. In addition, even when reaction solvents including a urea-based solvent, a benzene-based solvent, a halogen-based solvent, and an ether-based solvent were used, no precipitates occurred and the elongation efficiency was high. Further, even when the ratio of additive (here, the case of HOAt was shown.) and the condensing agent was changed, no precipitates occurred and the elongation of Fmoc-protected amino acids was possible.

In the elongation step of Reference Example 2-1, which is a widely-practiced elongation step in array synthesis of peptide consisting of general natural amino acids, and in the elongation step of Reference Example 2-2 with reference to the reaction conditions reported as a synthesis method of peptides containing N-substituted amino acids, the elongation efficiency of Fmoc-Nle-OH to MePhe on the membrane was only 3-4% and 50-64%, respectively. In the method described in Reference Example 2-3 in which the elongation reaction was performed while increasing the reagent concentration to increase the elongation efficiency, the elongation efficiency was improved to 83-96%, but the concentration of the active ester had been significantly decreased even 30 minutes after the start of the elongation reaction and there was another problem of the precipitation of DIC urea that occurred during preactivation of 15 minutes. An article discloses the examination of the types of carbodiimide to avoid cyanide generation during the combination use of OxymaPure with carbodiimide such as DCC and DIC (Org. Lett. 2021, 23, 6900-6904) and describes that the reactivity of amidation when using DsBC is lower compared to DIC. However, under the high concentration conditions of Reference Example 2-3, it was observed that the elongation efficiency was improved by the method of Example 2-4 where DsBC was used as carbodiimide, compared to the results of Reference Examples 2-3. At this time, it was confirmed that the active ester can be maintained at a high concentration state for a longer time than the case where DIC was used. In addition, when DsBC was used, no precipitation of DsBC urea was observed during the preactivation of 15 minutes. As shown in Example 2-5, this reaction can be applied even when the amino acids and activating agents are different, and it was confirmed that the desired peptide can be produced without precipitation of urea. In fact, as shown in Example 2-6, it was confirmed that the risk of precipitation is extremely low even if an entire of DsBC is converted to urea. It was also confirmed that the risk of precipitation of urea is extremely low when using tBEC that has an alkyl group different from that of DsBC. Furthermore, in the condensation reaction with compounds SS26, SS27, SS28, and SS30, no precipitates were observed in the preactivation solution, and Fmoc-protected amino acid was able to be elongated, as shown in Example 2-7. Furthermore, as shown in Examples 2-8, it was confirmed that the reaction conditions with DsBC, where the desired peptide can be produced without precipitation of urea, can be changed also in the temperature-increasing conditions such as 40°C and 60°C, various reaction solvents, and the equivalent ratios of reagents.

From the results of Example 2 above, a part of reaction conditions including the type of carbodiimide that can suppress precipitation of urea derived from carbodiimide while improving the elongation efficiency was specified in the elongation step of peptide synthesis on a membrane.

### Example 3: Experiments of application scope expansion of substrate and application to automated synthesis with peptide synthesizer

In this experiment, elongation reactions were performed using preferred reaction conditions in peptide synthesis on a membrane specified in Example 2 and with other amino acids. The evaluation of the elongation efficiency of amino acids was performed in the same manner as in Example 2. In addition, these conditions were applied to a peptide synthesizer.

### Example 3-1: Peptide synthesis experiments on membrane with various amino acids and activating agents

### Example 3-1-1: Removal of Fmoc group on Pro supported on membrane

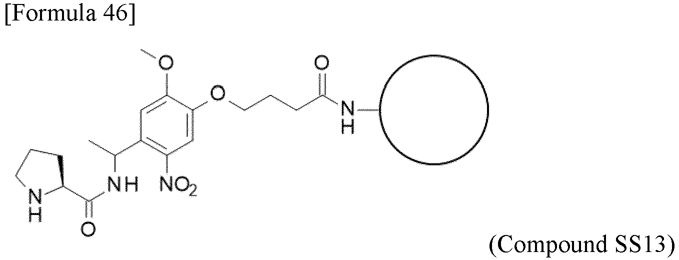

Compound SS13 was obtained using the peptide-supported membrane disc (compound SS03) prepared in Example 1-2-1 and removing the Fmoc group according to the method described in Reference Example 2-1-1.

### Example 3-1-2: Elongation reaction of Fmoc-protected amino acid to Pro supported on membrane

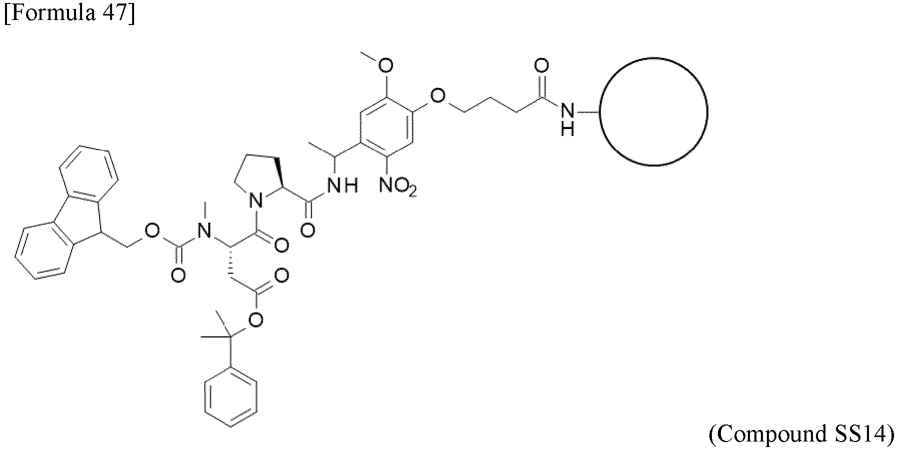

An elongation reaction of Fmoc-Asp(OPis)-OH to Pro on a membrane was performed using the membrane obtained in Example 3-1-1 (compound SS13) under reaction conditions where tBEC was used instead of DIC.

A solution of Fmoc-Asp(OPis)-OH (0.62 mol/L) and an activating agent (0.194 mol/L or 0.387 mol/L) dissolved in DMF was mixed with tBEC (neat) at a ratio of 10 : 1.355 and allowed to stand for 15 minutes. Then, 1.2 µL of this mixed solution was added to the membrane and allowed to stand at room temperature for 30 minutes. Thereafter, the reaction was performed in the same manner as in Reference Example 2-1.

### Example 3-1-3: Isolation and analysis of various peptides supported on membrane

The peptides were isolated from the membrane obtained in Example 3-1-2 by the method described in Example 1-2, and the production of the peptide of interest (compound SS14*) and the peptide (compound SS15*) in which Fmoc-Gly-OH was elongated in place of a desired Fmoc-protected amino acid was confirmed. 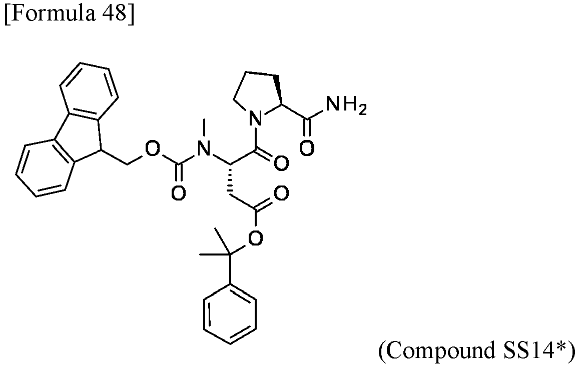 LCMS (ESI) m/z = 584.5 [M + H]⁺
Retention time: 3.55 minutes (analysis condition SQDAA05long) 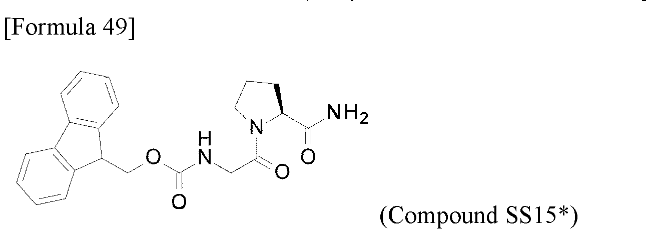 LCMS (ESI) m/z = 394.3 [M + H]⁺
Retention time: 2.61 to 2.62 minutes (analysis condition SQDAA05long)

The elongation efficiency was calculated according to the equation described in Reference Example 2-5.

The results are shown in Table 17.

**[Table 17]**

| Example No. | No. | Compound No. of starting material membrane and amino acid on membrane | Fmoc-protected amino acid | Activating agent (concentration in reaction solution) | Presence or absence of precipitates after 15 minutes of preactivation | Peptide of interest | Glycine capping peptide | Elongation efficiency |
|---|---|---|---|---|---|---|---|---|
| 3-1 | 1 | SS13 Pro | Fmoc-MeAsp(OPis)-OH | HOAt (0.17mol/L) | absence | SS14* | SS15* | 94% |
| | 2 | | | HOAt (0.34mol/L) | | | | 92% |
| | 3 | | | HOOBt (0.17mol/L) | | | | 97% |
| | 4 | | | Oxyma (0.17mol/L) | | | | 98% |

In all the cases of any sequence and any activating agent were used, no precipitates were observed in the preactivation solution and the synthesis of a peptide of interest was possible.

### Reference Example 3-1-4: Elongation reaction of Fmoc-protected amino acid to MePhe supported on membrane under conditions using DIC

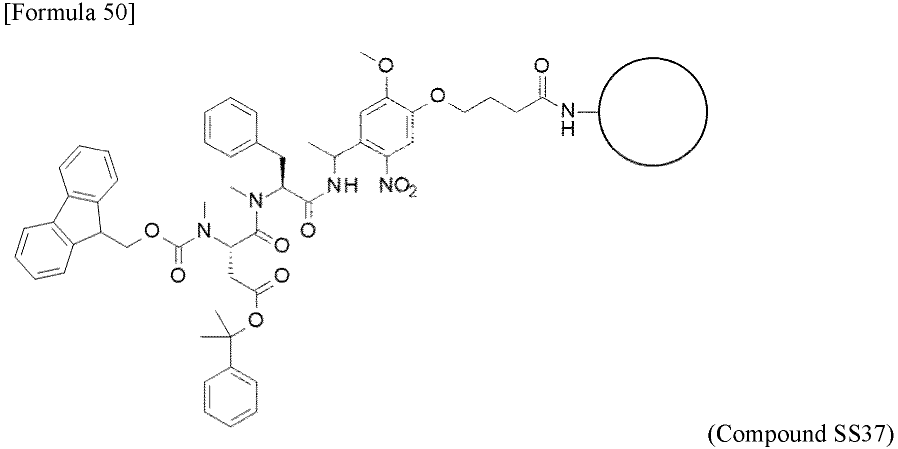

To confirm the effectiveness of this condition for the elongation reaction of various N-alkylamino acids, an elongation reaction of Fmoc-Asp(OPis)-OH to MePhe on a membrane was performed using the membrane (compound SS08) obtained in Reference Example 2-1-1 and referring to the reaction conditions of Reference Example 2-3-1.

A solution of Fmoc-Asp(OPis)-OH and Oxyma dissolved in DMF was mixed with DIC (neat) according to Table 18 below and allowed to stand for 15 minutes. Then, 1.2 µL of this mixed solution was added to the membrane (compound SS08) obtained in Reference Example 2-1-1 and allowed to stand at room temperature for 30 minutes. Thereafter, the reaction was performed in the same manner as in Reference Example 2-1.

### Example 3-1-5: Elongation reaction of Fmoc-protected amino acid to MePhe supported on membrane

An elongation reaction of Fmoc-Asp(OPis)-OH to MePhe on a membrane was performed with various carbodiimides synthesized in Example 1-4, using the membrane (compound SS08) obtained in Reference Example 2-1-1 and referring to the reaction conditions of Example 2-7-3.

A solution of Fmoc-Asp(OPis)-OH and Oxyma dissolved in DMF was mixed with each of various carbodiimides (neat) according to Table 18 below and allowed to stand for 15 minutes. Then, 1.2 µL of this mixed solution was added to the membrane (compound SS08) obtained in Reference Example 2-1-1 and allowed to stand at room temperature for 30 minutes. Thereafter, the reaction was performed in the same manner as in Reference Example 2-1.

**[Table 18]**

| Example or Reference Example No. | No. | Solution A | | Solution B | Mixing ratio of solution A and solution B |
|---|---|---|---|---|---|
| | | Concentration of Fmoc-MeAsp(OPis)-OH in DMF solution | Concentration of Oxyma in DMF solution | Carbodiimide (concentration of neat) | |
| Reference Example 3-1-4 | 1 | 0.62mol/L | 0.19mol/L | DIC (6.46mol/L) | 10 : 1.353 |
| Example 3-1-5 | 2 | 0.63mol/L | 0.20mol/L | DsBC (5.46mol/L) | 10 : 1.642 |
| | 3 | 0.65mol/L | 0.20mol/L | SS26 (4.69mol/L) | 10 : 1.964 |
| | 4 | 0.68mol/L | 0.21mol/L | SS27 (3.97mol/L) | 10 : 2.402 |
| | 5 | 0.66mol/L | 0.21mol/L | SS28 (4.54mol/L) | 10 : 2.041 |
| | 6 | 0.66mol/L | 0.20mol/L | SS29 (4.58mol/L) | 10 : 2.020 |
| | 7 | 0.66mol/L | 0.20mol/L | SS30 (4.59mol/L) | 10 : 2.017 |

### Example 3-1-6: Isolation and analysis of various peptides supported on membrane

The peptides were isolated from the membranes obtained in Reference Example 3-1-4 and Example 3-1-5 by the method described in Example 1-2, and the production of the peptide of interest (compound SS37*) and the peptide (compound SS10*) in which Fmoc-Gly-OH was elongated in place of a desired Fmoc-protected amino acid was confirmed. 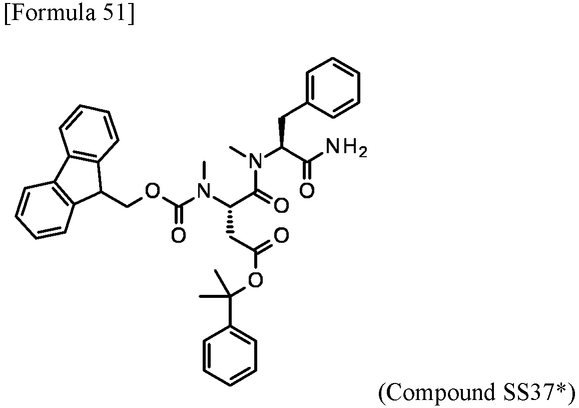 LCMS (ESI) m/z = 648.5 [M + H]⁺
Retention time: 3.80 minutes (analysis condition SQDAA05long)

The elongation efficiency was calculated according to the equation described in Reference Example 2-5.

The results are shown in Table 19.

**[Table 19]**

| Example or Reference Example No. | No. | Fmoc-protected amino acid (concentration in reaction solution) | Activating agent (concentration in reaction solution) | Carbodiimide (concentration in reaction solution) | Presence or absence of precipitates after 15 minutes of preactivation | Elongation efficiency |
|---|---|---|---|---|---|---|
| Reference Example 3-1-4 | 1 | Fmoc-MeAsp(OPis)-OH (0.55mol/L) | Oxyma (0.17mol/L) | DIC (0.77mol/L) | presence | 49% |
| Example 3-1-5 | 2 | | | DsBC (0.77mol/L) | absence | 55% |
| | 3 | | | SS26 (0.77mol/L) | absence | 56% |
| | 4 | | | SS27 (0.77mol/L) | absence | 64% |
| | 5 | | | SS28 (0.77mol/L) | absence | 59% |
| | 6 | | | SS29 (0.77mol/L) | presence | 71% |
| | 7 | | | SS30 (0.77mol/L) | absence | 64% |

Under the conditions in which DIC was used as the condensing agent, the elongation efficiency was only 49%, and precipitates occurred, thus these conditions were difficult for applying to automated synthesis. On the other hand, when DsBC or various carbodiimides were used as the condensing agent, the elongation efficiency was 55% to 71% as shown in Table 19, which was higher than when DIC was used. In addition, no precipitates occurred and the elongation of Fmoc-protected amino acids was possible in the condensation reactions using DsBC and compounds SS26, SS27, SS28, and SS30, thus these conditions were possible to apply to automated synthesis.

### Example 3-2: Peptide synthesis experiment on membrane with peptide synthesizer

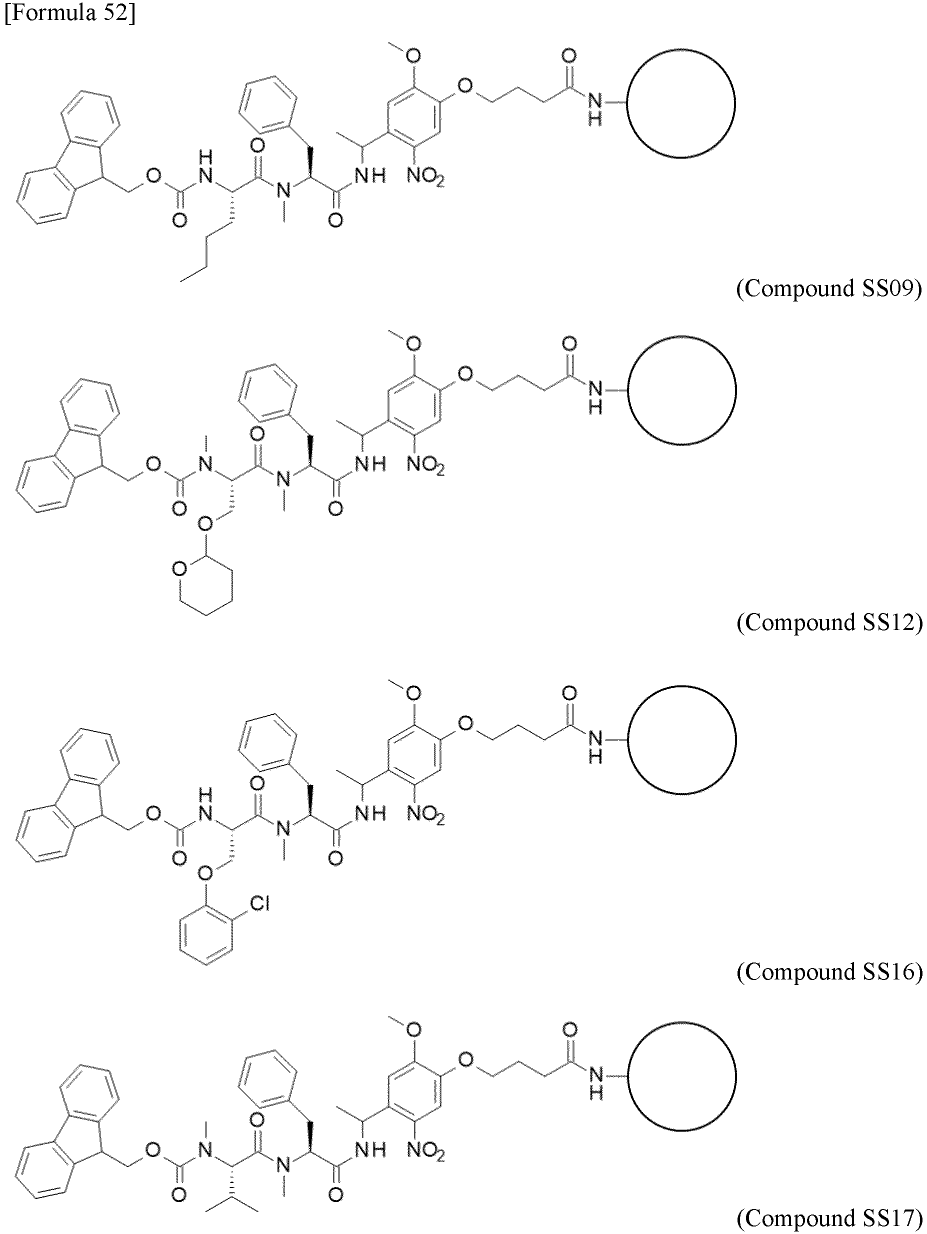

Preferred reaction conditions in peptide synthesis on membrane specified in Example 2 were applied to synthesis on an automated synthesizer. This experiment was performed by an Fmoc method using a peptide synthesizer (Multipep RS; manufactured by Intavis Bioanalytical Instruments AG). Detailed procedures of operations were performed by the method below in accordance with the manual attached to the synthesizer. In the automated synthesizer, double coupling was performed to yield a stable and high elongation efficiency even when the different types of Fmoc-protected amino acids were used. For example, when the elongation efficiency in a single coupling is about 90%, it is expected that the elongation efficiency will become about 99% by performing a double coupling.

The Fmoc-protected amino acid (0.63 mol/L) to be elongated and HOAt or Oxyma or HOOBt (0.198 mol/L or 0.397 mol/L) were dissolved in DMF to prepare solution 1. DsBC was used without dilution as solution 2.

Solution 3 was prepared by dissolving Fmoc-Gly-OH (0.6 mol/L) and HOAt (0.375 mol/L) in NMP to perform glycine capping after elongation of Fmoc-protected amino acid. DIC (0.71 mol/L) and DMF were mixed to prepare solution 4.

The membrane (compound SS02) obtained in Example 1-2-1 was mounted on CelluSpots 384 frame with acid stable discs (manufactured by Intavis Bioanalytical Instruments AG), and set into a peptide synthesizer. Solutions 1 to 4 were set in the peptide synthesizer, and automated synthesis with the peptide synthesizer was started.

### De-Fmoc step

A solution of DBU in DMF (2%v/v) was added in amounts of 2.0 µL and 4.0 µL per membrane disc to deprotect the Fmoc group at room temperature. After 2.0 µL of the solution was added and allowed to react for 5 minutes, the solution was discharged once, then 4.0 µL of the solution was added and allowed to react for another 10 minutes, and then the solution was discharged. Subsequently, the membrane disc was washed 7 times with DMF (25 µL per membrane disc), 2 times with ethanol (25 µL per membrane disc), 2 times with ethanol (37.5 µL per membrane disc), and 2 times with ethanol (25 µL per membrane disc), and then dried under reduced pressure for 15 minutes.

### Elongation step (double coupling)

Solution 1 and solution 2 were mixed at a ratio of 10 : 1.642 in a mixing vial of the synthesizer and allowed to stand for 15 minutes. The mixed solution was then added in an amount of 1.2 µL per membrane disc and allowed to react at room temperature for 40 minutes to perform a condensation reaction between the amino group on the membrane disc and the Fmoc-protected amino acid, then the solution was discharged. Subsequently, the membrane was washed 4 times with DMF (25 µL per membrane disc) and 3 times with ethanol (25 µL per membrane disc), and then dried under pulling pressure for 15 minutes. Then, the condensation reaction, washing and drying were repeated one more time.

### Glycine capping step

Subsequently, solution 3 and solution 4 were mixed at a ratio of 5 : 6 in a mixing vial of the synthesizer and allowed to stand for 15 minutes. The mixed solution was then added in an amount of 3.0 µL per membrane disc and allowed to react at room temperature for 40 minutes to perform glycine capping, then the solution was discharged. Then, the Fmoc-Gly-OH elongation reaction described above was performed again. The membrane disc was then washed 7 times with DMF (25 µL per membrane disc) and dried under reduced pressure for 10 minutes. After the amino acid elongation, a de-Fmoc step was not performed, and the membrane disc was washed 7 times with DMF (25 µL per membrane disc), 2 times with ethanol (25 µL per membrane disc), 2 times with ethanol (37.5 µL per membrane disc), and 3 times with ethanol (25 µL per membrane disc), and then dried under reduced pressure for 10 minutes.

The peptides were isolated by the method described in Examples 1-2 using the prepared compounds SS09, SS12, SS16, and SS17, and the production of the peptides of interest (compounds SS09*, SS12*, SS16*, and SS17*) was confirmed. 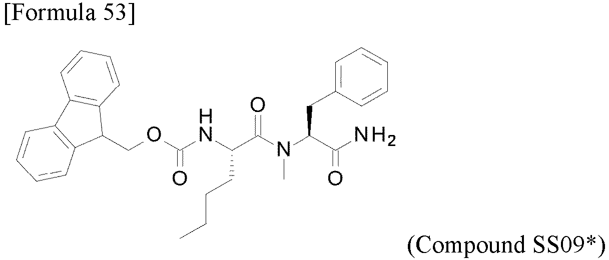 LCMS (ESI) m/z = 514.4 [M + H]⁺
Retention time: 3.63 minutes (analysis condition SQDAA05long) 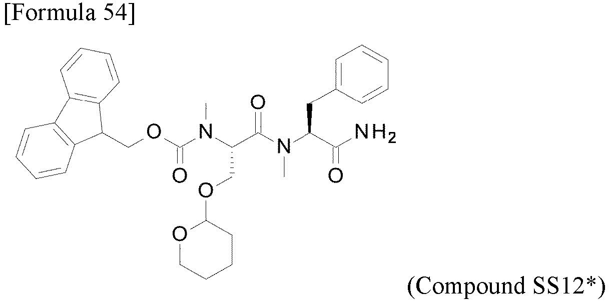 LCMS (ESI) m/z = 586.4 [M + H]⁺
Retention time: 3.61 minutes (analysis condition SQDAA05long) 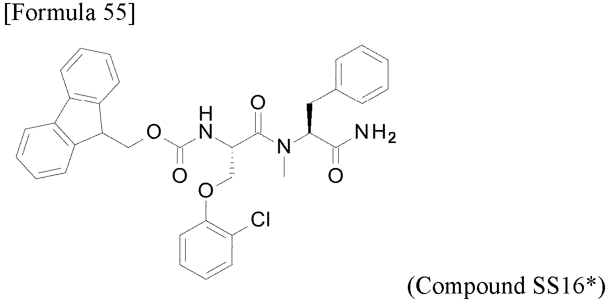 LCMS (ESI) m/z = 598.3 [M + H]⁺
Retention time: 3.72 minutes (analysis condition SQDAA05long) 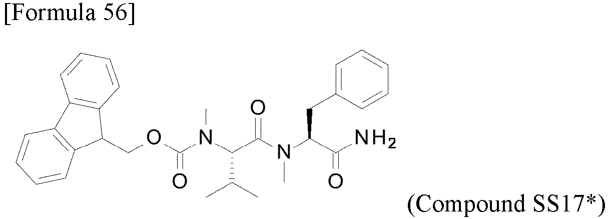 LCMS (ESI) m/z = 514.4 [M + H]⁺
Retention time: 3.49 minutes (analysis condition SQDAA05long)

The results of Example 3-2 are shown in Table 20.

**[Table 20]**

| Example No. | No. | Fmoc-protected amino acid (concentration in reaction solution) | Activating agent (concentration in reaction solution) | Carbodiimide (concentration in reaction solution) | Peptide of interest | Elongation efficiency |
|---|---|---|---|---|---|---|
| 3-2 | 1 | Fmoc-Nle-OH (0.55mol/L) | HOAt (0.17mol/L) | DsBC (0.77mol/L) | SS09* | >99% |
| | 2 | Fmoc-MeSer(THP)-OH (0.55mol/L) | HOOBt (0.17mol/L) | | SS12* | >99% |
| | 3 | Fmoc-Ser(Ph-2-Cl)-OH (0.55mol/L) | HOOBt (0.17mol/L) | | SS16* | >99% |
| | 4 | Fmoc-MeVal-OH (0.55mol/L) | Oxyma (0.34mol/L) | | SS17* | 87% |

With any of the substrates, no precipitation of DsBC-derived urea was observed and automated synthesis by a peptide synthesizer was possible.

### Example 3-3: Cyclic peptide synthesis experiment on membrane with peptide synthesizer

Preferred reaction conditions in peptide synthesis on membrane specified in Example 2 were applied to synthesis on an automated synthesizer to perform parallel synthesis of cyclic peptides.

### Example 3-3-1: Peptide elongation on membrane with peptide synthesizer

Elongation of the peptide was carried out with an automated synthesizer with referring to the reaction conditions of Examples 1-2 and 3-2, and the sequences described in Table 21 below were elongated.

**[Table 21]**

| Compound No. | N-terminus | 14 | 13 | 12 | 11 | 10 | 9 | 8 | 7 | 6 | 5 | 4 | 3 | 2 | 1 | C-terminus |
|---|---|---|---|---|---|---|---|---|---|---|---|---|---|---|---|---|
| SS38 | Fmoc | MeAla | Thr(THP) | nPrGly | MePhe | MePhe | MeLeu | Pro | MeLeu | Ala | MeVal | Asp(OPis) | Pic(2) | PEG6 | Photo-Linker | Membrane |
| SS39 | Fmoc | MeGly | MeLeu | MeLeu | Thr(THP) | MePhe | Ala | MeLeu | MeLeu | MePhe | Ile | Asp(OPis) | Pic(2) | PEG6 | Photo-Linker | Membrane |
| SS40 | Fmoc | MeAla | Leu | MeLeu | MePhe | Ala | MePhe(3-Cl) | MeLeu | Thr(THP) | MeGly | MeLeu | Asp(OPis) | Pic(2) | PEG6 | Photo-Linker | Membrane |
| SS41 | Fmoc | D-Ala | MePhe | Leu | MeLeu | Thr(THP) | MeGly | MeLeu | Ser(iPen) | MePhe | MeLeu | Asp(OPis) | Pic(2) | PEG6 | Photo-Linker | Membrane |
| SS42 | Fmoc | MeAla | MePhe | MeLeu | Val | MeLeu | MePhe | MePhe | Thr(THP) | MeAla | MePhe | Asp(OPis) | Pic(2) | PEG6 | Photo-Linker | Membrane |
| SS43 | Fmoc | MeGly | MePhe | Ile | MeLeu | Thr(THP) | nPrGly | MePhe | MeLeu | Ser(tBuOH) | MeLeu | Asp(OPis) | Pic(2) | PEG6 | Photo-Linker | Membrane |
| SS44 | Fmoc | D-MeAla | MeLeu | Leu | MeLeu | Thr(THP) | Pic(2) | MeLeu | Val | MeLeu | Leu | Asp(OPis) | Pro | PEG6 | Photo-Linker | Membrane |
| SS45 | Fmoc | MeAla | Ser(iPen) | MeGly | Aze(2) | MeSer(iPen) | MeGly | Phe(3-Cl) | Nle | MeSer(iPen) | MeSer(nPr) | MeAsp(OPis) | Pro | PEG6 | Photo-Linker | Membrane |
| SS46 | Fmoc | D-MeAla | Ser(Ph-2-Cl) | MeLeu | Ser(iPen) | Leu | Pro | MePhe | R-AMPA | MePhe | Ile | MeAsp(OPis) | Pro | PEG6 | Photo-Linker | Membrane |

This Example was performed by an Fmoc method using a peptide synthesizer (Multipep RS; manufactured by Intavis Bioanalytical Instruments AG). Detailed procedures of operations were performed by the method below in accordance with the manual attached to the synthesizer. It should be noted that double coupling was performed in the automated synthesizer to yield a stable and high elongation efficiency even when the different types of Fmoc-protected amino acids are used.

To adjust the number of reaction points on a membrane at the first residue elongation, Fmoc-Photo-Linker (0.072 mol/L), 4-phenoxybutyric acid (0.22 mol/L) and HOBt (0.181 mol/L) were dissolved in DMF to prepare solution 1. DIC was used without dilution as solution 2. The Fmoc-protected amino acid (0.63 mol/L) to be elongated and HOAt or Oxyma or HOOBt (0.198 mol/L or 0.397 mol/L) were dissolved in DMF or NMP to prepare solution 3 as a solution for use in the elongation of the second residue or after. The combinations are described in Table 22 below. DsBC was used without dilution as solution 4.

**[Table 22]**

| Example No. | Solution 3 | | | | Example No. | Solution 3 | | |
|---|---|---|---|---|---|---|---|---|
| | Fmoc-protected amino acid (concentration in solution 3) | Activating agent (concentration in solution 3) | Solvent | | | Fmoc-protected amino acid (concentration in solution 3) | Activating agent (concentration in solution 3) | Solvent |
| | Fmoc-MeVal-OH (0.63mol/L) | Oxyma (0.397mol/L) | NMP | | | Fmoc-Ser(Ph-2-Cl)-OH (0.63mol/L) | HOOBt (0.198mol/L) | DMF |
| | Fmoc-MeLeu-OH (0.63mol/L) | HOAt (0.198mol/L) | NMP | | | Fmoc-Ser(tBuOH)-OH (0.63mol/L) | HOOBt (0.198mol/L) | DMF |
| | Fmoc-MePhe(3-Cl)-OH (0.63mol/L) | HOAt (0.198mol/L) | DMF | | | Fmoc-Ser(iPen)-OH (0.63mol/L) | HOOBt (0.198mol/L) | DMF |
| | Fmoc-MePhe-OH (0.63mol/L) | HOAt (0.198mol/L) | DMF | | | Fmoc-Nle-OH (0.63mol/L) | HOAt (0.198mol/L) | DMF |
| | Fmoc-MeSer(iPen)-OH (0.63mol/L) | HOOBt (0.198mol/L) | DMF | | | Fmoc-Ala-OH (0.63mol/L) | HOAt (0.198mol/L) | DMF |
| | Fmoc-MeSer(nPr)-OH (0.63mol/L) | HOOBt (0.198mol/L) | DMF | | | Fmoc-D-Ala-OH (0.63mol/L) | HOAt (0.198mol/L) | DMF |
| 3-3-1 | Fmoc-MeAla-OH (0.63mol/L) | HOAt (0.198mol/L) | DMF | | 3-3-1 | Fmoc-Pic(2)-OH (0.63mol/L) | HOAt (0.198mol/L) | NMP |
| | Fmoc-D-MeAla-OH (0.63mol/L) | HOAt (0.198mol/L) | DMF | | | Fmoc-Pro-OH (0.63mol/L) | HOAt (0.198mol/L) | NMP |
| | Fmoc-Thr(THP)-OH (0.63mol/L) | HOOBt (0.198mol/L) | DMF | | | Fmoc-Aze(2)-OH (0.63mol/L) | HOAt (0.198mol/L) | NMP |
| | Fmoc-Ile-OH (0.63mol/L) | HOAt (0.198mol/L) | DMF | | | Fmoc-nPrGly-OH (0.63mol/L) | HOAt (0.198mol/L) | DMF |
| | Fmoc-Val-OH (0.63mol/L) | Oxyma (0.198mol/L) | DMF | | | Fmoc-MeGly-OH (0.63mol/L) | HOAt (0.198mol/L) | DMF |
| | Fmoc-R-AMPA-OH (0.63mol/L) | HOAt (0.198mol/L) | DMF | | | Fmoc-MeAsp(OPis)-OH (0.63mol/L) | Oxyma (0.198mol/L) | DMF |
| | Fmoc-Leu-OH (0.63mol/L) | HOAt (0.198mol/L) | DMF | | | Fmoc-Asp(OPis)-OH (0.63mol/L) | Oxyma (0.198mol/L) | DMF |
| | Fmoc-Phe(3-Cl)-OH (0.63mol/L) | HOAt (0.198mol/L) | DMF | | | Fmoc-PEG6-OH (0.63mol/L) | HOAt (0.198mol/L) | DMF |

CelluSpots 384 frame with acid stable discs (manufactured by Intavis Bioanalytical Instruments AG) were set into a peptide synthesizer. Solutions 1 to 4 were set in the peptide synthesizer, and automated synthesis with the peptide synthesizer was started.

### De-Fmoc step

### [First residue]

This operation was omitted because there was no Fmoc group at the N-terminus on the membrane.

### [Second residue or after]

A solution of DBU in DMF (2%v/v) was added in an amount of 6.0 µL per membrane disc to deprotect the Fmoc group at room temperature. After 6.0 µL of the solution was added, the mixture was allowed to react for 15 minutes, then the solution was discharged. Subsequently, the membrane was washed 7 times with DMF (37.5 µL per membrane disc) and 6 times with ethanol (37.5 µL per membrane disc), and then dried under reduced pressure for 15 minutes.

### Elongation step (double coupling)

### [First residue]

Solution 1 and solution 2 were mixed at a ratio of 11.29 : 0.72 in a mixing vial of the synthesizer and allowed to stand for 15 minutes. The mixed solution was then added in an amount of 1.2 µL per membrane disc and allowed to react at room temperature for 40 minutes to perform a condensation reaction between the amino group on the membrane disc and the Fmoc-protected amino acid, then the solution was discharged. Subsequently, the membrane was washed 4 times with DMF (37.5 µL per membrane disc) and 3 times with ethanol (37.5 µL per membrane disc), and then dried under reduced pressure for 15 minutes. The condensation reaction was then performed one more time for 30 minutes, and then the membrane was washed 7 times with DMF (37.5 µL per membrane disc) and 5 times with ethanol (37.5 µL per membrane disc), and then dried under reduced pressure for 15 minutes.

### [Second residue or after]

Solution 3 and solution 4 were mixed at a ratio of 10.31 : 0.169 in a mixing vial of the synthesizer and allowed to stand for 10 minutes. The mixed solution was then added in an amount of 1.2 µL per membrane disc and allowed to react at room temperature for 30 minutes to perform a condensation reaction between the amino group on the membrane disc and the Fmoc-protected amino acid, then the solution was discharged. Subsequently, the membrane was washed 4 times with DMF (37.5 µL per membrane disc) and 3 times with ethanol (37.5 µL per membrane disc), and then dried under reduced pressure for 15 minutes. The condensation reaction was then performed one more time for 30 minutes, and then a solution of acetic anhydride (Ac₂O) in DMF (4%v/v) was added in an amount of 4.0 µL per membrane disc to perform acetyl capping of unreacted amines at room temperature. After the reaction for 5 minutes, the solution was discharged. The membrane disc was then washed 7 times with DMF (37.5 µL per membrane disc) and dried under reduced pressure for 10 minutes.

This condensation reaction of the Fmoc-protected amino acid subsequent to the deprotection reaction of the Fmoc group and the acetyl capping were set to one cycle, and this cycle was repeated to elongate a peptide on the surface of the membrane disc. After the final amino acid elongation, acetyl capping, DMF washing, and drying, the membrane was further washed 6 times with ethanol (37.5 µL per membrane disc) and dried under reduced pressure for 15 minutes.

### Example 3-3-2: Deprotection, cyclization, isolation and analysis of various peptides supported on membrane

Deprotection reaction, cyclization reaction, isolation from the membrane, and analysis were performed using the membranes obtained in Example 3-3-1 (Compounds SS38 to SS46), and the production of the peptides of interest (compounds SS47 to SS55) described in Table 23 below was confirmed.

**[Table 23]**

| Compound No. | Structure |
|---|---|
| SS47 | 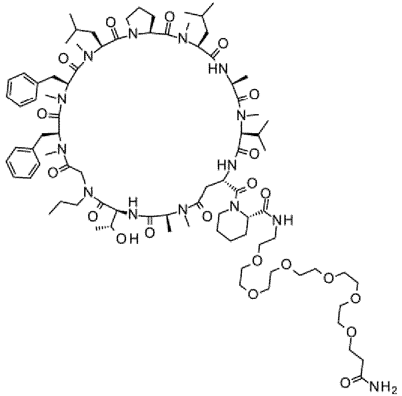 |
| SS48 | 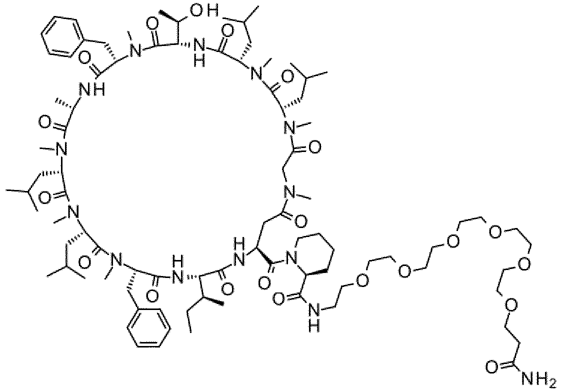 |
| SS49 | 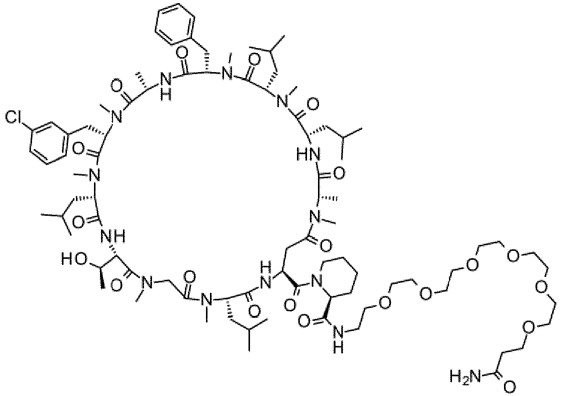 |
| SS50 | 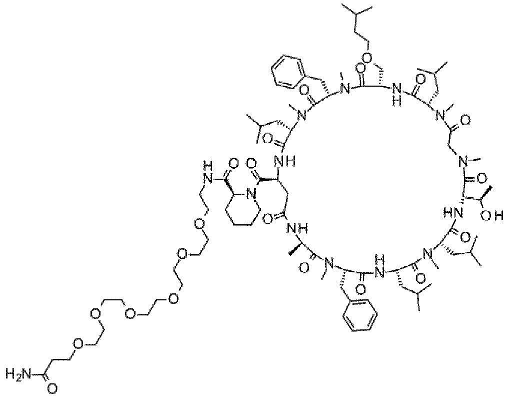 |
| SS51 | 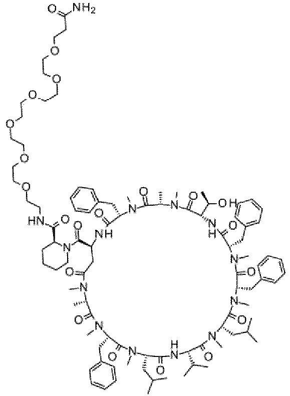 |
| SS52 | 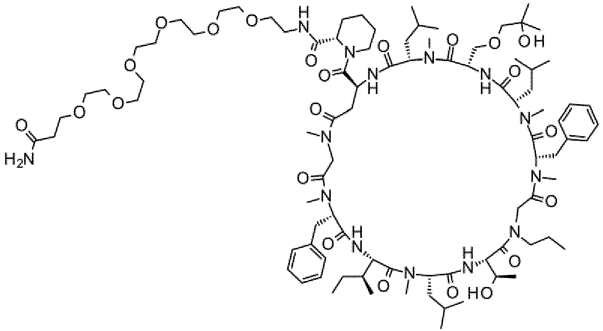 |
| SS53 | 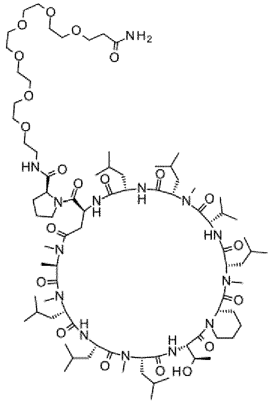 |
| SS54 | 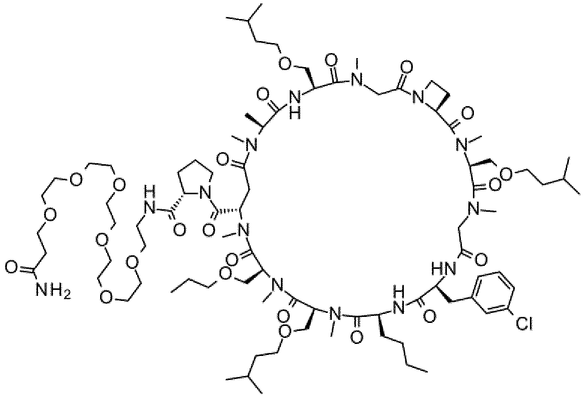 |
| SS55 | 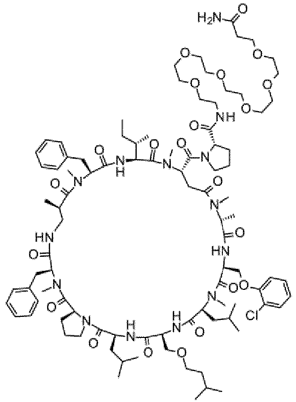 |

### [Removal of N-terminal Fmoc group]

The membrane discs were placed in reaction vessels one by one, and a solution of DBU in DMF (2%v/v) was added in an amount of 4.0 µL per membrane disc and allowed to react at room temperature for 5 minutes, and then added again in an amount of 4.0 µL and allowed to react at room temperature for 10 minutes to remove the Fmoc group. Subsequently, the membrane disc was washed 4 times with 100 µL of DMF per membrane disc and 3 times with 100 µL of DCM per membrane disc, and dried in air.

### [Removal of protecting groups of side chain functional groups]

In a mixed solution of HFIP (2.47 mL), TIPS (51.2 µL), and DCE (21.3 µL), 21.9 mg of tetramethylammonium hydrogen sulfate was dissolved to prepare a 0.05 M solution of tetramethylammonium hydrogen sulfate.

The membrane discs were placed into reaction vessels one by one, and the prepared solution was added in an amount of 150 µL per membrane disc and allowed to react at room temperature for 4.5 hours to remove Pis and THP groups. Subsequently, the membrane disc was washed 2 times with 100 µL of NMP per membrane disc, then 100 µL of a solution of DIPEA in NMP (60 mM) was added and allowed to stand at room temperature for 5 minutes. The solution was discharged, then the membrane disc was washed once with 100 µL of NMP and 3 times with DCM, and dried in air.

### [Cyclization reaction]

PyOxim (49.8 mg) was dissolved in a mixture of NMP (184 µL) and THF (1.66 mL), and DIPEA (19.7 µL) was added to prepare a 50 mM PyOxim/60 mM DIPEA solution.

The membrane discs were placed into reaction vessels one by one, and the prepared solution was added in an amount of 150 µL per membrane disc, heated to 50°C, and allowed to react for 2 hours to perform a cyclization reaction. Subsequently, the membrane disc was washed 4 times with 100 µL of DMF per membrane disc and 3 times with 100 µL of DCM per membrane disc, and dried in air.

### [Isolation, Analysis]

The membrane discs were placed into reaction vessels one by one, and the peptides were isolated and analyzed in the following procedure with reference to the method described in Examples 1-2, and the production of the peptides of interest (compounds SS47 to SS55) was confirmed.

To the membrane disc in the reaction vessel placed at room temperature, 10 µL of DMSO was added. It was then irradiated with light at a UV wavelength of 365 nm and an illuminance of 380-600 mW/cm² for 2 minutes and 30 seconds. Then, 10 µL of DMSO was added to the membrane, and the mixture was allowed to stand for 15 minutes or more to dissolve the peptide. The solution was analyzed by LCMS.

The purity of the peptide of interest was calculated from the following equation using UV area values (wavelength 210-400 nm PDAtotal) of peaks of each compound in the LC data. Purity (%) = (UV area value of peptide of interest)/(sum of UV area values of all peaks except peaks in background and from impurities contained in Fmoc-PEG6-OH) × 100

It is estimated that the impurities contained in Fmoc-PEG6-OH were compounds in which the Fmoc group had been oxidized and no longer deprotected.

The results of Example 3-3 are shown in Table 24.

**[Table 24]**

| Compound No. | Analysis conditions | LCMS (ESI) m/z | | Retention time (min) | Purity (%) |
|---|---|---|---|---|---|
| SS47 | SQDAA05long | 1702.4 | [M+H]⁻ | 3.79 | 41.7 |
| SS48 | SQDAA05long | 1746.3 | [M+H]⁻ | 4.04 | 37.1 |
| SS49 | SQDAA05long | 1738.4 | [M+H]⁻ | 3.87 | 46.0 |
| SS50 | SQDAA05long | 1776.2 | [M+H]⁻ | 4.02 | 67.1 |
| SS51 | SQDAA05long | 1828.1 | [M+H]⁻ | 4.03 | 58.9 |
| SS52 | SQDAA05long | 1806.1 | [M+H]⁻ | 3.93 | 67.6 |
| SS53 | SQDAA05long | 1676.4 | [M+H]⁻ | 3.94 | 53.1 |
| SS54 | SQDAA05long | 1806.1 | [M+H]⁻ | 3.96 | 68.8 |
| SS55 | SQDAA05long | 1856.1 | [M+H]⁻ | 4.03 | 66.6 |

In the crude product stage before the purification step, the cyclic peptide of interest was obtained with a purity of 37 to 69%. The reaction conditions were applied to peptide elongation in an automated synthesizer and showed that parallel synthesis of cyclic peptides on a membrane was possible.

### Example 4: Study and experiment on range of applicable support

In this experiment, peptide synthesis experiments on resin were carried out using the reaction conditions according to the synthesis method of peptides containing N-substituted amino acids (WO 2018/225851) and the preferred reaction conditions for peptide synthesis on membrane specified in Example 2. Cl-Trt(2-Cl) resin (1.25-1.60 mmol/g, 100-200 mesh, 1% DVB) was purchased from WATANABE CHEMICAL INDUSTRIES, LTD. or SUNRESIN Co. Ltd.

### Example 4-1: Synthesis of Fmoc-Asp(O-Trt(2-Cl)-resin)-NMe2

### Example 4-1-1: Synthesis of (3S)-4-(dimethylamino)-3-(9H-fluoren-9-ylmethoxycarbonylamino)-4-oxobutanoic acid (Fmoc-Asp-NMe2, SS18)

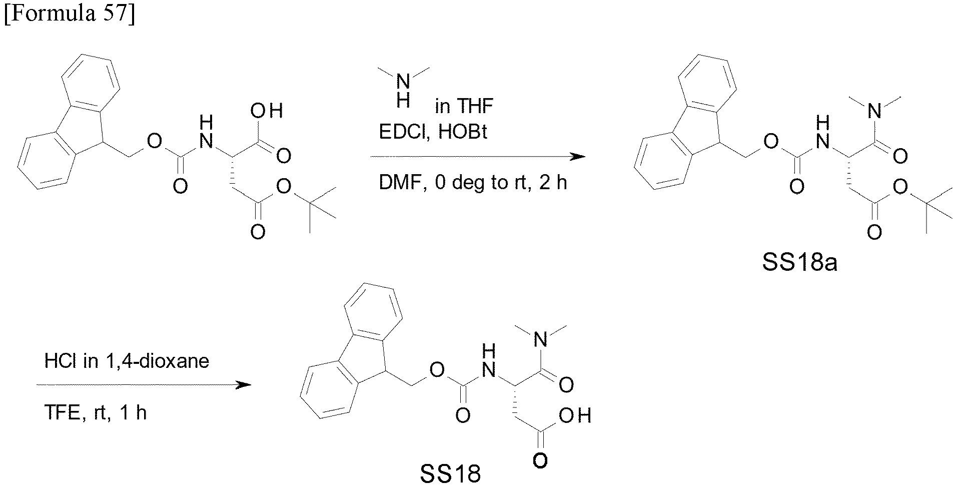

According to the scheme described above, (3S)-4-(dimethylamino)-3-(9H-fluoren-9-ylmethoxycarbonylamino)-4-oxobutanoic acid (Fmoc-Asp-NMe2, SS18) was synthesized.

### Example 4-1-2: Synthesis of (3S)-4-(dimethylamino)-3-(9H-fluoren-9-ylmethoxycarbonylamino)-4-oxobutanoic acid-2-methylpropan-2-yl (Fmoc-Asp (OtBu)-NMe2, compound SS18a)

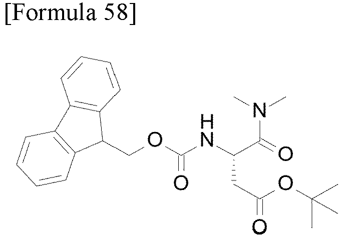

Synthesis was carried out using Fmoc-Asp(OtBu)-OH (25.0 g, 60.8 mmol) purchased from a commercial supplier as a starting material. Fmoc-Asp(OtBu)-OH (25.0 g, 60.8 mmol), EDCI·HCl (14.0 g, 72.9 mmol), and DMF (122 mL) were added to a reaction vessel under nitrogen stream, and the mixture was stirred at room temperature for 5 minutes, then cooled to 0°C. HOBt (9.03 g, 66.8 mmol) was added at 0°C and the mixture was stirred for 45 minutes. Dimethylamine (2 mol/L THF solution, 31.3 mL, 62.6 mmol) was added dropwise at 0°C for 15 minutes, and the mixture was stirred at 0°C for 1 hour. The mixture was further stirred in a water bath for 1 hour, then ethyl acetate/TBME (1/1, 250 mL) was added to the reaction solution, and the organic layer was washed once with 0.5 mol/L aqueous hydrochloric acid solution (200 mL) and 2 times with water (200 mL), and then the organic layer was dried over sodium sulfate. The desiccant was filtered off, and then the filtrate was concentrated under reduced pressure to obtain 29.8 g (yield 112%) of compound SS18a as a crude product.
LCMS (ESI) m/z = 461.3 [M + Na]⁺
Retention time: 0.88 minutes (analysis condition SQDFA05_2)

### Example 4-1-3: Synthesis of (3S)-4-(dimethylamino)-3-(9H-fluoren-9-ylmethoxycarbonylamino)-4-oxobutanoic acid (Fmoc-Asp-NMe2, compound SS18)

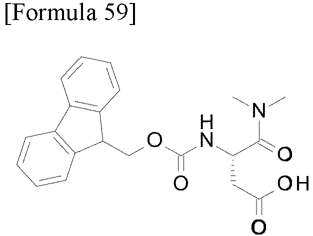

A crude product of compound SS18a (29.8 g) and TFE (270 mL) were added to the reaction vessel, then a 4 mol/L dioxane hydrochloride solution (15.2 mL, 60.8 mmol) was added dropwise, and then the reaction solution was stirred at room temperature for 1 hour. The reaction solution was diluted with TBME (500 mL) and then extracted with a 5% aqueous sodium carbonate solution (600 mL). The obtained aqueous layer was made acidic to a pH of about 2 to 3 by adding an 85% aqueous phosphoric acid solution (40 to 50 mL), and the aqueous layer was extracted with TBME (400 mL). The obtained organic layer was washed with a 10% aqueous sodium chloride solution (400 mL) and water (400 mL), and the organic layer was then dried over sodium sulfate. The desiccant was filtered off, and then the filtrate was concentrated under reduced pressure to obtain 21.4 g (yield 92%) of compound SS18.
LCMS (ESI) m/z = 383.2 [M + H]⁺
Retention time: 0.66 minutes (analysis condition SQDFA05_2)

### Example 4-1-4: Synthesis of (3S)-4-(dimethylamino)-3-(9H-fluoren-9-ylmethoxycarbonylamino)-4-oxobutanoic acid-2-chlorotrityl resin (Fmoc-Asp(O-Trt(2-Cl)-resin)-NMe2, compound SS19)

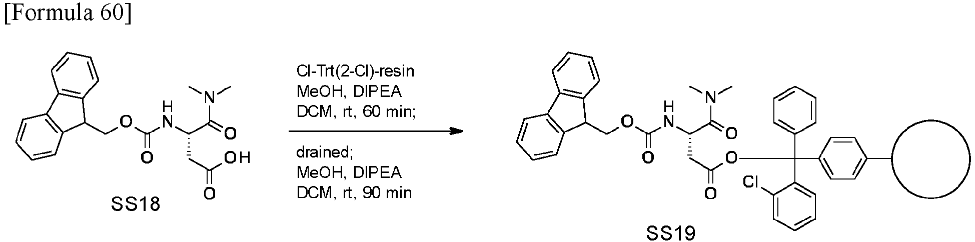

To a reaction vessel with a filter, 2-chlorotrityl chloride resin (1.44 mmol/g, 39.0 g, 56.2 mmol) and DCM (390 mL) were added, and the mixture was shaken at room temperature for 20 minutes. Nitrogen pressure was applied to remove DCM, then a mixture of compound SS18 (10.7 g, 28.0 mmol), methanol (9.05 mL, 224 mmol), DIPEA (23.5 mL, 134 mmol), and DCM (273 mL) was added to the reaction vessel and shaken at room temperature for 60 minutes. Nitrogen pressure was applied to remove the reaction solution, then a mixture of methanol (36.2 mL, 895 mmol), DIPEA (23.5 mL, 134 mmol), and DCM (273 mL) was added to the reaction vessel and shaken at room temperature for 90 minutes. Nitrogen pressure was applied to remove the reaction solution, then DCM (390 mL) was added. The reaction vessel was shaken for 5 minutes, and then nitrogen pressure was applied to remove the reaction solution. The resin washing operation using DCM was repeated 3 times, and the obtained resin was dried overnight under reduced pressure to obtain 45.0 g of compound SS19.

### Fmoc quantitation method

For determination of the amount supported, the obtained compound SS19 (10.48 mg) was put in the reaction vessel, DMF (4.0 mL) was added, and the mixture was shaken at room temperature for 30 minutes. Thereafter, DBU (40 µL) was added, and the mixture was shaken at 30°C for 15 minutes. Thereafter, DMF was added so that the amount of the reaction mixed solution was 10.0 mL, and 80 µL of the resulting solution was diluted with DMF (920 µL). The resulting dilution solution was analyzed by LC/MS (analysis condition SQDFA05 _1, injection volume: 5 µL) and the amount of compound SS19 supported was calculated as 0.469 mmol/g from the UVarea value of dibenzofulvene (UVarea value at 294 nm: 4929.82, UVarea value at 304 nm: 4428.76). (A calibration curve prepared on the basis of UVarea values of dibenzofulvene at wavelengths of 294 nm and 304 nm every measurement day using a mixed solution of Fmoc-Gly-OH having a known concentration (purchased from a commercial supplier) and DBU as a standard substance were used to calculate the amount supported at respective wavelengths, and the average value of the amounts supported was taken as an amount supported on resin.)

Another lot with a different amount supported, which had been similarly synthesized, was also used for peptide synthesis, studies and the like.

### Example 4-2: Peptide synthesis experiments under different reaction conditions on resin

### Example 4-2-1: Preparation of compound SS20 (MeVal-Asp(O-Trt(2-Cl)-resin)-NMe2)

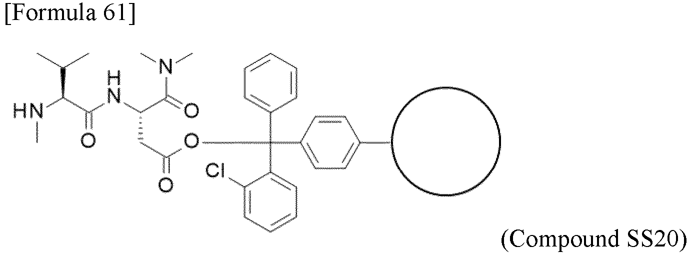

Preparation of compound SS20 was performed by an Fmoc method according to the synthesis method of peptides containing N-substituted amino acids (WO 2018/225851) using a peptide synthesizer (Multipep RS; manufactured by Intavis Bioanalytical Instruments AG). Detailed procedures of operations were performed in accordance with the manual attached to the synthesizer.

Fmoc-MeVal-OH (0.6 mol/L) constituting a peptide of interest and HOAt as a carboxylic acid activating agent (0.375 mol/L) were dissolved in NMP to prepare solution 1. DIC (0.71 mol/L) and DMF were mixed to prepare solution 2.

(3S)-4-(Dimethylamino)-3-(9H-fluoren-9-ylmethoxycarbonylamino)-4-oxobutanoic acid-2-chlorotrityl resin (compound SS19, Fmoc-Asp(O-Trt(2-Cl)-resin)-NMe2) (100 mg per column) prepared in Example 4-1-4 was added to a solid phase reaction vessel and set into a peptide synthesizer. To this resin (100 mg), DCM (1.0 mL) was added, and the mixture was left standing for about 30 minutes to swell the resin. Solutions 1 and 2 were set in a peptide synthesizer, and automated synthesis with the peptide synthesizer was started. The solution was discharged from a frit, and subsequently, the resin was washed 2 times with DMF (0.7 mL per column).

### De-Fmoc step

A solution of DBU in DMF (2%v/v, 0.7 mL per column) was added, and the deprotection of the Fmoc group was performed at room temperature to 30°C. After the reaction for 4.5 minutes, the solution was discharged from the frit. Subsequently, the resin was washed 4 times with DMF (0.7 mL per column).

### Elongation step

Solution 1 (0.3 mL) was mixed with solution 2 (0.36 mL) in a mixing vial of the synthesizer, and then the mixture was added to the resin. The solid-phase reaction vessel was heated to 40°C, and the mixture was allowed to react for 2.5 hours to perform a condensation reaction of the amino group on resin with the Fmoc-protected amino acid, followed by discharge of the solution from the frit. Subsequently, the resin was washed 3 times with DMF (0.7 mL per column).

### De-Fmoc step

A solution of DBU in DMF (2%v/v, 0.7 mL per column) was added, and the deprotection of the Fmoc group was performed at room temperature to 35°C. After the reaction for 10 minutes, the solution was discharged from the frit. Subsequently, the resin was washed 4 times with DMF (0.7 mL per column).

The resin was used for the experiments described in Reference Example 4-2-2a or Example 4-2-2b while still set in the synthesizer after the end of the reaction.

### Example 4-2-2: Elongation reaction of Fmoc-MeVal-OH to MeVal supported on resin under various reaction conditions

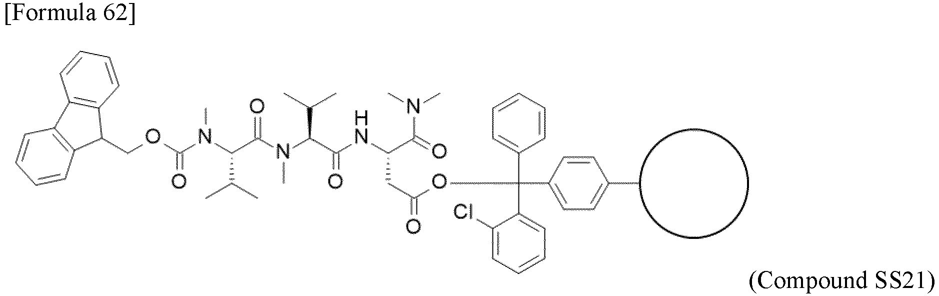

The elongation reaction of Fmoc-MeVal-OH to MeVal supported on resin was carried out using the reaction conditions according to the synthesis method of peptides containing N-substituted amino acids (WO 2018/225851) and the preferred reaction conditions for peptide synthesis on membrane specified in Example 2 to synthesize compound SS21.

### Reference Example 4-2-2a: Elongation reaction of Fmoc-MeVal-OH to MeVal supported on resin under reaction conditions as described in WO 2018/225851

The elongation reaction of Fmoc-MeVal-OH was performed with MeVal-Asp(O-Trt(2-Cl)-resin)-NMe2 (compound SS20, 100 mg per column) prepared in Example 4-2-1, using a peptide synthesizer (Multipep RS; manufactured by Intavis Bioanalytical Instruments AG) under the reaction conditions described in Example 2-4, No. 1. Detailed procedures of operations were performed in accordance with the manual attached to the synthesizer.

Fmoc-MeVal-OH (0.6 mol/L) and HOAt (0.375 mol/L) were dissolved in NMP to prepare solution 1. DIC (0.71 mol/L) and DMF were mixed to prepare solution 2.

Solutions 1 and 2 were set in the peptide synthesizer, and automated synthesis with the peptide synthesizer was started.

Solution 1 (0.3 mL) was mixed with solution 2 (0.36 mL) in a mixing vial of the synthesizer, and then the mixture was added to the resin. The solid-phase reaction vessel was heated to 40°C, and the mixture was allowed to react for 2.5 hours to perform a condensation reaction of the amino group on resin with the Fmoc-protected amino acid, followed by discharge of the solution from the frit. Subsequently, the resin was washed 3 times with DMF (0.7 mL per column).

### Example 4-2-2b: Elongation reaction of Fmoc-MeVal-OH to MeVal supported on resin under high reagent concentration reaction conditions using DsBC

The elongation reaction of Fmoc-MeVal-OH to MeVal supported on a resin was performed with MeVal-Asp(O-Trt(2-Cl)-resin)-NMe2 (compound SS20, 100 mg per column) prepared in Example 4-2-1 under the reaction conditions described in Example 2-4, No. 2 to No. 5 while increasing the reagent concentration using DsBC and referring to the reaction conditions of Examples 2-4.

Solution 1 (601 µL) prepared by dissolving Fmoc-MeVal-OH (0.63 mol/L) and HOAt (0.198 mol/L or 0.397 mol/L) in DMF was mixed with DsBC (neat) (98.7 µL), then 660 µL of the mixed solution was added to a resin, and a condensation reaction between the amino group on the resin and the Fmoc-protected amino acid was performed by heating the solid phase reaction vessel to 40°C or 50°C and allowing them to react for 2.5 hours. Thereafter, the solution was discharged from the frit. Subsequently, the resin was washed 3 times with DMF (0.7 mL per column).

### Example 4-2-3: Synthesis of compound SS22

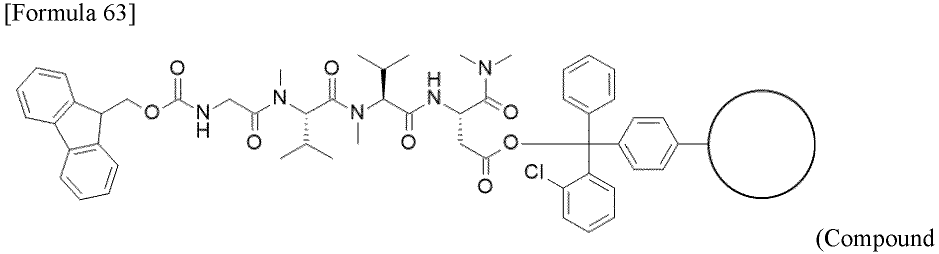

SS22)

Compound SS21 obtained in Reference Example 4-2-2a or Example 4-2-2b was used to perform an elongation reaction of Fmoc-Gly-OH to MeVal on a resin according to the synthesis method of peptides containing N-substituted amino acids (WO 2018/225851). This Example was performed by an Fmoc method using a peptide synthesizer (Multipep RS; manufactured by Intavis Bioanalytical Instruments AG). Detailed procedures of operations were performed in accordance with the manual attached to the synthesizer.

Fmoc-Gly-OH (0.6 mol/L) constituting a peptide of interest, and HOAt as a carboxylic acid activating agent (0.375 mol/L) were dissolved in NMP to prepare solution 1. DIC (0.71 mol/L) and DMF were mixed to prepare solution 2.

Compound SS21 (100 mg per column) prepared in Reference Example 4-2-2a or Example 4-2-2b was added to a solid phase reaction vessel and set into a peptide synthesizer. Solutions 1 and 2 were set in the peptide synthesizer, and automated synthesis with the peptide synthesizer was started.

### De-Fmoc step

A solution of DBU in DMF (2%v/v, 0.7 mL per column) was added, and the deprotection of the Fmoc group was performed at room temperature to 35°C. After the reaction for 10 minutes, the solution was discharged from the frit. Subsequently, the resin was washed 4 times with DMF (0.7 mL per column).

### Elongation step

Solution 1 (0.3 mL) was mixed with solution 2 (0.36 mL) in a mixing vial of the synthesizer, and then the mixture was added to the resin. The solid-phase reaction vessel was heated to 40°C, and the mixture was allowed to react for 2.5 hours to perform a condensation reaction of the amino group on resin with the Fmoc-protected amino acid, followed by discharge of the solution from the frit. Subsequently, the resin was washed 3 times with DMF (0.7 mL per column). The resin was further washed 4 times with DCM (1.0 mL per column), dried, and then used for subsequent studies.

Peptides were isolated from a part of the obtained resin with a TFE/DCM solution (1/1 (v/v)) containing DIPEA (0.042 mol/L). The solution of isolates was analyzed by LCMS, and the result showed the production of peptide of interest (compound SS22*). In addition to the peptide of interest, the production of compound SS23*, which lacks one MeVal, was also confirmed. 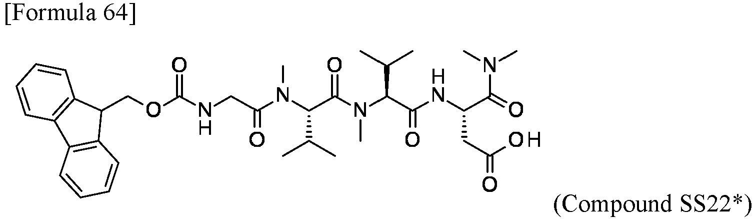 LCMS (ESI) m/z = 666.7 [M + H]⁺
Retention time: 0.80 minutes (analysis condition SQDFA05_3) 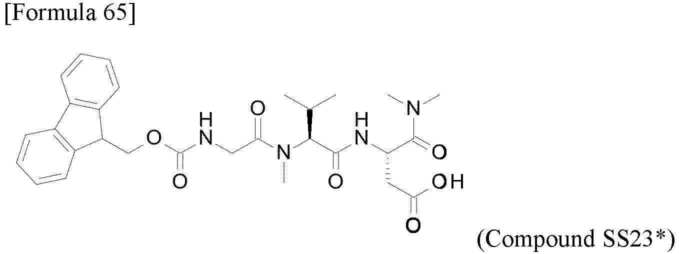 LCMS (ESI) m/z = 553.5 [M + H]⁺
Retention time: 0.72 minutes (analysis condition SQDFA05_3)

The elongation efficiency was calculated from the following equation using UV area values (wavelength 299 nm) of peaks of each compound in the LC data. Elongation efficiency (%) = (UV area value of compound SS22*)/(sum of UV area value of compound SS22* and UV area value of compound SS23*) × 100

The results of Example 4 are shown in Table 25.

**[Table 25]**

| Reference Example or Example No. | No. | Reaction conditions of Reference Example 4-2-2a or Example 4-2-2b | | | | | Peptide of interest | Elongation efficiency |
|---|---|---|---|---|---|---|---|---|
| | | Fmoc-protected amino acid (concentration in reaction solution) | Activating agent (concentration in reaction solution) | Carbodiimide (concentration in reaction solution) | Reaction solvent | Reaction temperature | | |
| 4-2-2a | 1 | Fmoc-MeVal-OH (0.27mol/L) | HOAt (0.17mol/L) | DIC (0.38mol/L) | NMP/DMF=5/6 | 40°C | SS22* | 50% |
| 4-2-2b | 2 | Fmoc-MeVal-OH (0.55mol/L) | HOAt (0.17mol/L) | DsBC (0.77mol/L) | DMF | 40°C | SS22* | 74% |
| | 3 | | | | DMF | 50°C | | 85% |
| | 4 | | HOAt (0.34mol/L) | | DMF | 40°C | | 83% |
| | 5 | | | | DMF | 50°C | | 89% |

In the elongation of Fmoc-protected amino acid on a resin, the improvement of elongation efficiency was also confirmed by performing the elongation under the reaction conditions of the present invention.

## Claims

1. A method for producing a peptide compound by a solid phase method, the method comprising:
a preparation step of preparing a first amino acid having an amino group or a first peptide having an amino group supported on a solid phase; and
a condensation step of condensing the first amino acid or first peptide, and a second amino acid having a protected amino group and/or protected hydroxy group and a carboxy group, or a second peptide having a protected amino group and/or protected hydroxy group and a carboxy group in the presence of at least one carbodiimide-based condensing agent represented by the following formula (A):
R^{A}-N=C=N-R^{B} (A)
wherein R^{A} is C₄-C₁₀ secondary or tertiary alkyl, and R^{B} is C₂-C₁₀ alkyl, C₆-C₁₀ aryl, or C₇-C₁₄ arylalkyl, and each group in R^{A} and R^{B} is optionally substituted with one or more groups independently selected from halogen, C₁-C₆ alkoxy, di-C₁-C₆ alkylamino, or 4- to 8-membered cyclic amino,
and an additive.

2. The method according to claim 1, wherein R^{A} is C₄-C₈ secondary or tertiary alkyl, and R^{B} is C₄-C₁₀ secondary or tertiary alkyl, or C₇-C₁₄ arylalkyl.

3. The method according to claim 1 or 2, wherein the carbodiimide-based condensing agent includes at least one selected from the group consisting of N,N'-di-sec-butylcarbodiimide (DsBC), N'-(1-phenylethyl)-N-sec-butyl-methanediimine, N'-(1-methylheptyl)-N-sec-butyl-methanediimine, N,N'-bis(1-methylbutyl)methanediimine, N,N'-bis(1-ethylpropyl)methanediimine, and N'-(1-ethylpropyl)-N-(1-methylbutyl)methanediimine.

4. The method according to any one of claims 1 to 3, wherein the additive is at least one selected from the group consisting of 1-hydroxy-7-azabenzotriazole (HOAt), 1-hydroxybenzotriazole (HOBt), 3,4-dihydro-3-hydroxy-4-oxo-1,2,3-benzotriazine (HOOBt), cyano(hydroxyimino)ethyl acetate (Oxyma), and 5-(hydroxyimino)-1,3-dimethylpyrimidine-2,4,6(1H,3H,5H)-trione (Oxyma B).

5. The method according to any one of claims 1 to 4, wherein the condensation step is performed in a solvent, and a concentration of the carbodiimide-based condensing agent in the solvent is 0.4 mol/L or more, 0.5 mol/L or more, 0.6 mol/L or more, or 0.7 mol/L or more.

6. The method according to any one of claims 1 to 5, wherein the solid phase is a membrane or a resin for solid phase synthesis.

7. The method according to claim 6, wherein the membrane is a cellulose membrane, a polypropylene membrane, or a polyaminoethylmethacrylamide membrane.

8. The method according to any one of claims 1 to 7, wherein the solid phase and the first amino acid or first peptide are linked via a photo-cleavable site, a disulfide bond, or an acid-labile site.

9. The method according to any one of claims 1 to 8, wherein the first amino acid or an amino acid at the N-terminus of the first peptide is a non-natural amino acid.

10. The method according to any one of claims 1 to 9, wherein the first amino acid or an amino acid at the N-terminus of the first peptide is an α,α-di-substituted amino acid, a β-branched amino acid, or an N-alkylamino acid provided that the alkyl in the N-alkylamino acid is optionally substituted with one or more groups independently selected from C₃-C₆ cycloalkyl, C₂-C₆ alkenyl, C₂-C₆ alkynyl, or C₆-C₁₀ aryl.

11. The method according to any one of claims 1 to 10, wherein the second amino acid or an amino acid at the C-terminus of the second peptide is a non-natural amino acid.

12. Use of a carbodiimide-based condensing agent represented by formula (A) in the method according to any one of claims 1 to 11.

13. A method for producing a peptide compound supported on a membrane, comprising the method according to any one of claims 1 to 11.

14. A method for producing a peptide library supported on a membrane, comprising a step of performing the method according to claim 13 to obtain 10 or more kinds of peptide compounds supported on the membrane.

15. The method according to claim 13 or 14, wherein the method uses an automated synthesizer.
